# EUROPEAN PATENT APPLICATION

(11) **EP 2 407 559 A2**
(43) Date of publication of application: **18.01.2012**
(21) Application number: 11167462.8
(22) Date of filing: 06.08.2007
(51) Int. Cl.: C12Q 1/68, C12N 15/29

(54) **Genomics-based quality diagnostics for fresh agricultural products**

(30) Priority: 07.08.2006 EP 06118502
(62) Divisional of application: 07808527.1
(71) Applicant: Nsure Holding B.V., 6708 PD Wageningen (NL)
(72) Inventor: van Wordragen, Monique Francisca, 6824 NB, Arnhem (NL); de Maagd, Rudolf Aart, 6708 HL, Wageningen (NL); Mes, Jurriaan Johannes, 6703 AV, Wageningen (NL); Balk, Peter Albert, 6671 BD, Zetten (NL)
(74) Representative: Swinkels, Bart Willem

(57) **Abstract**

The invention relates to the field of quality testing of fresh plant-based and mushroom based products. Methods, carriers and kits for determining the quality stage are provided.

SEQUENCE LISTING

<110>  NSure Holding B.V.

<120>  Genomics based quality diagnostics for fresh agricultural
       products

<130>  P6004976EP1

<150>  EP06118502.1
<151>  2006-08-07

<160>  174

<170>  PatentIn version 3.3

<210>  1
<211>  692
<212>  DNA
<213>  unknown

<220>
<223>  b1nr013, upregulated sequence from beech

<400>  1
acaaacctac aaacaagcat ttaatacaca cacagatata tatatgccac agtgacacct      60

agtggcgtcc aggaagcttc tccttgatct tttccatgac tcccttcttc tcagcttgtt     120

gctcatggcc ctgaagattg taaccaggca aggtagttga agttgtatga tcttggtgat     180

gctgatcacc ctggaccttg tccttgttcc cagcacctgg aatcttctca aagaccttgt     240

ccatgcaccc ttgcccctgc ttctcaggtt gttgcccatg gccctgagca ttgtgaccag     300

gcaaggtagt tgaagttgta tgatcttggc gatactgatc accctggacc ttgtccttgt     360

tcccaacacc tggaatcttc tcaaagacct tgtccatggc ccccttcttc tcaggttgtt     420

gcccatggcc ctgagcattg taaccaggcg tggtagttga agttgtatga tcttggcgat     480

actgatcacc ctggactctg tccttgttcc cagcacctgg agtcttctca aagaacttgt     540

tcatggcccc cttcttctca gttgttgccc acctcgggcg ccgaccacgc taatcgaatt     600

ctcgcggccg ccatggcggc cggaacatgc gacgtccggc caaatcgccc tatagtgagt     660

cgtatacaat tcctggcgtc cttttaaaaa aa                                   692


<210>  2
<211>  284
<212>  DNA
<213>  unknown

<220>
<223>  b1nr031, upregulated sequence from beech

<400>  2
tagcggccgc ccgggcaggt ggcagggaga gaccgttatt cctggtggaa ctggtgggaa      60

gagccttgaa gctcaggagc accttgctga aggacggagc catggagggc aggctaggaa     120

ggagcagctg ggacatgaag gctaccagga gttgggcagc aagggagggc aggctaggaa     180

ggagcagctg ggacatgaag gccaccaaga gttgggcagc aaaggaggac aggctaggaa     240

agagcagata ggacatgagg ggtacctcgg ccgcgaccac gcta                      284

<210> 3
<211> 296
<212> DNA
<213> unknown

<220>
<223> b1nr039, upregulated sequence from beech

<400> 3
tagcgtggtc gcggccgagg tacccttgac aacaaggcat catagtctcc ttatgggcga 60

tgtccaacac caccttgccc atctctacgt ccctgaccct gtcccccttc ttgaccaggg 120

cctccttgtt gtcctggtcc ttgagcacca ggaccaccag gaccacgccc atctttttgt 180

ccctgacctt gaccctgtcc accaagctga ccttgaccct gtccaccgag ctgtccttga 240

ccttcaccac aaccaggccc gccaggacgt cctacctgcc cgggcggccg ctcgaa 296


<210> 4
<211> 293
<212> DNA
<213> unknown

<220>
<223> b4nr049, upregulated sequence from beech

<400> 4
tcgagcggcc gcccgggcag gtacccttga caacaaggca tcatagtctc cttatgggcg 60

atgtccacca ccaccttgcc catctctacg tccctgaccc tatccacctt cttgaccagg 120

gcctccttgt tgtcctggtc cttgagcacc aggaccacca ggaccacgcc catctttttg 180

tccctgacct tgaccctgtc caccaagctg accttgaccc tgtccaccga gctgtccttg 240

accttcacca caaccaggcc cgccaggacg tcacctcggc cgcgaccacg cta 293


<210> 5
<211> 396
<212> DNA
<213> unknown

<220>
<223> b4nr081, upregulated sequence from beech

<400> 5
tagcgtggtc gcggccgagg tacaagcttt tttttttttt tttttttttt ttttttccaa 60

ccaaaatttc cctttttttt ttaaatttcc cccccctttt tccccaaaaa aaggggcccc 120

tcctttttta aaaaaaccca atttttaaaa actttttttg cccttcccct ttcccctttt 180

ttttaaaatt taaaaaaaaa ttttattttt ttttccctaa aaaaaccccc cccccccctt 240

tttttttttt tttccccccc cacccaaaaa aaaaaatttt tccccttttt ttttttaaaa 300

cccaacccct ttttttttta aaaaccaaaa aaggccccca aaaggggcct cccccaattt 360

tccccaggtt tttttttttt tttccccccc gggggg 396


<210> 6
<211> 352
<212> DNA
<213> unknown

<220>
<223>  b4nr096, upregulated sequence from beech

<400>  6
tagcgtggtc gcggccgagg tacgggacta ttgagtaact ctggctaggc ccttctgtgg      60

tgcctcttct ggttttgtag aataaagtgg cttaggttag tagttatgta aaggtaatat     120

gttgttttct ggtttgtgct tgtattccat cagtgtgtgt gtgtatctga tggataaact     180

agttaatgtt tttgttgaag tctagctata gctggaatgg gacagtgcat gatatggtct     240

tagtatgttg gtttttttat catgcgtagt tccctctttt tgtgggtaat ggttggctgt     300

aatctaatag atgatgctga cttttggttg aaaaaaaaaa aaaaaaaaaa aa             352


<210>  7
<211>  508
<212>  DNA
<213>  unknown

<220>
<223>  b5nr012, upregulated sequence from beech

<400>  7
ttcgagcggc cgcccgggca ggtacaagga tgaagctgcg cgaaatgaag cttatagggg      60

taatgcggat gttgggcatg taggaagaga aatagagaag attgaggtga atattgaaaa     120

gacccgacca ggagctgtgg ctgctacact gaaagcagct gatgagatgg ctggtcagac     180

tttcaatgat gtgggaccct tagatgaaga gggcgtaatt ggtttggacc gtcaccgcaa     240

gatgtgatta cgtccaccta gcttcctaag agtggttttt ttacatcatg atgatggtgt     300

tttcttttgc tatcgttttc tgtattttgt tactgtgtta tatagtcctt actttttgag     360

tccgacggac tcgttatctt gttgctggaa gtgaattttg gataatatga atatgatctc     420

atagaataca aaatggatag ggcctgattt tattcaggcc aggtccatct tcgagtttta     480

tttggacacc tcggccgcga ccacgcta                                        508


<210>  8
<211>  321
<212>  DNA
<213>  unknown

<220>
<223>  b5nr018, upregulated sequence from beech

<400>  8
ttcgagcggc cgcccgggca ggtacaacgt aaggggcgct ttaaggtcac ttcggcagac      60

cttagtccca agggtcctac aaactgcatg tttaacccaa tttctggatg ttcaacgagt     120

ccaaccaccc caaaccttac ggctgcctca attctcccat ctctgcaatt tattttgcaa     180

cagaactcca cacaaaggga agaacttatg aaattgatca agtatgtgga acaaacctct     240

ggcaagcatg ctgagtcagc tgaggttgtc acaagtgacc tgttgcagat gcctcctggt     300

acctcggccg cgaccacgct a                                               321


<210>  9

<211>  405
<212>  DNA
<213>  unknown

<220>
<223>  b5nr019, upregulated sequence from beech

<400>  9
ttcgagcggc cgcccgggca ggttttttaa agcctaaatt actctctgtg ttttgagttc      60

taagtagaaa ctgaagatgg acaaatccca gaatacgtgt ttccaagctg gccaggccaa     120

gggccaaact caggaaaagg caagcaactt gatggaaaag gctggaaatg ctggtcagtc     180

tgccaaggaa acatgcctag aggctggtaa gcaagtgccg gctaaggcac aaggggctgc     240

taatactgct aaggatgcag ttggacttgg agcaaagaaa tgaaactaat attggaccta     300

ataaagccag ctctccccaa ataaattttg taatttaatt tttttttttc cttttaagtt     360

atggattctt gttttctact ttgtacctcg gccgcgacca cgcta                     405


<210>  10
<211>  642
<212>  DNA
<213>  unknown

<220>
<223>  b5nr052, upregulated sequence from beech

<400>  10
ttcgagcggc cgcccgggca ggtgtttttg ttttcaatgc ttggagatga gtgcgagcta      60

agcttcgctg ttattagaga tgtgctctgt caatgtggat atgatgttga aaaggccttg     120

gatgtgttgc ttgaattatc ggcttccacc tttgagcagt ccaggaatga tggttacctc     180

aacaatagtg ttaactataa agaagatata agatttctta ttgaacgcaa agactttatt     240

aatgatgggg cttccgattg cgcttcttat tcttctgaaa gtgaattcca ggataatata     300

tggtctctgg gatatgggtg caggaattat gcaaaggttc ttgataatcc tgaagctaat     360

tctccaacta gcccaacaat tactgtgtca gagctccctc aaaaggtgtt ggagtctttg     420

tttaaagtcc ccaagagtcc tcattatgag ccaaccacaa tgaattggag gaatgtagca     480

aagaaactgc agtctttggg gcctgggttt gatgcttgtt cttctagtgt tgcggaaccc     540

cagcaaaata cttgcgctaa aggagaggaa tatcatgtgt tcagaaaatc ttctaagcaa     600

cattgggatt caatgaagtc tagctatcag aaagctgcag tg                        642


<210>  11
<211>  385
<212>  DNA
<213>  unknown

<220>
<223>  b5nr078, upregulated sequence from beech


<220>
<221>  misc_feature
<222>  (359)..(359)
<223>  n is a, c, g, or t

<400>  11
tagcgtggtc gcggccgagg tcaggaaatt gattgtggag gtttgtgcaa gcagaggtgc      60

agtcttcatt cgagaccaaa cttgtgcaac agggcgtgcg gcacatgctg tgtgaggtgc     120

aagtgtgtgc cacctgggac tgctgggaac agagagctgt gtggggcatg ctacactgaa     180

atgaccaccc atgggaacaa gaccaaatgt ccataaggcc caatggcaat ggtggcggcc     240

cagattagct agctttggat ccatgttctc tgtgcctgtc aagcttttgt tatgttgtgt     300

gtttgtaatc aataatgtct gtctaaaatc taaatatagc tgttatgttt ctaaagccna     360

aaaaaaaaaa aaaaaaaaaa aaaaa                                           385


<210>  12
<211>  411
<212>  DNA
<213>  unknown

<220>
<223>  b6nr008, upregulated sequence from beech

<400>  12
ttcgagcggc cgcccgggca ggtacccttg acaacaaggc atcatagtct ccttatgggc      60

gatgtccaac accaccttgc ccatctctac gtccctgacc ctgtccccct tcttgaccag     120

ggcctccttg ttgtcctggt ccttgagcac caggaccacc aggaccacgc ccatcttttt     180

gtccctgacc ttgaccctgt ccaccaagct gaccttgacc ctgtccaccg agctgtcctt     240

gaccttcacc acaaccaggc ccgccaggac gtccttctcc tcctagacct tgaccacctt     300

ggcgtccctc accatgaggt gggttccctt gaggtccttg gtgagacatt atcctaagct     360

ttacttggaa attagagtat tgaatacaag acctcggccg cgaccacgct a              411


<210>  13
<211>  112
<212>  DNA
<213>  unknown

<220>
<223>  b6nr046, upregulated sequence from beech

<400>  13
tcgagcggcc gcccgggcag gtactctctt ttgagctggt tgaatttgtc ttttgaatta      60

acaagggaga tcttctatag aagaaaaaaa aaaaaaaaaa aaaaaaaaaa aa             112


<210>  14
<211>  473
<212>  DNA
<213>  unknown

<220>
<223>  b6nr057, upregulated sequence from beech

<400>  14
tcgagcggcc gcccgggcag gtaccagaga ggatcaacgg taggctagca atgattggct      60

ttgttgcagc gttgggacag gagttctata ctggccagga tgtgtttgcc caaatagcca     120

atggtggaat cccatggttc ctagcaacta gcattctgct atcttctgca tctctgattc     180

ctttgtttaa aggtatcagc gcagagtcca agtcagatgg ggtgatgacc tcagatgctg 240

agatgtggaa tgggaggttt gccatgttgg gtttggttgc actggctgtt accgaatatg 300

tgaagggtgg aacccttata tagatctctg atttggtaac acaggcagtc caacttgtgt 360

agataaaaag tatactaata atttccatgt aattcggaaa gaaactcatg tataatgtat 420

ttcaattttc agtaaatcaa atttcaattt taccaaaaaa aaaaaaaaaa aaa 473

<210> 15
<211> 600
<212> DNA
<213> unknown

<220>
<223> b6nr061, upregulated sequence from beech

<400> 15
tagcgtggtc gcggccgagg tttttttttt tttttttttt tggggaaaaa tgaaattttg 60

ttttcttaaa attgaaaata ctttttactt aatttttttc ccaattaact ggaaattttt 120

tattttattt tttttcaaca aaattggaat tgcttttttt cccaattaaa aatttttttt 180

agggttcccc cccttcaatt tttgggtaac acccgttgaa acaaacccca aattggaaaa 240

cccccctttc caattttaag tttttggggt cttcccccct tttaatttga attttgccct 300

attcctttta aacaagggat tcaaaattcc aaaaaatacc aaattgtttt tttttagaaa 360

ccttggattt ccccctttgg tttttgggga aaacacttct tggccattat aaaattcttt 420

tcccaacgtt gaaaaaaacc caatttttgt taccctaccg ttaaccctct ttgttacctc 480

cccggggggc cgttaaaatt caatttcccg gggccccatt gggggcggga accttgaaat 540

ttcgggccca tttcccccta tagtgattct tttaaaaatt cacggccgtc cttttaaaag 600

<210> 16
<211> 477
<212> DNA
<213> unknown

<220>
<223> b1nr005, downregulated sequence from beech

<400> 16
acctcaaacc ccatattcca ttacatctaa acaaacatcc catattccat tatatctaaa 60

caaacattca ccatttaaat aatgatattg aagaaataaa ttatttccca gaaaaaagtg 120

actctcagtt aatggagtcc aaggccaaga tagtgctgag attcatgggg ttcatgtggt 180

cagtagagcc agacatgatc tgctgaacaa taattttgtt tgccttttca gatggatgga 240

atgcatccca aaatgcatac aagtttcggt tggggcacaa gttggagagt ggtgtgcaga 300

gtccaatgcc attgaaaggt ccttgtccac aacaagctat ccttgatgta gtaaacccaa 360

atgcttgagg gtcagtaacg aaattcctgc tcatttcttg tgtattagca gcaatgaaga 420

catctctgcc aatttcgttg ttcagatttc ttatcatttc aacaagctgt gggttgt 477

<210> 17
<211> 400

<212>   DNA
<213>   unknown

<220>
<223>   b1nr019, downregulated sequence from beech

<400>   17
ctttccagtt aggccacaaa acactacaat gaagcagcag gttcaattcc catttcttct      60

tttcttgatc ttcttcctcc attgcaccac aacttctgct caatcccaag caccatcagg     120

ccctacaaac atcacaggaa tcctcaccaa ggctggccaa ttcaccaccc tcattcggtt     180

actaaaaagc acccaagagg ctgaccaaat caacacacag gttaacaact ccaaccaagg     240

cctaaccatc tttgcaccaa ccgacagtgc tttctcaagc ctcaaatcag gcgcactaaa     300

ctctctcact gaccaacaaa aagtccagct ggtccaattc catgtcctcc cttctttcta     360

ttctatctca cagttccaaa ctgtgagcaa ccctttgcgc                           400


<210>   18
<211>   713
<212>   DNA
<213>   unknown

<220>
<223>   b1nr025, downregulated sequence from beech

<400>   18
cttttttcag tctttgcttc catggcgtta cctaagttga tcacagcact tctccttata      60

ttggttcttg caagaataga gatttcaaca ggccatgttg tgaagggcaa gatctcttgc     120

catgattgca ctcacaatga tgtcctctcg ggcatcaagg tttttgtgaa gtgtgataga     180

gttaaaaagt ctgctacggc aatcacagaa gatgatggct cttttgaatc tgagcttccc     240

tcagaaactc caaaatcttc ttctccactg aattgccatg ccatgcttct cgggggtcca     300

acacagctct atgccttaaa ggaaaacttg gtatccaaaa ttatcaaaac ccacgagcct     360

aactcctaca ccatctccag tcctctcagc ttttccacat cttgctcttc tacaaaagaa     420

tctgcgaagt gcaaggacat catgaacaag tttggttctt ccaagactat caatctgcct     480

ctgccaccgg agtggggctt tgcaccatct agcttctaca tccctttctt tcccatcata     540

agcatacctt gattatctat acactctctg agtaaataat atatatactg gattggggta     600

aataaagggc taaaagggct gtgttttatg actgtgtgaa gataccagaa tggaacttta     660

aaacttttag gaaatcctgg atgggatgaa attttttttt tccaagaaag att             713


<210>   19
<211>   679
<212>   DNA
<213>   unknown

<220>
<223>   b1nr082, downregulated sequence from beech

<400>   19
accttgttca atgttcttgg gtcatccaca gctctaatgg taacggttcc aatgtcatct      60

tccttgttaa aaattgcctt gggatttcca tctccaaaga taatgacttt ttctctagga     120

ggagcagtgg ctcctggctg tgacaatgta gggagaaact tgccagcgaa gaagttggat 180

gatacataag tgaagggaat gccttcagcc tcaatggcac gtcggatttg ggccttggta 240

gcaaatgcag tcttggctgg ctcaacagca tggacacggt ccacatcgat tccgaattcc 300

gaagggaaaa atctcttaac attaccagct tctttaatgg cagcaatgat cttgacctga 360

tctactaact gaaggtgacc caccgttgat atcaccacat caacatgctt aatcgctttt 420

accaaactcc catggtcgta aagatctcca tggagcaaag tgacacccaa gttcttgaaa 480

ttctcaatga gttttccctt aacgggatca gagactgtgc tctctctcac caaagcaagg 540

tgggatggcc agactttggg cttgcttccc gatgaatttc cgatgtaggc tgtgcctcca 600

atgatcaaaa tcttgctttt ccaagcattt tcatttttgg gaggctaaaa gggaaattca 660

caataacact agtggagaa 679

<210> 20
<211> 379
<212> DNA
<213> unknown

<220>
<223> b2nr070, downregulated sequence from beech

<400> 20
tagcgtggtc gcggccgagg tgtgacggcc atggatgtcg tttacgcgct caagaggcag 60

ggaaggactc tctatgggtt tgggggttag ggtttttaaa tttctgggga ctttgtcaaa 120

aagtcttgct gtcaagaaag tctgcaaatg gtggtggtgg tgtctgtttt gtttgtaatt 180

agggtttgta atatgaaata tgtgggtttc tagggtttgg tttaagggtc tagttgtagt 240

ttttgttggc tttgttttgt aattcaaact ctgaattgca atttcagaag atttaatgca 300

aagtaggttt tgttaacttg aaattctctg ttgaatgtca aaattggaag ccaaacgaat 360

cagtgcattg taaaaaaaa 379

<210> 21
<211> 348
<212> DNA
<213> unknown

<220>
<223> b2nr074, downregulated sequence from beech

<400> 21
ttcgagcggc cgcccgggca ggtacaggtt ggtttccata attccatttg atgagatttt 60

ttgttttttt tttttccact ttactttaaa ttcattaata ataactaaac catacaaaat 120

tgggtggggg aatcatacaa accaaaggtt gattttaatg ttgatttttg gttactttga 180

taactctgtt ttggttggca attaacccta ataccattca gtttgaactt tggcgcaccg 240

aaaaagcaac cacctaaccc cactgtctca accttgcctt catcgtctga ggattatcac 300

cgtgggggcc taaaaggttt tggaaacacc tcggccgcaa ccacccta 348

<210> 22
<211> 379

<212>   DNA
<213>   unknown

<220>
<223>   b3nr031, downregulated sequence from beech

<400>   22
tagcgtggtc gcggccgagg tgaaagagtt gcggaggaag atcaattagc tagtatagta      60

agggtgagtg aggtaaagga acactgactg aaatggaagt taaaaatgcg aagggaggga     120

catatcctct ggcattgttt gtgatgctga tactcctctt taacgcatat cgttgtggtg     180

ctgctgtcct tgttaagggc aacaccactg tccattgcaa tggccgcctc gatgaatgcc     240

tgatcgagga cgaattggac ttggtgttcc tcaccaaccc atacataagc cggatacttg     300

atgatgggaa acattataaa tctgttgatg ccgtcaagaa ccgtaatcaa ccttgtacct     360

gcccgggcgg ccgctcgaa                                                  379

<210>   23
<211>   478
<212>   DNA
<213>   unknown

<220>
<223>   b3nr056, downregulated sequence from beech

<400>   23
ttcgagcggc cgcccgggca ggtacccgcg ctgccacaca cttggaacct caatttgata      60

gaaccacttg gcgggctggc catgtcccac accgccccat aggccctacg catggccctc     120

cattgctggc aatcctcccg ccacaattca acggctgtga tgtcgttttg tccagctgca     180

tatataacga ataaagctag gtagtgagga aatttgctgt tctcatggac cttaaacatg     240

aggttgtaac cagagtatgt gcatgagatc cttcggtatt ctacatcaac cacaccgtaa     300

ggaaacaact ccgaggccaa gttgggacgt gccgaccttg tgaaggcacg tgggctgagg     360

atgaagtcag ttttgtctcc ctcaccgtag tcagtcacca ctatgctcac cccttcatca     420

ccgcaatatt ttggtttcgt gcacctaacc tgatagcata caccacaccc agttccat       478

<210>   24
<211>   437
<212>   DNA
<213>   unknown

<220>
<223>   b3nr058, downregulated sequence from beech

<400>   24
ttcgagcggc cgcccgggca ggtaccgtgg tgatgttgtg cctaaggatg tcaatgctgc      60

cgttgctact atcaagacca agcgcaccat tcagtttgtt gactggtgcc ccactggatt     120

caagtgtggt attaactacc agcctcccac tgttgttcca ggaggtgacc ttgccaaggt     180

gcagagagct gtttgcatga tctcaaactc tacaagtgtg gctgaggtgt tctcccgcat     240

tgaccacaag ttcgacctca tgtatgccaa gcgtgccttt gtgcactggt atgttgggga     300

gggaatggag gaaggagaat tctctgaagc tcgtgaggac cttgctgccc tggagaagga     360

ttatgaggag gttggtgctg agggagatga gggtgaggat gatgaaggcg atgagtacct 420

cggccgcgac cacgcta 437

<210> 25
<211> 263
<212> DNA
<213> unknown

<220>
<223> b3nr083, downregulated sequence from beech

<400> 25
tagcgtggtc gcggccgagg tggggtctgc tgatctgctc cacaagtatt aaccaacaaa 60

caaacaaacc ctgcgcttcc tatagaatta actgacaaag aaattctctt cgtagatcgg 120

atcgtttttc tgagtttcaa acaaaaatgt cttcagtgtt cagcggcgat gaaacagccc 180

cattctttgg cttcctcggc gctgcagccg ccctagtctt ctcatgtatg ggagcggcgt 240

acctgcccgg gcggccgcct cga 263

<210> 26
<211> 410
<212> DNA
<213> unknown

<220>
<223> b3nr095, downregulated sequence from beech

<400> 26
ttcgagcggc cgcccgggca ggtaccctga ccggatgttg cttactttct ctgtgttccc 60

atcgccaaag gtctcagata cagttgttga gccttacaac gccacccttt ctgttcacca 120

gcttgttgag aatgctgatg agtgcatggt gttggataat gaggccttgt atgatatttg 180

tttcaggact ctcaagttaa ctaccccaag ctttggtgat ctgaaccatc tgatctctgc 240

aaccatgagt ggtgttacat gctgtctcag gttccctggc cagcttaact ctgaccttcg 300

gaaacttgcg gtgaacctca ttccgttccc ccgtctgcac ttctttatgg ttggatttgc 360

tcctcttacc tcacgtggtt ctcaacagta cctcggccgc gaccacgcta 410

<210> 27
<211> 213
<212> DNA
<213> unknown

<220>
<223> b6nr003, downregulated sequence from beech

<400> 27
tagcgtggtc gcggccgagg tacttgagtg tgtctacggt gtgggtgttc accaccgtct 60

tctccaccct cttttttcct tcctcgtcgt ggctcacagc tcggattgtc accgtttcgt 120

tcttgtcgtc aatgggcttc attttcttca tcaaactgat aacctgggtt gaaaaatgct 180

tcagataatg ttgacctgcc cgggcggccg cta 213

<210> 28
<211> 584

<212>  DNA
<213>  unknown

<220>
<223>  b3nr038, constant sequence from beech

<400>  28
ttcgagcggc cgcccgggca ggtgagagag agagagagag agcgaaaaac agagagccga      60

agacaaagaa gaaagagagg gaagcgaaaa gagatggccc gtactctttt ggggtggtca     120

tgttggaagt tctttgtggg agacctgtca ttgatccatc ccttccaagg gaaaaggtga     180

atctagttga ctgggcaatg gaatcacaga agagaaggca attggaggaa atcgtagatc     240

ctcgtcttgc aggtcaaatt aagccagatt cgttaaggaa gtttggcgac atagctgaga     300

aatgcttggc agaatgtggt gttgatcgac cttcaatggg tgatgtgtta tggaatttgg     360

agtttgcact tcaacttcaa gggaatgaac aaagatccaa ttgtgatggc gagctgtctt     420

cacaagttaa tcatgtcaat caagtggaga gcagtgtatc cattggacag ttcagtatgg     480

gcgatgttgc tggtatttca atgagtaagg tctttgccca aatggtgaga gaggatatga     540

ggtagctgat ggtaaagcaa tgtacctcgg ccgcgaccac gcta                      584


<210>  29
<211>  209
<212>  DNA
<213>  unknown

<220>
<223>  b4nr068, constant sequence from beech

<400>  29
tcgagcggcc gcccgggcag gtactgggaa cataaaaaaa aaaaaaaaaa aaaaaaattt      60

taaattcaaa aaattcgttt ttaaaaaaaa aaaattttcc aattgggttt tacccatttt     120

taaaaatttt tcaaattttt ttaaattgca caattttaaa tttcggaaaa cccatttttt     180

taaattaaaa ttttttttaa aaaaaaaaa                                       209


<210>  30
<211>  22
<212>  DNA
<213>  unknown

<220>
<223>  PCR primer b5nr019L

<400>  30
caggccaagg gccaaactca gg                                               22


<210>  31
<211>  22
<212>  DNA
<213>  unknown

<220>
<223>  PCR primer b5nr019L

<400>  31
agcagcccct tgtgccttag cc                                               22

<210>  32
<211>  22
<212>  DNA
<213>  unknown

<220>
<223>  PCR primer b5nr078L

<400>  32
agtgtgtgcc acctgggact gc                                                22


<210>  33
<211>  22
<212>  DNA
<213>  unknown

<220>
<223>  PCR primer b5nr078R

<400>  33
ctaatctggg ccgccaccat tg                                                22


<210>  34
<211>  23
<212>  DNA
<213>  unknown

<220>
<223>  PCR primer b1nr013L

<400>  34
gatccaattg tgatggcgag ctg                                               23


<210>  35
<211>  22
<212>  DNA
<213>  unknown

<220>
<223>  PCR primer b1nr013R

<400>  35
accagcaaca tcgcccatac tg                                                22


<210>  36
<211>  22
<212>  DNA
<213>  unknown

<220>
<223>  PCR primer b2nr070L

<400>  36
cggccatgga tgtcgtttac gc                                                22


<210>  37
<211>  23
<212>  DNA
<213>  unknown

<220>
<223>  PCR primer b2nr070R

<400>  37
caccaccacc accatttgca gac                                              23


<210>  38
<211>  22
<212>  DNA
<213>  unknown

<220>
<223>  PCR primer b3nr058L

<400>  38
tcccgcattg accacaagtt cg                                               22


<210>  39
<211>  22
<212>  DNA
<213>  unknown

<220>
<223>  PCR primer b3nr058R

<400>  39
tccagggcag caaggtcctc ac                                               22


<210>  40
<211>  23
<212>  DNA
<213>  unknown

<220>
<223>  PCR primer b3nr038L

<400>  40
gatccaattg tgatggcgag ctg                                              23


<210>  41
<211>  22
<212>  DNA
<213>  unknown

<220>
<223>  PCR primer b3nr038R

<400>  41
accagcaaca tcgcccatac tg                                               22


<210>  42
<211>  631
<212>  DNA
<213>  unknown

<220>
<223>  SAM synthase 1, constant sequence from pear

<400>  42
gatgttggtc ttgatgctga caactgcaag gttctagtga acattgagca gcagagtcct      60

gacattgctc aaggtgtcca tggtcatttc tccaagaggc ctgaggaaat tggtgctggt     120

gaccaaggcc acatgtttgg gtatgctact gatgagaccc ccgagctgat gccactcacc     180

cacgtccttg caaccaagct tggtgccaag ctgactgagg ttcgcaagaa cggcacttgt     240

gcctggttga ggcctgatgg caagacccaa gtcaccattg agtattttaa tgacaagggt 300

gccatggtcc caattcgcgt ccacaccgtc ctgatttcaa cccagcacga tgagaccgtg 360

acaaacgatg agattgccgc tgatctcaag gtgcattgtg caggtttcct atgccatcgg 420

tgtgcccgag cctttgtcag tgtttgttga ttcttatggc accggaaaga tccccgacca 480

aggagatcct caagattgtg aaggagacat tcgatttcag gccaggcatg atctccatca 540

acctggacct caagaggggt ggcaatggaa ggtttttgaa gaccgccgcc tacggacact 600

tcggcaggga cgaccccgac ttcacctggg a 631

<210> 43
<211> 472
<212> DNA
<213> unknown

<220>
<223> ACO-2, upregulated sequence from pear

<400> 43
cttgttgtgt gagaaccttg gactcgagaa gggttatctg aagaaggttt tctatggatc 60

caagggtccg aattttggga ctaaggtcag caactaccct ccatgcccca agccagacct 120

gatcaaggga ctccgggccc acagcgacgc cggtggcatc atcctgcttt tccaggatga 180

caaggtcagc ggcctccagc tactcaagga tggtgattgg gtggatgtcc ccccaatgca 240

ccactccatt gtcataaact taggtgacca gattgaggtg atcaccaatg ggaagtacaa 300

gagtgtgatg caccgggtga tagctcagtc ggacgggacc agaatgtcga tagcctcgtt 360

ctacaaccca ggcgatgatg catttatcag cccagcaccg gcagtgcttg agaagaaaac 420

tgaggacgcc ccaacttatc ccaagtttgt gttcgatgac tacatgaagc ta 472

<210> 44
<211> 456
<212> DNA
<213> unknown

<220>
<223> ACS3-4, upregulated sequence from pear

<400> 44
acctggggtt caaagggatc aaaaggagtg tctggcttta gagaaaatgc cttatttcaa 60

gactaccatg gccttttgtc tttcagaaag gcaatggcaa gttttatgga acaaattaga 120

ggaggaagag ccaaatttga ccctgctagg gtagtcttaa cggcgggtgc aactgcagca 180

aatgagctat tgactttcgt catagctgat cccggtgatg ctttgcttgt tccaacccca 240

tattatccag ggtttgatag agatttgagg tggaggactg gtgtgaacat tgtgccaatt 300

cactgtgaaa gctcaaacaa cttccagatt actcctcaag ctttagaagc tgcatacaaa 360

gaggcagaag ccaagaacat gagagtgaga ggggtactat tcacaaatcc atcaaaccca 420

cttggtgcaa caatccaaag gacagtcctt gaagag 456

<210> 45
<211> 418

<212>  DNA
<213>  unknown

<220>
<223>  PDC-6P, upregulated sequence from pear

<400>  45
gtatgagttc caaatgcaat atggatcaat tggttggtca gttggagcta ctcttgggta      60

tgctcaagct gttcctgaga agcgtgtgat tgctttcatc ggcgatggga gtttccaggt     120

gactgttcaa gatgtgtcca ccatgatccg aaatgggcag aagaacatca tcttcctgat     180

aaacaacggc ggatacacaa ttggggtggt gatccatgac ggaccctaca atgtgatcaa     240

gaactggaac tacactggac tagttgatgc catccacaac ggggagggca agtgctggac     300

aaccaaggtc cgttgcgaag aggagctgat tgaagcgatt gagactgcaa ccggggcgaa     360

gaaggatagc ttgtgcttca ttgaggtgat agcccacaag gatgatacca gcaaagag       418


<210>  46
<211>  347
<212>  DNA
<213>  unknown

<220>
<223>  PC17-6, upregulated sequence from pear

<400>  46
gactgcgtac cagcttactt tcttttgggt aattaggcct gacttggtca acggtgaatc      60

cgcaatttta ccacctgagt ttgtggatga aactaaggga agaagtctaa tagcgagttg     120

gtgcccacaa gagcaagtac ttaaccaccc atcgattgga gggtttttaa cacacagcgg     180

ttggaattca acaattgaga gtgtgtctgc aggagtgcct ctgttatgct gcccattcca     240

ttctgatcag caaacaaact gtcattatac ttgcaaagaa tggggcattg gtatggagat     300

ggataagaat gtgaagagag aggaagtgga gaagctggta cgcagtc                   347


<210>  47
<211>  20
<212>  DNA
<213>  unknown

<220>
<223>  PCR primer SAM-1 (F)

<400>  47
tcgcaagaac ggcacttgtg                                                   20


<210>  48
<211>  20
<212>  DNA
<213>  unknown

<220>
<223>  PCR primer SAM-1 (Reverse)

<400>  48
cctgcacaat gcaccttgag                                                   20


<210>  49

<211>  21
<212>  DNA
<213>  unknown

<220>
<223>  PCR primer ACO (forward)

<400>  49
gttgtgtgag aaccttggac t                                                21


<210>  50
<211>  21
<212>  DNA
<213>  unknown

<220>
<223>  PCR primer ACO (reverse)

<400>  50
aaaagcagga tgatgccacc g                                                21


<210>  51
<211>  21
<212>  DNA
<213>  unknown

<220>
<223>  PCR primer ACS3 (forward)

<400>  51
tggggttcaa agggatcaaa a                                                21


<210>  52
<211>  19
<212>  DNA
<213>  unknown

<220>
<223>  PCR primer ACS3 (reverse)

<400>  52
agttgcaccc gccgttaag                                                   19


<210>  53
<211>  20
<212>  DNA
<213>  unknown

<220>
<223>  PCR primer PDC-6P (forward)

<400>  53
tgattgcttt catcggcgat                                                  20


<210>  54
<211>  19
<212>  DNA
<213>  unknown

<220>
<223>  PCR primer PDC-6P (reverse)

<400>  54
ccgtcatgga tcaccaccc                                                   19

<210>  55
<211>  19
<212>  DNA
<213>  unknown

<220>
<223>  PCR primer PC17 (forward)

<400>  55
agcgagttgg tgcccacaa                                                    19


<210>  56
<211>  20
<212>  DNA
<213>  unknown

<220>
<223>  PCR primer PC17 (reverse)

<400>  56
tccatctcca taccaatgcc                                                   20


<210>  57
<211>  711
<212>  DNA
<213>  unknown

<220>
<223>  13nr042, upregulated sequence from apple


<220>
<221>  misc_feature
<222>  (13)..(13)
<223>  n is a, c, g, or t

<220>
<221>  misc_feature
<222>  (166)..(166)
<223>  n is a, c, g, or t

<220>
<221>  misc_feature
<222>  (355)..(355)
<223>  n is a, c, g, or t

<220>
<221>  misc_feature
<222>  (695)..(695)
<223>  n is a, c, g, or t

<220>
<221>  misc_feature
<222>  (697)..(699)
<223>  n is a, c, g, or t

<220>
<221>  misc_feature
<222>  (701)..(701)
<223>  n is a, c, g, or t

<220>
<221>  misc_feature
<222>  (709)..(710)
<223>  n is a, c, g, or t

<400>  57
acaccatctt tgngaccatc atcaatcatt gcgagaccct catctaataa agtgtcaaag      60

gcatcgattg cacggccagt ggtccaagat agccgtctct ccacctcagg gacggtcaca     120

aagccttgag cctgggctag ctctaaagtt tcattatggt ctttgntcaa ctcagttgga     180

acagaacgaa caagctttct ctttccaaca gaaataacct cataaccatt acctaatacc     240

ttgagcttgc ttatagcacg caggcaacca tcctcggaaa cagcttcacg gtcactcttc     300

cgcctctgac gaagcagact gcagagttcc tgcaggttga tcaaaccccc attgnggggt     360

cttgttgcca agcaaatgtc aacaatttgc accccaagtt catagtagaa atcaccaatc     420

cccaaaagct ctgcccagaa acccttattg gatgccaagg gatctactcc gaccttcgtg     480

cacatgttgt gaaattgtga tctgaaagct gggttctttc gaatgtcatt cttgtgcttt     540

cgagcgaaat cttccaactg cgttcggaaa gtggtgagct gctccttcat gagatcggtt     600

ctgatcttgg caacattctc gcccagctga cgatattggt ccctagcggc ggcggtggtc     660

tgcaagccgc cgattccaga ctgccccggg ccggncnnnt naaatactnn g              711


<210>  58
<211>  357
<212>  DNA
<213>  unknown

<220>
<223>  13nr062, upregulated sequence from apple


<220>
<221>  misc_feature
<222>  (10)..(11)
<223>  n is a, c, g, or t

<400>  58
acctcagctn nctggtcaat gtcattaaag atgttgatgc acatcttatc aaggaataag      60

cgccaagcac aagatttatg catacatacg atatggaaca ttgagtaata ttactatctt     120

caagaataaa gacctgagag ctgagcaagc tagttagcac tggtgcttat ttcggtttct     180

gatttggttt gtctttaatt ctatgagcaa gaatgaattg agtgtgatat tattataatg     240

ctgatgttgt aaacaagttc aatctattgg catgcttata ttttttcgag aacaacttag     300

ttttttaact aaagtctcaa ccttgtacct cggccgacct cggccgcgac cacgcta        357


<210>  59
<211>  249
<212>  DNA
<213>  unknown

<220>
<223>  15nr090, upregulated sequence from apple


<220>
<221>  misc_feature
<222>  (161)..(162)
<223>  n is a, c, g, or t

<220>
<221>  misc_feature
<222>  (206)..(206)
<223>  n is a, c, g, or t

<400>  59
acaaagcaaa cagacaacaa gcagccataa cctaggacca gatcccagcc tagtaggacg      60

tgcagctcag aagaagccat ggccacttta ttctaaaaac acgaccttcg taaatttgga     120

tcatgattca tgattacgac acacacgcat acatatatta nnggtaaaca tacaagcccc     180

gcgattctct tgcttcatca gaaggngaca tctgtccctc tcacttgcac ctcggccgcg     240

accacgcta                                                             249


<210>  60
<211>  252
<212>  DNA
<213>  unknown

<220>
<223>  5nr032, upregulated sequence from apple


<220>
<221>  misc_feature
<222>  (13)..(13)
<223>  n is a, c, g, or t

<220>
<221>  misc_feature
<222>  (250)..(250)
<223>  n is a, c, g, or t

<400>  60
acacaaagag gtngttcttg ctgagcccta catcgccgta ttgcatgaca tgagaaccaa      60

agccagaatt gtcatttgct gtccttgttt taaccagttc atactgctgg tgcagggttt     120

ctgacttcaa gttgtgtatg tcactgtctt ccatccacgc aacactatac aagtcaccca     180

agcaggtttc atattctgga ggaggactgg gatactctcc agggcagtac ctggcccggg     240

cggccgctcn aa                                                         252


<210>  61
<211>  723
<212>  DNA
<213>  unknown

<220>
<223>  5nr041, upregulated sequence from apple


<220>
<221>  misc_feature
<222>  (13)..(13)
<223>  n is a, c, g, or t

<220>
<221>  misc_feature
<222>  (168)..(168)
<223>  n is a, c, g, or t

<220>
<221>  misc_feature

<222>  (451)..(451)
<223>  n is a, c, g, or t

<220>
<221>  misc_feature
<222>  (554)..(554)
<223>  n is a, c, g, or t

<220>
<221>  misc_feature
<222>  (562)..(567)
<223>  n is a, c, g, or t

<220>
<221>  misc_feature
<222>  (575)..(578)
<223>  n is a, c, g, or t

<220>
<221>  misc_feature
<222>  (584)..(586)
<223>  n is a, c, g, or t

<220>
<221>  misc_feature
<222>  (589)..(593)
<223>  n is a, c, g, or t

<220>
<221>  misc_feature
<222>  (596)..(597)
<223>  n is a, c, g, or t

<220>
<221>  misc_feature
<222>  (599)..(602)
<223>  n is a, c, g, or t

<220>
<221>  misc_feature
<222>  (605)..(609)
<223>  n is a, c, g, or t

<220>
<221>  misc_feature
<222>  (613)..(613)
<223>  n is a, c, g, or t

<220>
<221>  misc_feature
<222>  (615)..(616)
<223>  n is a, c, g, or t

<220>
<221>  misc_feature
<222>  (621)..(624)
<223>  n is a, c, g, or t

<220>
<221>  misc_feature
<222>  (626)..(630)
<223>  n is a, c, g, or t

<220>
<221>  misc_feature
<222>  (632)..(634)
<223>  n is a, c, g, or t

<220>

<221>  misc_feature
<222>  (636)..(642)
<223>  n is a, c, g, or t

<220>
<221>  misc_feature
<222>  (644)..(664)
<223>  n is a, c, g, or t

<220>
<221>  misc_feature
<222>  (666)..(674)
<223>  n is a, c, g, or t

<220>
<221>  misc_feature
<222>  (676)..(694)
<223>  n is a, c, g, or t

<220>
<221>  misc_feature
<222>  (696)..(696)
<223>  n is a, c, g, or t

<220>
<221>  misc_feature
<222>  (698)..(722)
<223>  n is a, c, g, or t

<400>  61
actcacgctt gtnctcgcta aacatgcgag cagcttctga atttgcagga gagtttgggt      60

tgggatcaca tagtagtgac tggatggacg tgagtatagc tgccacgtca tatataggac     120

tccactgatt ctgtaaaata tccaaacaaa tacttccatc cgcataanta tttggatgaa     180

acattctaga aacaaaccgg actgttggtg gtttgtttgg ataatcctcc gtaaactgaa     240

gagtcaactt aaatgtgcct ccatcccaag gtgtatcatc gggaccaaat ataacagcat     300

tccacaacat tatattgtta tcctgtggcg ccccactaat ccctgcaggc gggtcctgtt     360

gcagcctctt gaagtctctc atcagcctct tccttgcagg agtcgacatc gctaatttgc     420

taccacctgg agctccgaat ctccaagagg ncccgatccg agcaggctcc gaattgggtt     480

gctgaatcgg gtgatcaagg aagggttttg gaggtgggtt tgatcagatc gaagccgaag     540

ccatacctgc cccngggcgg gnnnnnnaaa tcacnnnnga attnnnggnn nnntgnngnn     600

nnccnnnnng ggnannctcc nnnncnnnnn gnnngnnnnn nntnnnnnnn nnnnnnnnnn     660

nnnncnnnnn nnnntnnnnn nnnnnnnnnn nnnncngnnn nnnnnnnnnn nnnnnnnnnn     720

nng                                                                   723


<210>  62
<211>  445
<212>  DNA
<213>  unknown

<220>
<223>  5nr069, upregukated sequence from apple


<220>
<221>  misc_feature
<222>  (11)..(11)

<223>  n is a, c, g, or t

<400>  62
aataaaatgg ntgtgagtgg agctggtact tgcaagctgg agactatgga ggctgaggta      60

gagatcaaag ccaacgctga taagctgtac aaattcatca gcaaccagca ctatgacttc     120

cccaaagcag cctctgataa aatacacgat gttgcggtac atgaaggtga ctgggaaact     180

tctggctctg tcaaactctg gaaatacacc atagatggaa atgtcgagac ttacaaggaa     240

aaggtggaaa tagatgaagc aaacaagcgg gtgagtctta cagggttgga aggatcacat     300

gtgctggaca attacaaaag ctacaagatc attttccagg tcactccaaa tagtgaagga     360

ggttctgtga aaattacttt agaatataaa aaactcaacg agaacgatcc gcctccacag     420

aagtacctcg gccgcgacca cgcta                                           445


<210>  63
<211>  650
<212>  DNA
<213>  unknown

<220>
<223>  6nr013, upregulated sequence from apple


<220>
<221>  misc_feature
<222>  (93)..(93)
<223>  n is a, c, g, or t

<400>  63
ggaaacgtta tttcgaccgc tgcaatgcga gtttttgcta ctctctacga gaaagacatc      60

taattcgagc ttgttccaat cgacatgaga gcntggtgaa cacaaaaagg agcccttcat     120

atccctcaat ccatttggtt aagttccagc tttcgaagac ggagatctta agctctttga     180

atcaagggcg attacacaat acattgccca cgagtatgct gacaagggaa cccctctagt     240

gatccgagac tcaaagaaga tggcaattat atcattgtgg tcggaggtgg aggcccaaaa     300

gttcgacccg gcggccacaa aactgaccta tgagctagct ataaagccta tgtttaaaat     360

gaccacagac gcggcagttg tggaggaaaa tgaagccaag ttggctgtgg ttcttgatgt     420

atatgagact cgtctggctc agtcgaaata cttggcaggt gaaagcttca ctttggctga     480

tcttcaccac ctccccacca tacattactt gatgggaaca caatcgaaga agctgttcga     540

atcccgcccc catgttctcg catgggtggc ggacatcaca gcaaggccag cttggaacaa     600

agccgttgcc cagcaaaagt gaaacaagta cctcggccgc gaccacgcta                650


<210>  64
<211>  330
<212>  DNA
<213>  unknown

<220>
<223>  6nr096, upregulated sequence from apple

<400>  64
acgttagtaa ccgtaacgca atgacgaaaa gaacgaagag gtaggattct cttccctact      60

attacccctc acactcttct ttgtcttatt ctgtatataa aaaatttgca gaggtttgtt 120

gcgtgcattt acagaaagtc tttggcattg cctttgggat tctcttgaaa ccttgctatc 180

ctaaactgct tcagctccac tccttcagcc tccccagtaa gcgcaaggtc agccaaaatc 240

ctccccacca ccggcgacat cttgaacccg tgacccgaaa accccccgcc caccaccaca 300

tccttccacc tgcccgggcg gccgctcgaa 330

<210> 65
<211> 447
<212> DNA
<213> unknown

<220>
<223> 7nr006, upregulated sequence from apple

<400> 65
ctgatactta cacagtcgga gacgatttgg aatggactat tcctccagcc ggctctattg 60

cctacaccac ctgggctaac acaaaaagat ttcagaataa tgatacaata gtattcaagt 120

ggagtggatc acacacagta gctgaagtat cgaaggctga ttacgataac tgctcaaatt 180

cgaaccatct tgctttttat gattccagcc ctgcaagtat tacactgact tccaacgtga 240

cccgttactt catctgcacc gtaggtaatc actgcagtga tttaggacag aaggtgacta 300

tcaagatttc tgaagatgat gatcgctggt gggaccgcaa cgcctcttct tctctcaccg 360

ttaatattgg tgccctaatc ctctccactg cgatggccat cctcttctat agctttaatt 420

aagtgtacct cggccgcgac cacgcta 447

<210> 66
<211> 435
<212> DNA
<213> unknown

<220>
<223> 8nr019, upregulated sequence from apple


<220>
<221> misc_feature
<222> (427)..(427)
<223> n is a, c, g, or t

<220>
<221> misc_feature
<222> (433)..(433)
<223> n is a, c, g, or t

<400> 66
actccatgga gatcagttct gttgcttatg accagctttg gcgctttgac atggaagctt 60

tgccagcaga tctcatcaga aggggaatgg cagttgagga tcctacagct gagcatggct 120

tgaagctaac aattgaagac tacccatttg caaatgatgg tctcatgctg tgggatgcca 180

ttaaggagtg ggtgagtgat tatgtgaacc attactatcc agacccaaat cttattgagt 240

ctgatacgga gcttcaaggt tggtggacag atgttagaac caaaggtcat gcagacaaga 300

aagatgagcc atggtggcct gttttgaaaa ccacggaaag tctgattcac gctttgacta 360

ctatcatttg ggtaaccgct ggtcatcatg cagcagtgaa ctttggtcag tacctgcccc     420

gggcggncgc tcnaa                                                      435


<210>  67
<211>  742
<212>  DNA
<213>  unknown

<220>
<223>  11nr011, downregulated sequence from apple


<220>
<221>  misc_feature
<222>  (414)..(414)
<223>  n is a, c, g, or t

<220>
<221>  misc_feature
<222>  (497)..(498)
<223>  n is a, c, g, or t

<220>
<221>  misc_feature
<222>  (500)..(500)
<223>  n is a, c, g, or t

<220>
<221>  misc_feature
<222>  (509)..(510)
<223>  n is a, c, g, or t

<220>
<221>  misc_feature
<222>  (515)..(516)
<223>  n is a, c, g, or t

<220>
<221>  misc_feature
<222>  (526)..(526)
<223>  n is a, c, g, or t

<220>
<221>  misc_feature
<222>  (529)..(531)
<223>  n is a, c, g, or t

<220>
<221>  misc_feature
<222>  (538)..(538)
<223>  n is a, c, g, or t

<220>
<221>  misc_feature
<222>  (540)..(540)
<223>  n is a, c, g, or t

<220>
<221>  misc_feature
<222>  (543)..(543)
<223>  n is a, c, g, or t

<220>
<221>  misc_feature
<222>  (547)..(548)
<223>  n is a, c, g, or t

<220>
<221>  misc_feature
<222>  (551)..(551)
<223>  n is a, c, g, or t

<220>
<221>  misc_feature
<222>  (553)..(557)
<223>  n is a, c, g, or t

<220>
<221>  misc_feature
<222>  (559)..(559)
<223>  n is a, c, g, or t

<220>
<221>  misc_feature
<222>  (561)..(563)
<223>  n is a, c, g, or t

<220>
<221>  misc_feature
<222>  (565)..(565)
<223>  n is a, c, g, or t

<220>
<221>  misc_feature
<222>  (569)..(569)
<223>  n is a, c, g, or t

<220>
<221>  misc_feature
<222>  (576)..(576)
<223>  n is a, c, g, or t

<220>
<221>  misc_feature
<222>  (579)..(580)
<223>  n is a, c, g, or t

<220>
<221>  misc_feature
<222>  (584)..(584)
<223>  n is a, c, g, or t

<220>
<221>  misc_feature
<222>  (587)..(587)
<223>  n is a, c, g, or t

<220>
<221>  misc_feature
<222>  (591)..(591)
<223>  n is a, c, g, or t

<220>
<221>  misc_feature
<222>  (594)..(599)
<223>  n is a, c, g, or t

<220>
<221>  misc_feature
<222>  (601)..(602)
<223>  n is a, c, g, or t

<220>
<221>  misc_feature
<222>  (605)..(606)
<223>  n is a, c, g, or t

<220>
<221>  misc_feature
<222>  (609)..(609)
<223>  n is a, c, g, or t

<220>
<221>  misc_feature
<222>  (612)..(615)
<223>  n is a, c, g, or t

<220>
<221>  misc_feature
<222>  (618)..(618)
<223>  n is a, c, g, or t

<220>
<221>  misc_feature
<222>  (621)..(622)
<223>  n is a, c, g, or t

<220>
<221>  misc_feature
<222>  (624)..(626)
<223>  n is a, c, g, or t

<220>
<221>  misc_feature
<222>  (628)..(628)
<223>  n is a, c, g, or t

<220>
<221>  misc_feature
<222>  (632)..(632)
<223>  n is a, c, g, or t

<220>
<221>  misc_feature
<222>  (636)..(636)
<223>  n is a, c, g, or t

<220>
<221>  misc_feature
<222>  (638)..(638)
<223>  n is a, c, g, or t

<220>
<221>  misc_feature
<222>  (641)..(641)
<223>  n is a, c, g, or t

<220>
<221>  misc_feature
<222>  (645)..(647)
<223>  n is a, c, g, or t

<220>
<221>  misc_feature
<222>  (649)..(650)
<223>  n is a, c, g, or t

<220>
<221>  misc_feature
<222>  (654)..(654)
<223>  n is a, c, g, or t

<220>
<221>  misc_feature
<222>  (657)..(657)

<223>  n is a, c, g, or t

<220>
<221>  misc_feature
<222>  (659)..(659)
<223>  n is a, c, g, or t

<220>
<221>  misc_feature
<222>  (662)..(663)
<223>  n is a, c, g, or t

<220>
<221>  misc_feature
<222>  (665)..(666)
<223>  n is a, c, g, or t

<220>
<221>  misc_feature
<222>  (672)..(672)
<223>  n is a, c, g, or t

<220>
<221>  misc_feature
<222>  (688)..(689)
<223>  n is a, c, g, or t

<220>
<221>  misc_feature
<222>  (695)..(695)
<223>  n is a, c, g, or t

<220>
<221>  misc_feature
<222>  (697)..(699)
<223>  n is a, c, g, or t

<220>
<221>  misc_feature
<222>  (703)..(703)
<223>  n is a, c, g, or t

<220>
<221>  misc_feature
<222>  (706)..(706)
<223>  n is a, c, g, or t

<220>
<221>  misc_feature
<222>  (709)..(709)
<223>  n is a, c, g, or t

<220>
<221>  misc_feature
<222>  (711)..(711)
<223>  n is a, c, g, or t

<220>
<221>  misc_feature
<222>  (719)..(719)
<223>  n is a, c, g, or t

<220>
<221>  misc_feature
<222>  (722)..(724)
<223>  n is a, c, g, or t

<220>
<221>  misc_feature

<222>  (727)..(729)
<223>  n is a, c, g, or t

<220>
<221>  misc_feature
<222>  (733)..(735)
<223>  n is a, c, g, or t

<220>
<221>  misc_feature
<222>  (741)..(741)
<223>  n is a, c, g, or t

<400>  67
cggcacgagg gtcccctccc ccaccatccc cttcaccgcc tccccctgta gtccaatacc      60

aatcaccacc accgccaaca cctgttcact acaattcccc accaccacca tccccatcac     120

ccccaattcc atgtgaaacc ccgccacctc atccatcacc acccccacca gttcaccatc     180

tttcgccacc ggttcactac agttctccac caccaccaat tgtttatgaa agcccgccgc     240

ctcccactcc tgtatatgag ggcccattgc cacccatctt tggagtttcg tacgcgtctc     300

ctccgccgcc acccttctat tgattctttt gagaattttc tcctcctcta acctttccca     360

gagcacaatc accaaaaact caacagtcct cgttcacatt cacggcttct tctntccccc     420

acaccatcat ttctctctct ttcgggcaat tctcatcgct cgtgcagagt tagaaaagca     480

aaaaaaaaaa aaaaaanntn ggggggggnn cgggnnccaa atccgnccnn ntggcggngn     540

ccntatnnca ntnnnnngnc nnngnttcna gaaaanatnn agcnganggt ntgnnnnnnt     600

nngcnnaana annnncangt nnannnancc cngtantntc ncaannntnn tatntcnana     660

gnntnnttga tntttctttc ctcaattnnc aatangnnng acnaanaant ncatacatna     720

annnatnnna cgnnnggagc nt                                              742

<210>  68
<211>  406
<212>  DNA
<213>  unknown

<220>
<223>  12nr048, downregulated sequence from apple

<400>  68
acaggtcatg tgaagaccta gttgtagcta tttccaagag tatccaatag tttttagtca      60

acaacttcgt agattcatta gccaaaaatg catctctctc agcaggagcc agagtccaca     120

gcaccacagc cctctcgaaa tcacttgaga gttctttgtc caacgacttg agaagatctt     180

ccccataggt ttcggcgtat gtctgctgaa tcaattttct ctgagctgca ttcctgtgag     240

tcagaatgga tatgatcaat ccctcatttg tgccccatcc tgcaaaagct ttcctgagct     300

gctcagcgtc ttccactgga gaaggaacca catctggcac cctgtaactc gccattcttt     360

ctctctgtct gtttttgtat gtaagacctc ggccgcgacc acgcta                    406

<210>  69
<211>  695
<212>  DNA
<213>  unknown

<220>
<223>  12nr056, downregulated sequence from apple

<400>  69
acgagaagta tcatgtctgt tatggaggag atgaggaagc tagaaagtcc aactacactg      60

acatggttaa taaatactat gatttggtta ccagctttta tgagtatggc ctggggagag     120

tctttccact ttgcacccag atggaatggg gagtctcttc gagagagtat caagcgacat     180

gaacacttcc ttgctttaca actaggactc aaacctgggc agaaagtttt ggatgtaggt     240

tgtggaattg gtggaccact gagagaaata tctcgcttca gctcaacatc agttactggg     300

ttgaacaaca acgaatatca gatcacgaga ggaaaggaac taaaccgtat tgcaggagtg     360

gacaaaacct gcaactttgt taaggctgac ttcatgaaac taccatttcc tgaaaattca     420

tttgatgcag tatatgcaat tgaagcgacc tgccatgcac cagatgcata tgggtgctac     480

aaggagattt acagagtatt aaaacccggt caatgttttg ctgcatatga gtggtgcatg     540

actggtgcat ttgatcccaa taaccaagaa catcaaaaaa tcaaggcaga aattgagatt     600

ggtgatggcc tcccggacat cagattgacg ggaaagtgtc ttgaagcttt gaaacaagca     660

ggttttgagg tcgtatggga gaaagatctt gcggg                                695

<210>  70
<211>  345
<212>  DNA
<213>  unknown

<220>
<223>  12nr062, downregulated sequence from apple

<400>  70
acaagaacaa tgaagccaga ggaatcccat acccaaagct ccaaccaatg ggggtgtttt      60

cgacattgtg ggaggccggc gattgggcta ccagaggagg acttgagaag atcgactgga     120

gcaaagtccc tttctattca tattacaaag attttgacat tgagggatgc cctgtgccag     180

gaccagcctc atgtgcctca agcaccaaca actggtggga aggcgcttca taccaagccc     240

tcaacgccct tgagtatcga agatacaggt gggttcgtat caaccacatg atctacgatt     300

actgcaccga cagatcccgg tacctgcccc gggcggccgc tcgaa                     345

<210>  71
<211>  714
<212>  DNA
<213>  unknown

<220>
<223>  12nr094, downregulated sequence from apple

<400>  71
acgatacttc ctgtcgatac tgtagatgag ctgcatgtct tcatcgctca gctggaattc      60

aagaacctcc aaattctctt tcaatcgctg aattttcgat gattttggaa tcacctgctg     120

ttttcctctg aattccccac ctcagacaga tttgtgccac gctctttccg tatttcttag     180

ccacatcatt gagaactgga tcatccaaag gtgaaacaga accaaacata tccttgttgg     240

cagcagcacc tccgagaggg gtgtgagctg tgggaagaac gccgtgtttc atacagaatt 300

tgacgagaga gtcgcgctgg aaataggggt gggtttcaaa ttggctcaca gcaggcttta 360

ttttggagta agccaagcaa tctctagtta gaaagagctc atagttgctg agaccaatgc 420

tgcgaactaa gcccaaagag acggtctttt ccatgccctc ccaggtttgt tgaagggaaa 480

ttgttacatc gatgtccaac accttatcct cgcccaaaag actggcagtt ttaccaattg 540

cattgtgctt tgtgggcatt gggtagtgaa ccaggtagag atccagataa tctatctgaa 600

gcttctcgag gctgttctta caggcctcca ccacatgccc atggtctgaa ttccaaatct 660

tggtggtaat gaaaagttct tccctcttaa caagtccagt cttaaatgct tctg 714

<210> 72
<211> 357
<212> DNA
<213> unknown

<220>
<223> 1nr054, downregulated sequence from apple

<400> 72
gagagagcca tggatccaaa ccagcagaga ccaggaattc caccaaatcg ccctcagcca 60

ccacctcaag gcggcggcgg cgactcctcg gccattctct ttgtcctctt cgctttcctc 120

gccatttttt tcgccgtgac cgttcttcca acatcatcaa ctatatcaat tctgcaccaa 180

gtcccagaag gtcatgttgg ggtatattgg agaggaggcg ctcttctgaa gacaattaca 240

gatccaggtt tccatctcaa gctacctctg gtcacccatt ttgaacccgt tcaagtgacc 300

ctgcagactg atcaggtaag ggatattcca tgtggtacct cggccgcgac cacgcta 357

<210> 73
<211> 249
<212> DNA
<213> unknown

<220>
<223> 1nr089, downregulated sequence from apple

<400> 73
gttgaaatcg gccctaaaaa gttgaatgaa taaccccatt gtgtttaaag ttagttgctg 60

tattcaaaag gaattctaag catcaatttc attaatttgc ttatcatgct tttaaaaaac 120

gtttgtttcc ttacctttca ttgttagcca atcaccccta accccgttac aacccttaac 180

ttcaatcttg agtgttatgt gtttcaatat gtggagtttg ggattggtac ctcggccgcg 240

accacgcta 249

<210> 74
<211> 327
<212> DNA
<213> unknown

<220>
<223> 2nr039, downregulated sequence from apple

<400> 74
acttgtttgg gcgtgtgagc atgaagatca agctcatccc tggagactct gctggaaccg 60

tcaccgcttt ttatatgaac tccgacacca atacagttcg tgacgagtta gattttgaat     120

tcttggggaa ccggaccgga cagccttata cagtccaaac caatatctat gctcatggga     180

agggcaatag ggagcaaagg gttaatctct ggtttgaccc tgctgcagac ttccacactt     240

acactatcct ctggaaccat caccatattg tcttctatgt ggatgatgtg cctataaggg     300

tgtacctgcc ccgggcggcc gctcgaa                                         327


<210>  75
<211>  182
<212>  DNA
<213>  unknown

<220>
<223>  2nr080, downregulated sequence from apple

<400>  75
accccagatc actgtctgct tcgacattga cgctaacggt atcttgaatg tctccgccga      60

ggacaagacc accggacaaa agaacaagat cacaatcacc aacgacaagg gcaggttgtc     120

caaggaggag attgagaaga tggttcagga ggccgagaag tacctcggcc gcgaccacgc     180

ta                                                                    182


<210>  76
<211>  408
<212>  DNA
<213>  unknown

<220>
<223>  3nr034, downregulated sequence from apple

<400>  76
acctttgatc cgtcgagcga gagcccttcc acacgggact cccggatcac tatggccgac      60

tttcgtctct gttcgaccag taggtctcac agtcaggcag gcttatacca ttacgctcaa     120

cagcagaatc ttagcttgag cctaccttcg cacacctccg ttactcttta ggaggcatcc     180

gccccagata aactacccac ctcgcagtgt cccgcccccc ccgaattatc ggtgcggcgg     240

ttaggcatcc ttagacgaaa gagtggtctt tcaggattgg tcgttgtgtg tcaccacctc     300

ccacctatcc tacacattcg atcaaggttg tcactgcgaa gctatagtaa aggtgcacgg     360

ggtcttaccg tctagccgtt ggtacctgcc cgggcggccc gctccgaa                  408


<210>  77
<211>  661
<212>  DNA
<213>  unknown

<220>
<223>  OProseR1069 from rose


<220>
<221>  misc_feature
<222>  (579)..(579)
<223>  n is a, c, g, or t

<400>  77

aaatgaagag agcgcattct ccaagatggt caggagtaca ggcccttcta atgcgcagta 60

cttacgcagc ctggtatttg aaggcaagca gaataaggtg aatagagacg aagctgagca 120

actgtttggc cagagccaga ggagatggtt agcctcttcg cgctgggcag ctgctgccca 180

atttgcattg gctgtcagtc tcacatcctc acagaacgat ctccagaggt tagatattgg 240

agacgaggac aatatcctca tgaaaacaaa ggatgcagtt ataacactac agggagtttt 300

ggaaggaaag catgatgaag atatagacat atcactaaat cagcaccata ttccaaggga 360

ggggtggtgg tcagctttct tcagaatagt tgaaggtctg gccgtgatgg gcaggctggc 420

tcagaacaga ctccaccctt tagaagatga cgattttgaa gaccagtctt tatactagta 480

atttcctctg aatggagaag gctatggatt caagatgtta tattgtatag attagagtat 540

agctaatctg gatgatatcc aaattgcaac atgcagaana gagttacatc tgcatagaac 600

accagaacaa caatcatagc agtagttatt aattttaatt tatgcttaaa aaaaaaaaaa 660

a 661

<210> 78
<211> 670
<212> DNA
<213> unknown

<220>
<223> OProseR1072 from rose

<400> 78
cttaaaccaa actttcgtta cgcgcgctat tcagtgtaac actttagggt tctcgacatg 60

cacatccgtc ctgctttcca tggggctcta ctgggtctct ggatctgctt gtttacttca 120

agcttctgct acggtgatga gaagaccatc gaggcagccg gagggccaag caaagttttc 180

tcagagttga aaacacctga ggctgcagag ctcaatgaag agggaaagtt tgcagtacta 240

ccaccaaagc cgttcttcaa gaaaccactt ctgcacaagg ttcccactct gaagaagcca 300

tttccacaaa agccgtactt caaaaaacca cctttacata atattcccat tgtcaaaaag 360

ccatcaccac catctgttcc tgttttcaag atcccaccgt tcaagaagcc gattccttcc 420

ccaattccac aagaagaaaa atcttttttg aagaagccat atttccatcc agttccagtt 480

tacaagaagc caattttacc ccccgtgccg gtttacaaga agccatattt ccatccagtg 540

ccggtttaca ggaagccaat tttcccccca gtgcgtgttt acaagaagcc atatttccac 600

cccgttccgg tttaaaagag acgcatgttc ccccccgggg cctgtttaga gaaagccctg 660

tcgccctccg 670

<210> 79
<211> 436
<212> DNA
<213> unknown

<220>
<223> OProseR1093 from rose

<400> 79
aaaaccccca cccgagtgtg tcgccggcct ctaaattgca gccagttacc tattatcaac 60

ctattcatac tgattctggg tgaagaggtc cagaatatta catgttgtca tacaaggcca 120

aaaatttccg atgtgggcct cttctatgtt ctgaacataa acgtatgttc gttggcgtga 180

attaaaaagt cgatgcttaa gcgacgtaag gcgtactttt actctagaaa gcaataagag 240

ccatgcatag taaaagggtg tataggggaa tatggaaatt gttgttgtgt gtatatgtgg 300

atgaagaagg cccaatgagg caataagtct gttttacaag tgagcaaatg tcaattccta 360

cttgtgtctg tatccagctc ccattattgt accaataaag ctctcctgcg ctattgtgaa 420

aaaaaaaaaa aaaaaa 436

<210> 80
<211> 666
<212> DNA
<213> unknown

<220>
<223> OProse1094 from rose

<400> 80
caaaacaatc agtagagatc caagatccag acgacgtcgt tgcagatcca ctccccacga 60

tgtcttctac cttcagcggc gatgagacgg cgcccttctt cggcttcctc ggcgccgctg 120

cggccctcgt tttctcctgt atgggagctg cctatggcac agcaaagagt ggggtaggtg 180

ttgcatctat gggagtcatg aggcctgagc tggtcatgaa atccattgtt cctgttgtta 240

tggctggagt tttgggtatt tatgggctta ttattgctgt tatcatcagc accgggatta 300

atcccaaggc aaagtcatat tacctctttg atggttatgc acatctctcc tctggtcttg 360

cttgtggtct tgcggggctc tccgctggaa tggcaattgg tattgttggt gatgctggtg 420

tgagagctaa tgcacagcag ccaaaactct ttgtcgggat gatcctcatt ctgatttttg 480

ctgaagcatt ggcactgtat ggcctcattg ttggaatcat cttgtcctcc cgagctggcc 540

aatccagagc agactaagaa gtggctgtgc agtctaatat actttaattg gcatatgcac 600

ttagctgggg aattttttgg ctgctctcag gttgctgatt ggttatgctt gcagattcct 660

aaagat 666

<210> 81
<211> 674
<212> DNA
<213> unknown

<220>
<223> OProse1100 from rose

<400> 81
attcagttaa tctcttagga aatctagtta tcaatggagt ctctggctaa acaggcaaag 60

gttccagcca caatactagc cattgggaca gcaaatccaa taagctgtta ttaccaagaa 120

gactatcccg atttcttgtt caaagtcacc aaaagcgagc acaagaccga attaaaagac 180

aagttcaaac gcatatgtga aaagtcgatg gtaaagaagc gatatctggg cgtcacagaa 240

gagagtctaa aagccaaccc taatatatgc agctacaagg ctccctcact cgacgcacgt 300

caagacttac tgattcacga ggtccccaaa ctcggtaaag aagcggcgtt gaaggccatc 360

agagaatggg gccaacccat ttcaagcctc acccacctca tcttctgcac agcttcctgc 420

gtcgacatgc ccggtgccga ctttcagctc atcaagctcc tcggcctaaa tccatctatc 480

aaccggttca tgatctacca gcaaggctgc tttgctggtg ggaaggtgct acggattgct 540

aaagatgtgg ccgagaacaa tgccggagct cgtgttctga tcgtgtgctg tgagatcacc 600

accatgtttt ttcagcaacc ttgtgacagc cacttggatg ttttggtcgg acaggctctg 660

ttttcagacg gtgc 674

<210> 82
<211> 677
<212> DNA
<213> unknown

<220>
<223> OProseR1117 from rose

<220>
<221> misc_feature
<222> (578)..(578)
<223> n is a, c, g, or t

<220>
<221> misc_feature
<222> (584)..(584)
<223> n is a, c, g, or t

<400> 82
caggaacaga gccatatttg gtctcacact accagcttct agctcacgca gcagccgtaa 60

agttatacaa ggagaagtat caggctgatc agaagggtgt gatagggata acaatactgt 120

cacactggtt tgtgcccttt tctgatgcca agcaccatga agaagctgcg tgttatccgt 180

cacactggtt tgtgcccttt tctgatgcca agcaccatga agaagctgct ctacgagcat 240

tggattttat gtttggatgg tatatggatc ccttaacaaa cggtgaatat ccgcacagca 300

tgagatctct tgtcggagat cgattaccca agttcactaa agagcaatcc gagatgttaa 360

aaggttcatt tgattttctg ggattgaatt actacaccgc taactatgct acttatgcac 420

ctcatctcaa gaatgctgca aaccctagct actttacaga tgctgtcgct actgtttcca 480

ccgagcgtaa tggtattcct attggtcaaa aggctgcttc agattggctg tatgtttatc 540

cagaaggatt tcgagaacta ttgctttaca cgaagganaa gtancataat ccacttattt 600

acatcactga aaatggaagg gatgagcaca atgatccgaa attatcactt gaggaagccc 660

tagccgatac tcaccga 677

<210> 83
<211> 674
<212> DNA
<213> unknown

<220>
<223> OProseR1198 from rose

<400> 83

attcaactaa actagtatgt aaaatgccat cccggctaat taactggcag gtgcaatttt 60

ttgggaggga atgggacttc atggatttat accatattac tctctttctg ggagtccctt 120

tcctctgtct tttagcacca tttcaattca cttggggtgc actttgggtg gcaatatcac 180

tatatttggt gtcgggtatg ggtgtaacta tctcttacca tcggaacctt gcccaccaga 240

gctttaaggt ccccaaatgg cttgaatact cactggctta ttgtgcagtt ctgtcacttc 300

agggtagtcc acttgaatgg gtgagtagcc atagatacca ccatcaattt acagacaaat 360

tgagagaccc tcatagcccc actaagggat tttggtttag tcacgtgaat tgggcgttcg 420

attatcattc tcggtttgga agctatgacg gacaactgat gaagaacgtg ggagatttgg 480

aatgccaact atactatagg tttcttcatt atacctactt ccttcattca gttcttcttg 540

gagttgcact ctatgtggcc ggaggattac cttttgtggt ttggggaatg ggtgtaaggg 600

tggtagtcgt ttcacaaatt actttttcaa taaattctat ttgccacact tggggaaaac 660

aatatgggat actg 674

<210> 84
<211> 678
<212> DNA
<213> unknown

<220>
<223> OProseR1208 from rose

<220>
<221> misc_feature
<222> (669)..(669)
<223> n is a, c, g, or t

<400> 84
cttataagct tcacccaaag aaaccgaagt gggaggaata tggctcggca ttcaggagtc 60

ctagtagtat tggtggtggt gatgatggtg agctggagca attgcatgat gagcgacaac 120

aacattgttt ctaccactgc tggtaatagt actgcggtcg cctcttcagc ctggtgtaac 180

gggcacataa tcgacggcca gtgcgtactt gttgcagacg cggcttacag tggcggcgag 240

cacttggact tcaacctgga cgtgatggac atggtggcga tggagtacta taattcaggt 300

ggaagtaaca gaagggtgtt actgactggt ggtatagaca ttaaaaaagc tctaggtgct 360

caaaagcctg tggttgatga aaaaaacagg aaatatatca acaatagatg tgccacgtac 420

aatcgcgtct gtcctaatta aatgatattg caaccgatca agtagcgatg atcgatctcc 480

aattttaatt tttatcaaaa ctgtccgcaa ttcttaatgt attattaatc atatgtgtat 540

atgggtttaa gttactaaat aatgtctatg ggaggtggga agcttggaca gaccatttga 600

gttgtggact acgtcactcc ctcttgctta ttccacttta ctatttctct aagatttatg 660

tatgtattna ccatcgtc 678

<210> 85
<211> 666
<212> DNA
<213> unknown

<220>
<223>  OProseR1246 from rose


<220>
<221>  misc_feature
<222>  (553)..(553)
<223>  n is a, c, g, or t

<400>  85
gtttagccct ccgtctattt catcaccgtg tgtgtttctc tgaaattccc tgcttctact      60

accagaggtc tgagaaccaa tcatgtcgtc gtcttcttct atctcatcct cgtttcggtt     120

attcactacc aaaatccccc actcttcttc tctaaacccc aaaaccctct ccgccttccc     180

aaaacacccg aacttctctt ccttccgtcg acccatttct ttcgaaagaa tccccaccat     240

atcctcatcc cgatccccat ctgccttagc accatcagct tcaatgcccc tgcaacccat     300

cgaagaactc cccccgaagc ttcgagaaat tgtcaagctc ttccagtccg taccggttcc     360

aaaagccaag tacgaggagc tcctgtacta cggcaagaag ctcaaacccc tcgaagataa     420

gtacaagaca aaagagaaca aggtcgaagg ttgtgtctcg caggtttggg tgcgagccta     480

cctcgattcc gacaagaacg tgttctttga ggccgattcc gacgcgctta tatcgaaagg     540

tctcgctgct ctngctcgtt aagggctctc gggtgaaccc gtggaggaaa ttttgagggt     600

ccgacccgat ttcgcgctcc atcttgggct tcagcagagc ttgtcacctg gtaggaataa     660

tgggtt                                                                666


<210>  86
<211>  527
<212>  DNA
<213>  unknown

<220>
<223>  OProseR1322 from rose

<400>  86
tagcgtggtc gcggccgagg taccagcagc ataacgcaac tgtccatcaa ttgtggtgcc      60

agatgtatga ggggtcatgg catggtttgg catgtaacgc catggatggt ctttgggagc     120

tggttggggg ttccaaacat caccactgta tcctgctatg tgtccactgt tgcaggcatc     180

gacaactgct tgcgtgtcca tgattgcccc acgggcattg ttcacgatca ggactccctt     240

cttgagcttg gaaattctct ctttatcaaa catcccccct gtcttttctg ttagtggggt     300

gttgacaaca acaacatcac attttggaag catcttatca agatcctctt caaacttagc     360

ccccgtctct ttttcaaatt cggcatcagc cttaattctg tacctgcccg ggcggccgct     420

cgaaatcgaa ttcccgcggc cgccatggcg gccgggagca tgcgacgtcg ggcccaattc     480

gccctatagt gagtcgtatt acaattcact ggccgtcttt tttacaa                   527


<210>  87
<211>  517
<212>  DNA
<213>  unknown

<220>
<223>  OProseR1391 from rose

<400>  87
ttcgagcggc cgcccgggca ggtacttcga tacaatgaaa gagatcggtg catcttcaaa      60

gtccaattca gttttcatcc cacatggacc tggtgctgtg aaagacattg cttcacagat     120

aagagatggt ctccttcaag gaaattcaac tcaccagtaa agtcctaata cgaactatga     180

agttggaggt gcggcgtcta taagcttcaa cggttacttg ttagctctat cgtgcaaggc     240

aactaagaat caatttgggt gtgctttcgt catttgacat acatggtata tagtaggtgg     300

atttgagcgt gtttatatgc ttgttcatag ttggttgtct atattttatt cagttcgaag     360

cgatgtgaac tatggctggt ttagtttttt cctgagcagc agggtttctc ttttgaaaac     420

catacctgaa taagcctaga attcgttcag ataggccaag tattatgtag taatgccaag     480

gtgattattt ggttattaat tttcaatttt tgtgaaa                              517


<210>  88
<211>  689
<212>  DNA
<213>  unknown

<220>
<223>  OProse1459 from rose

<400>  88
ttcgagcggc cgcccgggca ggtacatttg aaactgcagc aaccattata attcccagta      60

acaaacctca actttgactc tatccccagg caaaatccta atgaaactct ttcggatttt     120

cccagaaatg tatcctatga tgttgtccgc gttgtctaat cggattcgaa acatgccgtt     180

cgggagcgac tccgtgacca gaccctctgc gctgaacttc tgctcctcaa ctttcgggga     240

tttactcatg gcaacgatgt tgggacaacg tttggagacg aggattgagg ttccgaggtg     300

aagctgaggt tttgggtttg gttggaattt gagggagggt ggggtgaggg ttggggtttt     360

ggagtggagg attgggtggt ggagtgagga tgatgatgtt gaggttaaca tggtggtggt     420

taatggtgat ggggtgatga gttctggtgc tctctgtgtt ttatgggtgg agactgacag     480

aggtggaccg gaatagactt caacgacttc tatcgcgaat ccacgtggga tagtcgtggt     540

gaaaacctta tgggatgact ctgggtgttt cctcagcagg tttgtagatc tgaaagtcct     600

cgggcataga gctttgcgag gggggattat agccctccac ccacttctta ttatcttcca     660

gctcgctgcg tgcctaccat cggtgggga                                       689


<210>  89
<211>  532
<212>  DNA
<213>  unknown

<220>
<223>  OProse1481 from rose

<400>  89
tagcgtggtc gcggccgagg tacgttgaga gttgagtgag agaatacgac gagtttcaga      60

aatatgttga gagtaggttt cttaatgttg cttgccatgg ctttggcagc aactagtgct     120

catgctcgct tagatctatt tggccagctg ctcacctcca agaaccaacc aaatcccaat 180

tcagaatgcc agaaaattcc atttctacta tgcgaccgtg tctgtgacgt ttgcgtttgc 240

gatagacgtg ctccagagtt tgcagagtgc ttttgcggac agtggaaacc ttatgcagaa 300

cttcaaaaat ctgacgaagc acaagtgcag gtgataggat tagaaaaatc ttctattaaa 360

gccaagtctt cgggcgccat tccatataca gaatgcatca aagtctgcaa aatttgtgct 420

tgtacctgcc cgggcggccg aaatcgaatt cccgcggccg ccatggcggc cgggagcatg 480

cgacgtcggg cccaattcgc cctatagtga gtcgtattac aattcactgg cg 532

<210> 90
<211> 485
<212> DNA
<213> unknown

<220>
<223> OProseR1674 from rose

<400> 90
ttcgagcggc cgcccgggca ggtacgagga gctcatcgag gaggcgcgtg acgagctcac 60

gctcatcgga aagatgatcg agtgggatcc atggggtgtt cctgatgatt acgagtgtga 120

ggtgatcgag aatgatgctc cagtccccaa acacgtgcct attcaccgac ctggtcctct 180

tcctgaggag ttttacaaaa cgctgcgggg tcttggttca gaccaaacaa agttggatga 240

ggcaaaggcc acctcaattg aatctgaatc aaaggcataa gggtctgctg tttctgtttt 300

gaagaactat agattccaaa tttgaatttt tatctttctc ttggaactct cctgcttatc 360

tttgattaca atgggtttgt ttgtagattt aaattatggg agagagaggc ttcaatattt 420

aataatctgc acttgtggtg tttgtagtaa caatgttgta ttcccaaaaa aaaaaaaaaa 480

aaaaa 485

<210> 91
<211> 476
<212> DNA
<213> unknown

<220>
<223> OProseR1700 from rose

<400> 91
ttcgagcggc cgcccgggca ggtacaactt tatttgacaa acaagtgctt catagttcat 60

actaataata ctcatgcagc tagcacgcct gacattaggc gaggcagcaa acggtatcta 120

attctcagtt tcatgagcac caggtccatg ccctccgtgc cctccacggc ctccatggcc 180

accatgtcct ccgtgtcctc catgtcctcc atgtccgtgt cctccttcgc ctccatgtcc 240

atggccacca tgtccatgat cgttgccgtg gaagtggtca gaattttcat gagctgagac 300

ctcagaggag atgaggagaa cagcagagac aagggcaacg agaagaatag tcacctcggc 360

cgcgaccacg ctaatcgaat tcccgcggcc gccatggcgg ccgggagcat gcgacgtcgg 420

gcccaattcg ccctatagtg agtcgtatta caattcactg gccgtcgatt tacaag 476

<210>  92
<211>  636
<212>  DNA
<213>  unknown

<220>
<223>  OProse1727 from rose

<400>  92
ttcgagcggc cgcccgggca ggtacctcct cctactgtgc cgacctcaat agaaggcatg      60

gtcactgaga cgtgaagatc cttggcatca ttgatggctt ccatcatggt gatgcagtga     120

gaactctcga tgttctgagc tggatcttga ccagtggcta ggtagactgc tgaaacaatg     180

ttactggcat gtgcattgaa accaccaagg gcaccagcaa ttgcagaacc agttaggttc     240

ttgagcatgt taagctccac caaggccgcc acattggttt tcaacacctt cgtcactatg     300

tctcccttga taacagcctc gcaaaccaca gatttgccac ggccttcaat ccagttgaca     360

gcagcaggct ttttgtctga gcagtagtta ccagaaatgc caatgacatc catgtcaggg     420

aagtcattct ggaggaaatc gagaacgttt tgaacacctt tatagaccat gttcatcccc     480

atagcatcac cagtgctgca gcagaatctc atgtacctcg gccgcgagca cgctaatcga     540

attcccgcgg cccgcatggg gggcgggagc atgcgaacgt cggccccatt ccgcgtatat     600

tgagtcggat tacaattcac cggcggaggg aatgaa                               636

<210>  93
<211>  565
<212>  DNA
<213>  unknown

<220>
<223>  OProseR1783 from rose

<400>  93
ttcgagcggc cgcccgggca ggtacgtcct gttgaggctg atggagtagt ggtgaacaac      60

aatgcacaac atgcttcact ccttgataaa atggactgcg gtggagcgtg cactgcgagg     120

tgtcgtttat catctcggcc tcggctgtgt aagagggcat gcgggacttg ctgccagcgt     180

tgtagctgcg tccctcccgg cacggctgga aactacgacg tctgcccctg ttacgctagc     240

ctcaccaccc acggcggcag acgcaagtgt ccttagttga acaacattaa aactacttca     300

agcaagctta gctatctagc tagctagctt ggttatatat gtaccggagc tactgagtga     360

ataaaaatgc atatataggt ttaacttatc caataaaaat ggctactgct tgtagccttg     420

taatttctgc ttcatattgt acctcggccg cgaccacgct aatcgaattc ccgcggccgc     480

catggcggcc gggagcatgc gacgtcgggc ccaattcgcc ctatagtgag tcgtattaca     540

attcactggc cgtcgttttt ggcaa                                           565

<210>  94
<211>  684
<212>  DNA
<213>  unknown

<220>

<223>  OProseR1792 from rose

<400>  94
tagcgtggtc gcggccgagg tacaacaaat tacaaagaga tgaggttgtg gcagaatgga      60

ggaaagtaaa ggacaagatg tctctccatg ttcattgcca tataagtgga ggtcatttcc     120

ttttggattt gttttcaagg ctgagatact tcatcttctg caaagagctt cctgttgttt     180

tgaaggcctt tattcatgga gatggcaacc tgttcaacag ttacccagaa ttggaggagg     240

ctttggtttg ggtttacttt cactccaaca ttccagaatt caacaaggtt gaatgttggg     300

gcccactcag gaatgcaaca gcaccgtctg gtaggggaca ccagaaggca gcttcctcaa     360

gcaaccagga ggactttgtg ccagaaccat gccaagagga ctgtagctgt tgcttcccac     420

cactgagctc catcccatgg ccccaagagc ttccccagcc agatgaaact ggatatgggg     480

cccagcagag ctttctgggg aagacccaag aaccaaacta aaatttttct ctggttcttc     540

atattggtta atgtaagaac aaaaatgtaa atagagacct agttatttgt cccattaggg     600

ttttcatagt ctttgtttgg acttaggttg atgcatatgt atatacctgt ccggtgactc     660

cagtccgatt ccagattctt ctgg                                            684

<210>  95
<211>  526
<212>  DNA
<213>  unknown

<220>
<223>  OProseR1807 from rose

<400>  95
ttcgagcggc cgcccgggca ggtacactga tgaaattgag cttgaagatg ctgtgcacac      60

ggctattttg actctgaagg agggatttga aggacagatc tcagggaaga acattgagat     120

tggaataatt ggcgaagaca aaatattcag ggtgctatcc ccagcagaga ttgaagatta     180

tttggctgaa gtcgagtagt gttcctgtta ccagacacaa tctgcaatgg ttataggttg     240

caagatggtt gatagtgcga aagtgattta atgtgttaca attatgtcga tcttttggaa     300

ccaaatcaca aaccttttgg gctgaagcag cattttgcca tcattaatag ccctggattt     360

ttaagccttc aaactcttaa agactcattc cgaacaattt gtacctcggc cgcgaccacg     420

ctaatcgaat tcccgcggcc gccatggcgg ccgggagcat gcgacgtcgg gcccaattcg     480

ccctatagtg agtcgtatta caattcactg gcggtcgttt ttacaa                    526

<210>  96
<211>  694
<212>  DNA
<213>  unknown

<220>
<223>  OProseR0008 from rose

<400>  96
ctccctccct tcccccctac taactaagat ggagagccag tacataggag gcaaaagcaa      60

taccgatggc ggtgggatgc cggagaagga agtgagggta gcaaaccaga gaagagttgg     120

gagttgtgag tcacttgtga gggttttggc cgtattacta actctggccg ctgctatagt 180

tcaagggttg aataagcaga ccaaagttgc tccaattaag gtggttccaa ccttgccagc 240

catttatatt ccactcacag ccaaatggca agacttatct gcttttgcgt acttgctggt 300

tgcaaatgtc atagcatgtg tatacgcagc attttcccta gtcctttcct ttgcaaaccg 360

gggcacaaag aagagctcac tagggctatt catcattgtc cttgatatat tgacagtggg 420

ggtgcttttc tcagccatcg gagccgccat ggctatcggt ctgatcgggt accatgggaa 480

ttcacatgtg cagtggaaca aggtctgcaa tgtgtttgac agattttgtc accaggtggt 540

cgcatcagtg gtcctgactc agtttgcagc tcttgcattt atcttcctag ttgtgcttgc 600

tatcttacgc cttaacagat cgaggtccac ttaagtaggc aaacctagct tacatggact 660

atatatatcg tttgtgaacc atagtagaga tgaa 694

<210> 97
<211> 580
<212> DNA
<213> unknown

<220>
<223> OProseR0053 from rose

<400> 97
ttcggcacga ggagagtgtt caaaaatgct gaatcactgg gtgttccatt cccaaagaac 60

caagccatga ggatttactc gagcctttgg aatgctgatg attgggctac cagaggagga 120

ttggtgaaaa ctgattggtc aaaggcaccc tttacagcat actacagaaa cttcaacgtc 180

atcgatgcta aatcatccaa atcattctct gattctcagc ctagttggca gaccaatgca 240

cttgatgctc ctagccgaag acgcctgaga tgggttcaga agtacttcat gatctacaac 300

tactgtaccg atttaaaacg cttcccacga ggttttcctg ctgagtgtag gaactgatct 360

ttccagctaa tcgttcagtt tgaatgcatt ctttagtact gtctgtgtat acagggttgg 420

tgttaaagcc atgtcaaata atgagttctt tgtagctttg gtggggggag tgatggtcaa 480

accaacacca aatttgtaaa ctagcatttg ttgaatatct gtttcatata ataaaatttc 540

agtgtccgtt gtttagtttc tcaaaaaaaa aaaaaaaaaa 580

<210> 98
<211> 726
<212> DNA
<213> unknown

<220>
<223> OProseR0060 from rose

<400> 98
ttcggcacga gggattggta aacataagct tcagattgga gatctggagc atcacatctc 60

cattgaagcc aatttaggag atattatatc ttaatcagga actatcaaac caaaagaaaa 120

gaaatttgat agtcacccat tattatatca tagtattaat tagttcctag tagtcttcct 180

tttcccatat caaggtggaa aagtccacta tactggtgaa ggaaacacga cacaagatca 240

tagtgttcag caaggtttga aataaatact cattttgagt tattccctcg tgccgaattc 300

ggcacgaggt gaagctaaag gaatcccata cccaaagctc caacccatgg gggtgttctc 360

aacattgtgg gaagctgatg attgggcaac aagaggaggg cttgagaaga taaactggag 420

caaagccccc ttctatgctt attacaagga ctttgacatc gaaggatgct ctgtgccagg 480

accagctaat tgtgcctcga gcactaataa ctggtgggaa ggcactgctt accaagcact 540

caatgccctt gaatatagaa gatacaagtg gggtacgtat aaaccacatg atctacgatt 600

actgctccga cagatcccgg tacccagaac ccccaccgta ttgtatcgcc ggtctctaaa 660

ttgcagccag gttacctatt atccacccta ttcatactgg attctggggt gaagacgtcc 720

agaata 726

<210> 99
<211> 684
<212> DNA
<213> unknown

<220>
<223> OProse0106 from rose

<400> 99
aacaaatggt caggtttcat ttatcccatt cggatggggt cctcggatat gcataggaca 60

aaactttggt tggatggaag ctaaaatggc gtagtccttc atattacgta gcttcacatt 120

tgagctttcc ccatcctata ctcatggtcc aatcacagct ataacgattc aaccacaata 180

tggtgcacac attatcttgc aaaaactgta actgcagtgc ggtgtaaaat gaacttcatt 240

ttgttgcaac cactaaggaa gttgcatgta taaaccgtta catagtgatg gtttcatatg 300

tcgtcactgg aatattagta gaaattgtta tatgttagtt gttgctcaag atatcgaaca 360

atgagggtgt tggtgccttc taggggggtg ccggacccct attgtccaag caagttccat 420

ataccatgac caagtgcgac gttttcgaaa aggtcgttga ggctatctac accaatgtcg 480

ataaatccaa gacttccgat ggaatgtcaa ccaccataca cttgggatcc ggtcttatca 540

ccggtatggc tgatgccgta gtctcacagc caggtgatac cgtgttgtca aacattcaca 600

ataccaaggg tcttccagga cagggaacca cttcccgatg gatcaatatc cgtagagact 660

aggcgttaag taggttccta cact 684

<210> 100
<211> 472
<212> DNA
<213> unknown

<220>
<223> OProseR0238 from rose

<400> 100
ctcgttcatt catcaatgga ggctgctcgg aagctggtgt tggtcatggc agcggtgctg 60

ctggtggttg cttgcattaa tgtagaggtc tccgatgcgc agaccatttg caatgtgtct 120

gtgaataact tgatgtcctg caagccggcc gtaacaaaac ctaacccttc caggccgacc 180

aagacctgct gctcggtgct gtcgcacgcc gacttgaagt gcctctgctc ctacaggaac 240

tcgaacctgt tgccttctct ggggattgac cctaaccttg ccatgcagct ccctgccaag 300

tgcaagcttc ctcaccctgc caattgctag agaggccctt agctacagct acactgtcag 360

atgtggctag ctagcttaag ttaaataaaa gctctatatg tatgattaag aagggattaa 420

tcttggagtg aaatgcttaa ttaatttcct gaaaaaaaaa aaaaaaaaaa aa 472

<210> 101
<211> 634
<212> DNA
<213> unknown

<220>
<223> OProse0260 from rose

<400> 101
aattccttca tccatctcat ctccttgtct caccatttcc tctccatttt ccttagttac 60

caattccttc atccatctca tcttctttgt ctctccattt cctttccatt ttcccaccaa 120

ttccttcatc catctcatct tctttgtctc accatttcct ctccattttc cttagtcacc 180

atttcctaca aaaattcctt catccatctc atcttctttg tctctccatt tccttttcat 240

tttttatctc cattctctag ttgcaaagtt ctgcattttc aagcaaggag aggaagaaga 300

agaaggagcc gtgaagatca tatcctccat catccacctt gaaggcttgc ttccaagatt 360

caagatccaa ccatctagct ctccatctcc atctccactc acggtgtaat tcgttccttt 420

tccttgtaac ctttttggtt tccttgtttg atttcgtatg aacttgtttc tagttaacct 480

aacgtttagg gcaaagttta agcccaattt ctatgtttaa ataaagtttt cgaattctat 540

aattgtgatt ctaagttgct tatgtgagtt tgtccgattg aatttgcttg atagacaact 600

gttatatgtg tatctttaca aaaagaaaaa aaaa 634

<210> 102
<211> 649
<212> DNA
<213> unknown

<220>
<223> OProseR0277 from rose

<220>
<221> misc_feature
<222> (497)..(497)
<223> n is a, c, g, or t

<400> 102
gcctctgccc tctctgctct tccaaagctt cctcctttca ttttaaagtt tgcaacttct 60

tcttccaagc attttgaaat ggagtcaatg aagatgaagc tttcagtggc tatgttggtg 120

gttgtgatgg tggccttgtc aagcattgag aaggtggctg cagctgatgc tccagcacct 180

agcccaacat ctgatgccgc caccctcttt gtgcccactt tctttgcatc tctagcagct 240

ttggcttttg gagtcctctt ctgaatcagc tcatgatcag tcatgactgc attttaggct 300

gattggtgta gtagttatat tatgcatttc tctatctttc tcactcactt ttccttctct 360

ctctagaatc gcttgtattc agtttttcag gaaagagaga gagtggaaat gggttggatc 420

gatattttat cttctttctt tcttggggat ttcaggttgt aatttgatta tttattggtg 480

ttgatatgag gaggganttg tttgagtgtg attgatgtat attaattaat cttcttcttc 540

ttgtacggga ttgtattgta ttttcttcct atgatgatct cctaatgcat tgtatttgtg 600

ctgcatttgt tataataaaa tcccattatt gtttccaaaa aaaaaaaaa 649

<210> 103
<211> 706
<212> DNA
<213> unknown

<220>
<223> OProseR0286 from rose

<400> 103
gttattttca tctctagtga cagggaccaa gcctcgtttg atgacttttt ctctggaatg 60

ccatggcttg ctcttccctt tggtgactca agaaaggcat ccttgagtcg ccgattcaag 120

gtccaaggca tccccatgct cgtagccatt ggtcctactg gtcaaacagt cacaaaagaa 180

gcaatacatc tcattatgct tcatggagct aatgcttatc ctttcactga ggagcggctg 240

aaagagatag aagcagaatt taaggagatg gcaaagaggt ggcctgagaa gttgaagaat 300

gcactccatg aggagcatga gcttgtgctc tctcgccgaa gaagtttcat atgtgatggg 360

tgcaacgaga aaggagaggt gtggtcattc tattgcgagg agtgtgactt tgatctccat 420

ccaaagtgtg ctttggagga ggagaaggga accaaaactg atgccaaccc ggaagaagat 480

cctcatccaa agtgtgcttt ggaggaggag aagggaacca aaactgatgc caagccggaa 540

gtagatcccc atccaaagtg tgctttggag gaggaaaagg gaaccaaaac tgatgccaat 600

ccgggaagaa gacccccacg aagatgggta tgccatggtg aattttgtaa aaagcttaag 660

gagcactttg ggtgtgatgc ctttaaactt atgtactcag cttcat 706

<210> 104
<211> 363
<212> DNA
<213> unknown

<220>
<223> OProseR0371 from rose

<400> 104
cttcatccat ttcatcttct ttgtctctcc atttcctttc cattttcctt ctccattctc 60

tagttgcaaa gttctgcgtt ttcaagcaag gagaggaaga agaagaagga gccgtgaaga 120

tcatatcctc catcatccac cttgaaggat tgcttccaag attcaagacc aaccatctag 180

ctctccatct ccatctcccc tcacggtgta attcattctt tttccttgta atgatctttt 240

gttttccttg tttgaattca tatgaacttg tttctagtta acaataatgt ttagggcaaa 300

gtttaagccc aatttctatg tttaaataaa gttttcgaat tctataaaaa aaaaaaaaaa 360

aaa 363

<210> 105

<211>   597
<212>   DNA
<213>   unknown

<220>
<223>   OProseR0556 from rose

<400>   105
cctcgtgccg ctttgcctct ccatttcctc tccattttcc ttagttctta gacatcttcc      60

ttcacccatc tcatcttctt tgcctctcca tttcctctcc attttcctta gttcttagac     120

atcttccttc atccatctca tcttctttgt ctctccattt cctttccatt ttcctaaaca     180

catcttcctt catccatctc atcttctttg tctctccatt tcctttccat tttcctaaac     240

acatcttcct tcatccatct catcttcttt gtctctccat ttcctttcca ttttcctttt     300

ccattctcta gttcttagtt tggcttttcc aggtacagag aagaagaaga agccgtgagg     360

acatatcctc catcgtccac cttgaatagt tgcttccaag attcgagatt ccgaggttca     420

agctctccat ctccatctcc cctgacggtg taatccattg ttttcccggt ggtgtgatct     480

tgttttttcc tctgttggaa ttcttgtgaa gttgtctggt ggttacaagg gtgtgtaggc     540

cagcgtttga cgccgattga ttggttagta ggggtgtcgc ggcttgccaa aaaaaaa        597


<210>   106
<211>   676
<212>   DNA
<213>   unknown

<220>
<223>   OPRoseR0626 from rose

<400>   106
cccttttcct tctcaattcc aaatctctca gaatttagtt taatcaaacc ccggcctctt      60

gctttcccga acataccaat caagaagctg cttttgcaga cactatggtt ttgatttcag     120

gtcttcctga tgaagtaatc tacgattgtc ttattcgtgt caagtatgat cagtttccga     180

ctataacatc tgtttctaaa ggttggaaat cagaggttga gctaccagag tttcatcgct     240

tcagaaagaa tggtggttat ggccaaaagc tcattgtgat ggttcaagca cgtgttcctc     300

ccaaccaagg tggtggtgct ggtgtcttca agtgcctgaa agaaccgctg taccggttca     360

ccgtctgtga gccggattcg ggtgattggg gagagttccc gccaatttct gttttccccg     420

gcgggtctct acccatgttt tgccagtttg cagcggttgg gaccaacttg gtggtggttg     480

gcggtttaga cccggtgacg tgggcgagct gtaaatcggt cttcgtctac aacttcatga     540

ctgccacgtg gcggcgcgga accgatatgc cgggtgggtc ccgcacattt tttgggtgtg     600

cgtctgactc taaccggatg gtgtttgtcg ccggcggaca cgatggcgag aagaatgcat     660

tgagatcggc aatggc                                                     676


<210>   107
<211>   674
<212>   DNA
<213>   unknown

<220>

<223>  OProseR0763 from rose

<400>  107
gaaaatccaa aatcatggac ctcaagctct ctaccctcct ctgcttttcc ctgttcttct      60

ccactatcct aaccccaact ctctctgagc tctgcaaccc tcaagacaaa aaggttctct     120

tcgaaatcaa gacagccttt aacaacccct acatcttgtc ctcatggaaa tccgacgccg     180

actgctgtac cgactggtac aacgtcgagt gtgatcccaa caccaaccgc atcaactccc     240

tcaccatctt caccgacgac cgcctcaccg gccaaatccc cgcccaagtc ggagacttgc     300

cctacctcga aaccctcgtg ctccgcaagc tccccaatct caccggtccc atccagccct     360

ccatcgccaa gctcaagcac ctcaagatgc tcaggctcag ctggaacggc ctctcaggct     420

cagtccctga cttcctcagc cagctcaaga acctcacctt cctcgaactc aacttcaaca     480

acttcacagg ctccatcccc agctctcttt ctcagctacc gaacttatta gcccttcatc     540

tagaccgcaa ccagctcaca ggtcatattc ctagctcatt cggacgattc gttggcaccg     600

ttccagatct attcctctcc cacaaccagc tcacaggcaa aatcccaacc tcatttgcta     660

acatgaactt tgac                                                       674


<210>  108
<211>  672
<212>  DNA
<213>  unknown

<220>
<223>  OProseR0774 from rose


<220>
<221>  misc_feature
<222>  (553)..(553)
<223>  n is a, c, g, or t

<400>  108
ctcaaatcac agcaaacgca gtcgatttag agcttttctc ttctactaca atcatgcagg      60

ccaaggagga agatgtgagt ttgggagcca acaagttccc ggagaggcag ccgatcggta     120

tcgccgctca aaccgaagac gagggcaagg actacaagga gccaccgcct gcgccgctct     180

tcgagcccgg cgagctgacg tcatggtcgt tttacagggc cgggatcgcc gagttcgtcg     240

ccacgtttct gtttctctac atcaccatct tgacggtgat gggcgtcttg aaaggtgaaa     300

ccaagtgcaa aaccgtaggt attcaaggca tcgcttgggc ttttggtggt atgatcttcg     360

ccttggttta ctgcacggcc ggaatctcag ggggtcacat aaacccggct gtgacttttg     420

ggcttttctt ggcccggaag ctgtccttga caagggcggt gttctacatc atcatgcagt     480

gccttggggc catagccgga gccgccgtgg tgaaagcttt tacgaacaag ggtcatttct     540

tcgagatcaa cancggtggc gccaactttg tagcccatgg ctacaccaag ggtagtggtc     600

ttggtgctga aattattggg acctttgtcc ttgtctacac cgtcttctct gccactgacg     660

ccaagcgtag cg                                                         672


<210>  109

<211>  676
<212>  DNA
<213>  unknown

<220>
<223>  OProseR0812 from rose


<220>
<221>  misc_feature
<222>  (635)..(635)
<223>  n is a, c, g, or t

<220>
<221>  misc_feature
<222>  (660)..(660)
<223>  n is a, c, g, or t

<400>  109
ggaacatttg tgatttgttt tacatatccc tcatcaatta ttttcgtgag ccccctctct      60

ctcaggattt ctaggttttc tgaaacggat tagggtttcc attatcttgg tcgccgtccc     120

aaaatttcga atctttaggt tttgatttgg aggtttcaaa agggggtctt cgaaggaggt     180

tgtagatttg gtcaaatttt attcatttat ttgtatcaac tgaatgagct aatggagtca     240

aagggtggga aaaagaagtc tagcagtagt aattcctcat tattctacga agctccccta     300

ggatacagca ttgaagacgt cagacctcac ggtggaatca agaaattcag atcagctgca     360

tactctaact gcgttcgaaa gccatcctga gttgtgctag gagttcacat agccccttaa     420

gatcgttaat actgcaatct aactttagtc tagttctctg tcttacttct ctcaatcttc     480

tctcgttttc ctctcgttct cctttcatct ggtgttgcgt catccttcac ttcaataatt     540

cgagatggct gtaccggtgt ctgcaatcgg atttgaagga tatgaaaaga ggctcgaggt     600

ctgtttcttc gagccaggac tgtttgctga ccctnatggc atgggtctcc gcactttgtn     660

caaagctcca attgat                                                     676


<210>  110
<211>  710
<212>  DNA
<213>  unknown

<220>
<223>  OProseR0125 housekeeping gene arginine decarboxylase from rose


<220>
<221>  misc_feature
<222>  (708)..(708)
<223>  n is a, c, g, or t

<400>  110
ggattcggca cgaggattgg gtctcagatc cctacgactg ctctgctcgc tgatggtgta      60

tccgaggcgg cgcagatcta ttgcgaattg gtccgcctcg gtgcccatat gaaggtcata     120

gacattggag gtggtttggg gatagattat gatggatcca aatccagcga ctctgagatt     180

tctgttggtt atgggcttgg agagtatgca atggctgttg ttggagcagt ccggtatgtt     240

tgtgaccgga ggtcagtgaa gcaccctgtg atttgcagcg agagcggcag agccattgtg     300

tctcatcact ctgttctgat atttgaggct gtttctgcta gtgcttgtga tgttgctcct 360

tccatgtctg cctttgcgct tcagtatttc attgagggac ttacagagga agctcgtgct 420

gattaccgga acctttcggc tgcggcgatc aggggagagt atgaagcttg tttgacatat 480

gctgatctgc tgaagcagcg ctgtgttgag caattcaaag aagggtctct gggtattgaa 540

caattagcca ctgtggatgg gctttgtgat ttggtctcga aagcaattgg agcgtccgat 600

ccagttcgta catacaatgt gcacctctcg gttttcactt ccattcccga cttctggggc 660

attgggcagc ttttccctat tgtcccaatc accggcttga tcaacggncg 710

<210> 111
<211> 1196
<212> DNA
<213> unknown

<220>
<223> OProseR0128 housekeeping gene ubiquitin from rose

<400> 111
aattcggcac aaggcggcac gaggtcgaat tctacgtcaa cctctctctc tctctctctc 60

tcaaagatgc aaattttcgt gaagaccctc accggcaaga ctatcaccct tgaggtggag 120

tcttctgaca ccattgacaa cgttaaggcc aagatccagg acaaggaagg aattccccca 180

gaccagcagc gtctgatctt tgctggcaag cagcttgagg atggccgaac cctagctgac 240

tacaacatcc aaaaggagtc aaccctccac ttggttcttc gtctgcgtgg tggtatgcag 300

atctttgtca agactcttac cggaaagacc atcactcttg aggttgagag ctctgacacc 360

attgacaacg tgaaggccaa gatccaggac aaggagggca ttcccccaga ccagcagagg 420

ctcatctttg ccggcaagca gcttgaggac ggccgcaccc ttgcagacta taacatccag 480

aaggaatcca ccctccactt ggtgcttcgt ctccgtggtg gtatgcaaat ctttgtcaag 540

accttgaccg gaaagaccat caccttggag gtggagagct cagacaccat tgacaacgtg 600

aaggctaaga tccaagacaa ggagggtatc ccaccagacc agcagaggct tatctttgcc 660

ggaaagcagc ttgaggatgg ccgcaccctt gcagactata acatccagaa ggaatccacc 720

cttcatctgg tgctccgtct tcgtggtggt atgcagattt tggtcaagac ccttacaggg 780

aagaccatca cccttgaggt ggagagctca gacaccattg ataatgtgaa ggctaagatt 840

caagacaagg aggggatccc accagaccag cagaggctta tctttgccgg caagcagctc 900

caggatgggc gtacccttgc agactacaac atccagaagg agtctaccct tcaccttgtc 960

ctccgtctcc ctgggggttt ctgaagtctg gtgattgcaa agcagcagct gtgaagatcc 1020

tgaatgtttt cttttatgtt tttacttttc gcggtacttt tattgcttcc ttgctgatgc 1080

aagtatttta atttcatgtt tggtattgcc tccctgttga ggtacttgga caagttttat 1140

ttcctatggt acttttggtt ttataaataa atttggatct tctcctcgtg ccgaat 1196

<210> 112
<211> 757
<212> DNA
<213> unknown

<220>
<223>  OProseR0948 housekeeping gene nucleoid DNA binding protein cnd41
      from rose


<220>
<221>  misc_feature
<222>  (709)..(709)
<223>  n is a, c, g, or t

<400>  112
ttcggcacga ggcgccagtc tgctctctac tcacatcagc cacaggtaat acacctggtt      60

gctcaagcgg cacatcaact tgcatatatg ccatacaaga acgagataac tccttctccg     120

tgggatactt tagcaaagat aggttgacac taacgtcaac tgactatttc gacgggtttc     180

tcttcggttg tggccaaaac aatcaaggcc tttttggcgg gtctgcaggg ttattgggct     240

tgggccgcaa caaaatctcc ctcgtcgaac agagcgcgca aaagtacagc cgcttcttct     300

cctactgcct accctcaact tccagcgcca ctggttacct cagcttcgga aaaggcggcg     360

gaccttccaa cgccgtcaag ttcacgccgc tatccactgt atctgaaggt gggtcctttt     420

acggcctgga tgtcgttgga atcaatgtcg gcggacgtag ggtatcgatt cccgcttcag     480

ttttttcgtc ctcagggacg atcatcgact cagggacggt gataacgcgg cttccggcaa     540

cagcgtacag tgctttgagg gatgcgttta agcaaggaat gaaaagctat ccacaagctg     600

aggagctttc gatactggac acgtgctacg acctgagcgg gagcagcaca gtgtcgtatc     660

ccaaaatagc gttcgctttc agcggtgggt tgactctgga tttggatgnc acaggcatat     720

tttacgtggc cagtgcttcg caggtgtgct tggcgtt                              757


<210>  113
<211>  2289
<212>  DNA
<213>  unknown

<220>
<223>  AJ271698 - Agaricus sequence

<400>  113
   gcgatacacc ctttctctct ctcccgcacc cctaggtcct ctcacgacat caacccctcc      60
   cgtcctcgct tcaagttggc aaccccctcc ttggttgata tatgcgcctc ggtcgatctc     120
   agtcttttgc taggcatcca agaggtcgct agtcttgccc ttccgaatga tatctcgaca     180
   gactcggatc tctgcctatg tcttcaggat ctcgatcttt acttggatag tgacaccagg     240
   ctcgcttggt tgacaaacct catcggccag gacgccttgt tgacgtatct gacagctctc     300
   atcgacacct caccatctcg ccaagactgc attctgcctg cgcacgcgca tcgtacttgc     360
   aatgatcagg acgtctgcca ctttgattgc gatacaaact tcgtccgttt tggtaattct     420
   tgcatttgca atcctccgtt ctctgtctgt aacggcgttt gcggaaattt caatgggtgt     480
   ccatcttcca ttcctcgaat cggtcgccgc gaaccagaga acgttacagg tattggacac     540
   gcaaaatcag gcgtcaagtc gctacacgtt gaacgggtca cttgtgtcga cggcgaatgt     600
   ttttccaaga cctgtgccgt ggggtggatt tattccaaag aggaagacgc ctgcattcgg     660
   agcgcgacca tgtcccgtcg taataaacgt tctgtggcgc gacagcttgg agatggcact 720
   ataacactcg ataaccacgt cactatcaac atggtccgcc ttcttcgggc tataggcgaa 780
   ctttatacac attcttcctt tctccgcaaa cagtctgatc ctcgggcacg ttcaattatg 840
   cggtcttgcg ataggcttga ccaactcgct attgatatgg tcggatcgga caccctcggt 900
   tctttccttg acagcactta cgggctcttg aatgttgcac agcttcttga tggattgctc 960
   tcttccttga atagcacggg attagaactc ttgcaagccg accttggaac tatcgttgcg 1020
   tcgagcacca atatcctgaa ttggtaccat tttcctggat ctggcgtttc tgggtctggc 1080
   gctcctggat cgaatacgtc tggatcgtcc tcttctgtcg acttggatac tccgattacg 1140
   atcacgctca accgctggct tggtgatctg ggacttaatg gagtcaaaac tactgtcaga 1200
   tacggaggcc tcggtggact tggcactgct atcaacggac tgttggatgg tctcggaatc 1260
   ggcccagctg acgagggcca ggcgctcccc gcgaacgcta gtttcgacga tgctctgcat 1320
   atgcaaatgg gggcgttgtt aggctatgcc ttagacgtgg gcagctctgt tcccccggcc 1380
   agtcctgctc ctcccattgg tactgcttcg acaagttcgg ctcctcaagt tggttccgcc 1440
   cccccagctg gatctgctgt gactggtgaa tctctagacc tccaaacgat actagctcct 1500
   gtctcacggg ccactctggg actcttgctg gcaacggatc tttctgcgct tttggattat 1560
   gttgacttga tcattgcggc atcgactaat gcccgaacaa ctctagacct ttacgataat 1620
   tttgatggta tgacgactca gttatctctg gtgaccaaga cggcattgga tgtgaagtct 1680
   ctttgctcga aacaactagg tagtgacgcc tccttagctt caggacgaaa ggttgctgcg 1740
   gcaaaggcac ctggcgatgg ttccgtggtt atcccgcttg acggtctttt gtacagccta 1800
   ggtatatctg ggaatgtgac cgtcggagga cttggcagag gtttttcggc cgctttgaat 1860
   gggattttga acgcgttcaa ccttggggct actcgggcaa ggcgggctgg tggtggaagc 1920
   gccgccgtca attccgccat cttcatggac tcgggtctga cgcaattatt ccgagcagca 1980
   gttgagaatg ccatagatct ggagatgaag acgggttcct cgccgggacc agttgcttcg 2040
   ctattggctg gctcgatgtc ttcggtgata tcgtcgatgc aggcaatgct cggttctcct 2100
   acagtagctg gactgattgg agaagtagac ggcttgttga gagcgactgg aaagatgaac 2160
   aggtcgctga cggcttgcca ttgtacaagc ttgcccgtag aagagtctac gggacggttt 2220
   gttggggctg tgaaggaagt tggaaggtgg ttggatgagc acgcaaggat gagccgtgaa 2280
   gccattttg 2289

<210> 114
<211> 885
<212> DNA
<213> unknown

<220>
<223> AJ271702 - Agaricus

<400> 114
ccctcgaact cgttcatact tttcctcgag aaaatttaca tggcatccta tcccgaactc 60

catgccaggt ctcgtcaacc tcgttctcgt caaccacggc ccattttaaa gtcgctaccc 120

gagttcaccc cacttccatt ctcaacctat ccgctcgatt cccctcatgt ccattttccg 180

ccaactccaa acctcacttc tacgactttt acgcatgccg ctggtgttta cgaccgtgcc 240

cctataactg tatccgaaaa cgtttgcgcg cttcccgaac gtggtgggcg atgttatatg 300

ctacagtctc agggtatccc tccgtcaggg ggagtggagg ggcggtatga tgattcacgg 360

aggagacgag acagtaatga attcaaggga aattattttc accctcgcgc attcaaggtt 420

tgcgaagttg aacgtgataa caatcatccc gaccgctacg agtcctctcc accaatccca 480

gacttgttgt caacgcattc ttctacctcc tcagattcat cctctccgtc aacaccgtcg 540

gacttcaaat cagattcatc atatggttct cttactacat cttcgatacc cactctctcg 600

tcgaatttgt cactcgacga gacctcatcc gtatacccgc tagagacatc agtccgtgac 660

catcacggat gtctaccatg gcaagcgtat tactcgccat gtaaccaggc aattgtaagt 720

tacgtgtgtc ccgctagtgt ttcggaaagc aagtcgaaga aaacagttca tggaaggaaa 780

atggcaaggt caaaaggttg tggtgagact gcttcacgac tggctaagtt tggtggggga 840

ttctcagacc aggccattgt attggaagga tgtttgggtg gtttt 885

<210> 115
<211> 816
<212> DNA
<213> unknown

<220>
<223> AJ271701 - Agaricus

<400> 115
atacagatcc ccactctcct tcgcccccac cagtcttttg agcctcggtc tgactaccat 60

ctgctatcca accctttcca aatcctagcc atgtttacct tcgcctcctt cgctcttttt 120

gctctcgcca tctcttccgt gaccgctctc gtcgtccctc gaggtcctga cgcccccgct 180

gactggtcca gctctctcga ggactatgcg ggatatcatg cccgctattt ggctatcagc 240

tgctacacaa agcacaatga ccctgctttc tttgaccgct gctgccatcc tcttcagaat 300

ggtgcccagc caaacccgtc ttgtccgtca ccttcggccg tcgatgacag tttggacaat 360

gatgatgact gcgaggatga ctccagttca gcacccaccc caacagcaag tccgactact 420

gcaacaagtc ccactccggc tccccacaac aattctgctg caaaggaaca agataccgat 480

gtcgacctcg ccgtagatgc ccatatcgtt cagggcggaa tcgcaacttt ctattatcag 540

caaggcaatg cgggagcatg cggacactac aacagcgatg atgccctcat tgccgccatg 600

gactacagaa cctacgggaa tactagccgc aaatcgcctc tttgcggcaa acaagttgag 660

atcacaaata caaagaacca caagaaagta gtcgtaacca tcgccgacgc atgtcccaca 720

tgcaagaata aaaactctat cgacctttcc gttcgtgcct tcgaaagaat tgccaccaaa 780

gacgagggcg aggttcccat tacctggaag tatctc 816

<210> 116
<211> 1080

<212>   DNA
<213>   unknown

<220>
<223>   AJ271693 - Agaricus

<400>   116
cggcttgcag ctggtttctt gcttgcttcc tttctctttt ctggattttc caatggatgt      60

cccgttagct caacgctcag taactcggtg gatgatgttg acgatcgcgt tttcgtaacg     120

atgcctactg cggctctcag acccgcgtca ggctgctttc ctgggttcaa tttcaagatg     180

cccgaaagcc ctcccgcgtc cttaactggc tggtggtgtg actgggacac cgaatatgca     240

tttgttggtt ttagctatga ggtttccgca tgccagagtc ttcacacgct gaaaactgaa     300

ttcatggata tcagacagcg gtttcacggg cgctatgtac gaatttacgg aacttgtgat     360

cggaaaggtt tcaatgatga tgtgattaat gcagcatggc acgctgggat cggtgtacac     420

gcactcattt ggttcggatt cgacggagac aataaatgga aaggccgacg cgacgctttg     480

ttctcgacgt tacattcgaa cccatatgcc aaatttgtcg tccgtgtggt ccaattcggt     540

tctgaacctt tgtttgatgg cgtcttgagc tcgagctcct tagcaaaaga agtgtggcgt     600

gcaaaatccg aacttgccaa acttcaaatt ccagtaactg tcagcgatat ggcttattca     660

taccaaaagg atcgcgcttc gagccatgtt atagatgcca tcgacattat tgatgcccat     720

attctgccct ttttctctcg ggatgcttcg acagcaaaaa aatcctggcc actcgtccaa     780

caagatctaa actggttttt gaataacggt caaggaaaga agatatacct tagccagaac     840

ggatggccgt caaaatcata ccctggagta gaaccaaaca gtgacaaggc ggtagccaat     900

gtacaaaatg aaaaggacta cttcaatctt cttgatgctc attgctcaca tttcaaatca     960

gcgcccggag gaggaatagg ttggtttgcg catatatact ctgataatca ggaaccgggt    1020

tatggaatct atgataccaa aggaagactc aaattccctt ttagtccccg tgttacttgt    1080


<210>   117
<211>   1098
<212>   DNA
<213>   unknown

<220>
<223>   AJ271707 - Agaricus

<400>   117
cagttgaaga gtgtacggac ctttatccga aacgtaatgg aatcaccgga cgagttcagc      60

gaatggatac atttctacac atcctcatca atcatggaaa tcatctatgg catgaaagct     120

aaacccgaag atccatatgt cgacaatgcc aagaaagcca ttgagggttt taacgaagct     180

gctgttccag gcaaattcct ggttgaaact ttccccgtca tgaagcacat tccgagttgg     240

tttcccggag ctgggtggaa aagacaagca ctgttttgga gagacgttaa tcgcgaggtc     300

cgtgtcaggc cgtttaatct cgtcaaagat caagtgaatg aaggtactgc cacgcggtcg     360

gtatgtagga cgttgatcgg aaatctgcct gactcaaccg cgcccgaccg gattgtgaaa     420

gaaaatattg ccatcgatac atgcgccgtg tccttcatag gcgctgctga aactagtcat     480

tctgctgccc gggtattttt tatggccatg cttatgaatc cagaagttca aaagaaaggt     540

caagctgaac ttgacaaggt tctcaatgga cgtcttccgg aaccaaacga cggtccaaat     600

ttgccctaca taagcgcgat ggtgaaagaa acactgcgat ggcaacttgt attacccctt     660

gccgttcccc atgttgccat cgaagctgat gagtacaatg gatactacat acccaaaggt     720

acaattgtct ttggaaactc ttggacgttc atgcatgatc ctgaagtcta caaagacccg     780

gaatcgtaca tgcctgagcg tttcctgaaa gacgggaaac tcgacagttc tattcgagac     840

ccatcgacag ctgtattcgg ttatggtcga aggatttgtc ccggccgata ttttgctctg     900

aatgctctct acttgatgat cgcacatacg ttggctgttt tcgatatcaa gcctgcgctt     960

gatgaaaacg ataacgagaa agagttcaag gctgatgtta cagggggaat gatttcgcag    1020

cctgtacctt tccagtgcat gattgtgcct cgttcaaagg ctgcggctga cttgatacag    1080

aacagcgacc tcatggaa                                                  1098

<210>  118
<211>  423
<212>  DNA
<213>  unknown

<220>
<223>  AJ271696 - Agaricus

<400>  118
tcgtcgtcga tccaatcgcc cctagacagg gtatataaac cactggtttc tctaaatcgt      60

atcttcagca tcttcatctt caacaccatc atcatcatga cttccaaccc ttcccttaac     120

aacaccgcct ctagtgaacc ttccaaggtc tccggccaac ttaactctgc catgggcacc     180

gccaaggaga ccgtcggtaa aatgactggt gcgacttctt tacaacaatc cggcaaggag     240

gaccacgccg ctggtgaagc tgagaacaag gccgctcaaa ccaaaggtca cgccgaaggt     300

atgacggacc aaatcgctgg aaagaaggat tccatgatcg gcgctatgac tggcgataag     360

aagcaacaaa cttcgggcaa tatgcgcgag gacaagggtc aagccgagaa gaaaatgaac     420

tcg                                                                  423

<210>  119
<211>  1916
<212>  DNA
<213>  unknown

<220>
<223>  X85113 - Agaricus

<220>
<221>  misc_feature
<222>  (1903)..(1903)
<223>  n is a, c, g, or t

<220>
<221>  misc_feature
<222>  (1906)..(1907)
<223>  n is a, c, g, or t

<400>  119

ccggcacgag cttgtttctt cagagtttcc atccgctctg tctccgcact ctcttgacca 60
ttccactctt tttttctttt tgatttagat gtctcatctg ctcgtttctc ctcttggagg 120
aggcgttcaa cctcgtcttg aaataaataa ttttgtaaag aatgaccgtc aattctctct 180
ttacgttcaa gctctcgacc ggatgtacgc cacccctcag aatgaaactg cgtcctactt 240
tcaagtagct ggagtgcatg gatacccact catccctttc gatgatgcag tcggtccaac 300
cgagttcagt ccttttgacc aatggactgg gtattgcact cacggctcaa ctctttttcc 360
aacttggcat cgtccttatg ttttgattct cgaacaaatt ttgagtggac acgctcaaca 420
aatcgccgat acttacactg tcaataaatc cgagtggaaa aaggcggcaa ccgaattccg 480
tcatccgtat tgggattggg catctaatag cgttcctcct ccggaagtca tctccctacc 540
caaagtcact atcacgactc cgaatggcca aaagacgagc gtcgccaacc cactgatgag 600
gtatactttc aactctgtca acgacggcgg tttctatggg ccgtataatc agtgggatac 660
tactttgaga caacccgact cgacgggtgt gaacgcaaag gataacgtta ataggcttaa 720
aagtgttttg aaaaatgctc aagccagtct tacacgggct acttacgaca tgttcaaccg 780
cgtcacgact tggcctcatt tcagcagcca tactcctgcg tctggaggaa gtaccagtaa 840
tagtatcgag gcaattcatg acaatatcca tgtcctcgtc ggtggtaacg gccacatgag 900
tgatccttct gtcgccccct ttgatcctat cttcttcttg catcatgcga acgttgatcg 960
actgattgct ttatggtcgg ctattcgtta cgatgtgtgg acttccccgg gcgacgctca 1020
atttggtaca tatactttga gatataagca gagtgttgac gagtcgaccg accttgctcc 1080
gtggtggaag actcaaaatg aatactggaa atccaatgaa ctgaggagca ccgagtcgtt 1140
gggatacact taccccgagt ttgttggttt ggatatgtac aacaaagacg cggtaaacaa 1200
gaccatttcc cgaaaggtag cacagcttta tggaccacaa agaggagggc aaaggtcgct 1260
cgtagaggat ttatcaaact cccatgctcg tcgtagtcaa cgccctgcga agcgctcccg 1320
ccttggtcaa ctcttgaaag ggttattctc ggattggtct gctcaaatca aattcaaccg 1380
ccatgaagtc ggccagagct tctcggtttg tcttttcctg ggcaatgttc ctgaagaccc 1440
gagggagtgg ttggttagcc ccaacttggt tggcgctcgt catgcgttcg tccgttcggt 1500
caagaccgac catgtagccg aggaaatagg tttcattccg attaaccagt ggattgccga 1560
gcacacgggt ttaccttcgt ttgcagtaga ccttgtaaaa ccactcttgg cacaaggttt 1620
acagtggcgc gtgctcttgg cggatggaac ccctgctgag ctcgattcac tggaagtgac 1680
tatattggag gtcccatccg agctgaccga cgatgagcct aatccccgct ccaggccgcc 1740
caggtaccac aaggatatta cacacggaaa gcgtggtggt tgccgcgagg cttgataggt 1800
gttattcatt ggacattgga cttgttgcta gaagtatata gataaagtta gcgtacatgg 1860
tttaattgat ttaccttgtt tgagaaaaaa aaaaaaataa tgntannaaa aaaaaa 1916

<210> 120
<211> 280
<212> DNA
<213> unknown

<220>
<223>  SSH03nr023 from Agaricus


<220>
<221>  linker
<222>  (1)..(23)
<223>  putative SSH linker

<400>  120
tagcgtggtc gcggccgagg tacgccaatc acatgtcatc gttgggcact ggtacgcgaa      60

ctgtgtgtgt tcggcgctct cagcgggatg atagtgtatg taggcgtgac cacgtttcac     120

gcattcgtga cggtgtcctt tataattcca ccccagtccg gcgtaatgcc agacctcctg     180

ttcgccaagg ggggtagttg gttgagttag ttttccactg ttgtcgggtt gggccgccat     240

ttgtggatag ttgtacaagc tttttttttt tttttttttt                           280


<210>  121
<211>  273
<212>  DNA
<213>  unknown

<220>
<223>  SSH5nr012 - Agaricus


<220>
<221>  Linker
<222>  (1)..(25)
<223>  putative SSH linker

<400>  121
ttcgagcggc cgcccgggca ggtaccgcag gaacagcggc agccgcgtcg caaaaggtgt      60

tcacaaatcg ggaacagtca gttgcgggtc ccgcagaagt gggtactgcg acgttttcac     120

gtccgcaagt ttggcagggc ccttcaaagt caggttgtct tgcacgaggt cggaatgtct     180

tcatggaaat gaagaaagtt tcgtcggtga acatgttaat gtcccaggtc aacccgaggt     240

caccttcgac gtacctcggc cgcgaccacg cta                                  273


<210>  122
<211>  229
<212>  DNA
<213>  unknown

<220>
<223>  SSH5nr019 - Agaricus


<220>
<221>  Linker
<222>  (1)..(23)
<223>  putative SSH linker

<400>  122
tagcgtggtc gcggccgagg tacgagaacg gatcccgctc ctgtcctgca ggcgactact      60

gctatctaac gcgcaagtcc gcacgggcct ctagttgtga gcccgaacct attgaacacc     120

agacgtctcc catatgtatg cttcaactct caccaaaggg catcactcct ctggtgcgcg     180

tgctcatgaa tcacgatatt ttgagtacct gcccgggcgg ccgctcgaa 229

<210> 123
<211> 330
<212> DNA
<213> unknown

<220>
<223> SSH07nr010 - Agaricus

<220>
<221> linker
<222> (1)..(26)
<223> putative ssh linker

<400> 123
tttcgagcgg ccgcccgggc aggtacccca tttccaggac atcatatttt cgcagctgaa 60

gttgacttta accttgagtc gagtagaaat cgtatccggt tttgggatat cgttctttgt 120

taccgcgata gtcaaacgat tattgtcgcg tttgataatc tgctcggtag atactgtgat 180

agtatcatta tttctacttt gggcacgtcc actcctgcgg ttcccgcggg ggcgttgctt 240

gtttgatctt tgttgtgccg acatgttagg tgggtaagtt ggttggttgt tagttagtta 300

gttaaaagta cctcggccgc gaccacgcta 330

<210> 124
<211> 327
<212> DNA
<213> unknown

<220>
<223> SSH07nr032 - Agaricus

<220>
<221> linker
<222> (1)..(23)
<223> putative SSH linker

<400> 124
tagcgtggtc gcggccgagg taccccattt ccaggacatc atattttcgc agctgaagtt 60

gactttaacc ttgagtcgag tagaaatcgt atccggtttt gggatatcgt tctttgttac 120

cgcgatagtc aaacgattat tgtcgcgttt gataatctgc tcggtagata ctgtgatagt 180

atcattattt ctactttggg cacgtccact cctgcggttc ccgcgggggc gttgcttgtt 240

tgatctttgt tgtgccgaca tgttaggtgg gtaagttggt tggttgttag ttagttagtt 300

aaaagtacct cggccgcgac cacgcta 327

<210> 125
<211> 143
<212> DNA
<213> unknown

<220>
<223> SSH07nr041 - Agaricus

<220>

<221>  linker
<222>  (1)..(25)
<223>  putative SSH linker

<400>  125
ttcgagcggc cgcccgggca ggtacatggg cttgttagct gccaaagatg ctaccagacg      60

cccgacccta tcccttcctg acggaccttc aattgatatg agtggtgtta tgttcgcggc     120

gtacctcggc cgcgaccacg cta                                             143


<210>  126
<211>  656
<212>  DNA
<213>  unknown

<220>
<223>  SSH07nr083 - Agaricus


<220>
<221>  linker
<222>  (1)..(25)
<223>  putative SSH linker

<220>
<221>  misc_feature
<222>  (564)..(564)
<223>  n is a, c, g, or t

<220>
<221>  misc_feature
<222>  (593)..(593)
<223>  n is a, c, g, or t

<400>  126
ttcgagcggc cgcccgggca ggtacagtcg taaagttgag aagcgctttc gcgaaggcag      60

ttctactgtc ggcagtagca accgaagaag agaaaaatct cgagccagac caccctccaa     120

cggatatcat tgttttaaca ttttttgctc tagcttgttc gacgaaatca ttgaaggcag     180

cttcgccatc cggcagttca agattttgct gaacatcctc actaggaaca gcaaaaccat     240

aagtcacgta tgaatatttt tcccaaggaa tttgacttgc ggtgaacttg tcactcgccc     300

tcgaaccata ccaaccacca taccagccag tgaccagttt actttgtgtt tgtgatgttg     360

cagttgcgtt ggaagcagca gagttgatgc cactcgacgg tcgttgccga gagcaggttg     420

gcgcacaaaa gacaaaggaa gtgagggcga caatgacaag gaaggctttc atcgcgaatt     480

atagtgtatt tattttcacg aatcaatcgt gcgcgtttca aggagcggat ggtatatggt     540

tcacgaccag cgtaataaga gagncaacgg actagcagga gcgagagacg tcnaattggg     600

cgatggggag tgaagagaag agaaacaggt ttaatgccca ttttgtacct cggccg         656


<210>  127
<211>  369
<212>  DNA
<213>  unknown

<220>
<223>  SSH09nr088 - Agaricus

<220>
<221>  linker
<222>  (1)..(25)
<223>  putative SSH linker

<400>  127
ttcgagcggc cgcccgggca ggtaccctca acttgaattt catgtaacag cgctggaaca      60

tctggtagca catgccttcg cccaagtatc tttttcccgt gttgcatcaa ttctgcgaca     120

gaatgtcgac catctcgtat tcgctcttga agaaccgaag caatcaaggc cactgcctct     180

gtcttattca acttgacacc tcttgcaagt cgcttttgcg caataaatcc aacctggtgg     240

aggaggactt tggcctcttc gcgcgggagg agacgcatga cccttttcta ttttctttca     300

ggagaatgaa aatgggtctg agacaactgg tagcagcaaa cagaggaaac ctcggccgcg     360

accacgcta                                                             369


<210>  128
<211>  567
<212>  DNA
<213>  unknown

<220>
<223>  SSH10nr021 - Agaricus


<220>
<221>  linker
<222>  (1)..(23)
<223>  putative SSH linker

<400>  128
tagcgtggtc gcggccgagg tacgaaaacg atcgtcacac tctgggtcgt agtctggttt      60

ctcgaatccc tcagatctta accgcctcag atctcggcaa acggggctta gggaccaccg     120

aaccacgaga tgtttacaat actcttgtca ggcgttatca ggatgtgctg tcctcatctc     180

tacaactgcg cgcgttgtcc caggattctt ccgttgcgga acccactgtc gttgaaagct     240

tgactacgta taaaaccagt atatcggaat ttcaatctgc ccttgcatat ctgggacgtg     300

acaaaggact ggccaattac aataaggatg acaagctcga gactttactc aaggacctgg     360

tcaaccttca caaacacacc ctatcctata tcgatgtagt cgtctatcaa ctcccggtgg     420

ttggccctat gctcggaccc attgtttacg atatcaagtg catcctggac gaagttctta     480

acgctgtaga aaacatcagt gacggcctac tcaacgtcct cgatcctctc ctaataaaaa     540

ctgtacctgc ccgggcggcc gctcgaa                                         567


<210>  129
<211>  235
<212>  DNA
<213>  unknown

<220>
<223>  SSH10nr028 from Agaricus


<220>
<221>  linker
<222>  (1)..(23)

<223>  putative SSH linker

<400>  129
tagcgtggtc gcggccgagg tactctttta ttctgcatcg gaatttcaac atttgtattg      60

cactagcatc aataggcatc ttctatcttc agagatcttt ttaacgcttc cactctccac     120

catttcgaag ccgttaaaaa ataccctaca cccttttcgt ttttccgtgt attaatgtgt     180

tgcataaaca caatcgtttt cgctttcgtt gtacctgccc gggcggccgc tcgaa          235


<210>  130
<211>  327
<212>  DNA
<213>  unknown

<220>
<223>  SSH10nr080 - Agaricus


<220>
<221>  linker
<222>  (1)..(25)
<223>  putative SSH linker

<400>  130
ttcgagcggc cgcccgggca ggtacagaac ataataagtc ggtcgtatac gcagcgactg      60

tgtctgcagt atcgagggag ttgtcgagag aatatttcgc atcgatctgt tgccattcgg     120

cgatggtagc cagaaacgcg gtttgccctc gacgttgtct cgtcgcctcg aataacacag     180

caccgaaaga aacgatagtc actgaggaaa catgagtgca acactattat tatttttaat     240

ggattaatgc gggcctacca tacaccaggc ctttcacttt ggatcgccga tttccatctg     300

aacggtacct cggccgcgac cacgcta                                         327


<210>  131
<211>  254
<212>  DNA
<213>  unknown

<220>
<223>  SSH11nr017 - Agaricus


<220>
<221>  linker
<222>  (1)..(23)
<223>  putative SSH linker

<220>
<221>  misc_feature
<222>  (158)..(162)
<223>  n is a, c, g, or t

<220>
<221>  misc_feature
<222>  (241)..(241)
<223>  n is a, c, g, or t

<400>  131
tagcgtggtc gcggccgagg taccgtcgca gcttccttgt gtttcccgtg ttcaacaagc      60

catctcggcg attctggttg gaaaaacatg gctatgatga acaagaggct cggaatcatt     120

tggataatta acggaattcg ccattgcatt tcaccaannn nnggcacgtt tttcgacacg     180

ctgtagttca cccagaagcc catcatcaga ccactgttag tcgtaaattg gagtgttcca     240

ntacctgccc gggc                                                       254

<210>  132
<211>  386
<212>  DNA
<213>  unknown

<220>
<223>  SSH12nr023 - Agaricus

<220>
<221>  linker
<222>  (1)..(23)
<223>  putative SSH linker

<400>  132
tagcgtggtc gcggccgagg tacgcccact gagttcttca cgagtccgct tttcccacct      60

attgaggaga atcgtctttt ctccagccaa ctgcgcgtca atacgaaact ccttaacttc     120

accaccacca atccaccgta agagtttccg caggccattg cggtcggtta caaggtccat     180

agttccccaa ggaaacggat cagcgccttc atttttgcat ttctggttga cagccacgag     240

caatggcaat aacgatgcag agggcatcct gtgggtattt tgatcgatga taaaaatacc     300

cgagtcaggc ttgacattgt aaggcatggc cctgttattc cattcaggag cggagcccgg     360

tacctgcccg ggcggccgct cccgaa                                          386

<210>  133
<211>  540
<212>  DNA
<213>  unknown

<220>
<223>  SSH12nr053 - Agaricus

<220>
<221>  linker
<222>  (1)..(23)
<223>  putative SSH linker

<400>  133
tagcgtggtc gcggccgagg tacacagata agcttaaaac aacaagcgac taaattagcg      60

tgataaacga gcaaaggtaa gcgctggggt atatgtttcg tagaagtggt catcgagatg     120

tcagcacgat agacgatggg acttccgcag tattgtagat aatgtgaatg tgtcaacgtg     180

cagacggtcg tggcaaaggc atggtttact cttcatcagg ctcaccgctt acttgaggct     240

ctaatccggc tttgagtagc tgatctttca gtatccgcat atcatcctgt aatctcgtca     300

cagtctcatg ttcgtcatct tgttccgact ccatgaactg gatttccatc actttcctcg     360

atacctccga accgagctcg tcgatctttt tctgtgcatc ctccagctgc ttctgcattg     420

ccttcatatt cttttcatgc tcggcttgca gatcgcccaa tttgaggtcg tgagcctcgt     480

gtaattggcg cagcatctca tccgttacaa tgccagtacc tgcccgggcg gccgctcgaa     540

<210>  134
<211>  342
<212>  DNA
<213>  unknown

<220>
<223>  SSH13nr021 - Agaricus


<220>
<221>  linker
<222>  (1)..(23)
<223>  putative SSH linker

<400>  134
tagcgtggtc gcggccgagg tacgtaaagg cgaaagtgcg accacaagtg aatgtgaccg      60

aacagacgtc taccgaggcg tatagctggt tcctctacgg gttctagccg caaccacaaa     120

ctgatttggg aaaccgaaag acatttacgg atagcccttt tagatggatg gggtttgata     180

acccatcact ttattgcgca ttatagcaag cgaggcttaa agggaatggg agccatttat     240

atgtatggtg cagcaacttg aatacacatc gcacagtgca aaaagaccga ttggtagtaa     300

tattacgcct cccaaaggta cctgcccggg cggccgctcg aa                        342


<210>  135
<211>  358
<212>  DNA
<213>  unknown

<220>
<223>  SSH13nr086 - Agaricus


<220>
<221>  linker
<222>  (1)..(25)
<223>  putative SSH linker

<400>  135
ttcgagcggc cgcccgggca ggtacggatc ttatccgctc ctgtcctcca gtcggcaaaa      60

ctggtcaagc gctaatagat gatgctatgt tcttttttca ccctgaacat tgcattaagc     120

cctacgacgg ttcggtttcc cgtattagtc gatgggcgca tcaccgtcgc aagccttcct     180

tacaacactc tcctgactac taagatccta acagacacgt gttgacagga gccttcttcc     240

aattgagtta ggaattagac tccgatgatc tggcttacag ttcataatca atctaccata     300

caacaaaaac tacgatttcc acatacattg gtagggtacc tcggccgcga ccacgcta       358


<210>  136
<211>  331
<212>  DNA
<213>  unknown

<220>
<223>  tomato indicator gene 1Fnr092 - downregulated sequence

<400>  136
acgcggggac cacacaactt tcttcttctg ctcatcaatt agcaattaat ccaaaaccat      60

tatggctgcc aaaaattcag agatgaagtt tgctatcttc ttcgttgttc ttttgacgac 120

cactttagtt gatatgtctg gaatttcgaa aatgcaagtg atggctcttc gagacatacc 180

cccacaagaa acattgctga aaatgaagct acttcccaca aatattttgg gactttgtaa 240

cgaaccttgc agctcaaact ctgattgcat cggaattacc ctttgccaat tttgtaagga 300

gaagacggac cagtatggtt taacataccg t 331

<210> 137
<211> 435
<212> DNA
<213> unknown

<220>
<223> tomato indicator gene 1Fnr176 - downregulated sequence

<400> 137
cagacaacac tatacgcgca gagacagata aatagataga tagatcgatc gatagatcat 60

tgatattgtt caaggcaaca ggttgcatgt acgcggggag ctcaaaaatc aaaatcctat 120

cccctctgct ccttcaccaa aaatggagtc cctccttctt acctcttcac ctgatatcgg 180

gaagttgctc agctcctctt cctcctcttc ttgtaaatgt tatccttcag tttcagcaag 240

tgggcaccct tctttcttcc tatattttcc ccacaagaac aaccgaattc cctctgttat 300

tgtttcagca tcaaacaaga agaagaagaa atctgactcc cacagcttta ttcctaaacc 360

tgatgaagcc actggtcctt ttcccgaagc tgtgctcctt atagagagga aggtccaaga 420

agatggtata cttgt 435

<210> 138
<211> 255
<212> DNA
<213> unknown

<220>
<223> tomato indicator gene 3Fnr355 - downregulated sequence

<400> 138
accatacccg cagcagcaac cttcgcagcc atcttcttcc acttcaactt tagcaaatac 60

aaccaaaaca aattgttcaa aaacctaaca acctaaatat tcgaatcatc tattgccctc 120

gatagcatta agagtgaatc gacggcaccg gtagaggctg agacacggcg gagtgatacc 180

tccggtaggg gggaagagga atggtgaaag agggaaaaaa aaaaaaaaaa aaaaaaaaat 240

ttccctgccg ggggg 255

<210> 139
<211> 419
<212> DNA
<213> unknown

<220>
<223> tomato indicator gene 7Rnr558 - downregulated sequence

<400> 139
ctgttttacc cactccagct cccccgaata gtccgatttt tcctccacgg cgataagggg 60

ctaaaagatc tactacttta attcctgttt caaaaataga taattttgta tccaactgta 120

taaaggcggg cgcagatcta tgaataggag acgttgtacg cggggataac taataatatt 180

actgattaaa tatctcataa aaataagaaa aatgaaaatg atgaaatttt ttgtgcttgt 240

tgtaactatt ttagcattgt tgctaagtgt agcaaatgca caacaatgtg gaagtcaagc 300

tggtggagct ttgtgtgcca atgggttatg ttgtagtcaa tatggttatt gtggcactac 360

tcctgattac tgtggacagg gatgccagag tcaatgcaac taaattatgt tttgtgtgt 419

<210> 140
<211> 80
<212> DNA
<213> unknown

<220>
<223> tomato indicator gene 9Fnr046 - downregulated sequence

<400> 140
cgcggggatc cctaactaat aatctgactg attatctctc tcatataaat aaaataaatg 60

agaataatga aattgtgcgt 80

<210> 141
<211> 524
<212> DNA
<213> unknown

<220>
<223> tomato indicator gene 9Fnr216 - downregulated sequence

<400> 141
cgcggggata taaaaggata gtggacatca aaaggttcat attgaaccaa aaaaaagaga 60

gaagaagcaa tatggcttcc tctgtcattt cttcagcagc tgttgccaca cgcagcaatg 120

ttacacaagc tagcatggtt gcacctttca ctggtctcaa atcttcagcc actttccctg 180

ttacaaagaa gcaaaacctt gacatcactt ccattgctag caatggtgga agagttagct 240

gcatgcaggt gtggccacca attaacatga agaagtacga gacactctca taccttcctg 300

atttgtctga cgagcaattg cttagtgaaa ttgagtactc ttctagctaa ttgtttgttg 360

cctattgctg atgtttcatg tgctattcct atcacaacta aagcttgtga tgccttcatt 420

tcattaccac caagtggtgg ttcatcgttt tcgagatttt aagcttatga gttctgaaac 480

gcaggcgtat ttacttggtt cttgaaaaag tgatttacat gtgt 524

<210> 142
<211> 909
<212> DNA
<213> unknown

<220>
<223> tomato indicator gene 9Fnr244 - downregulated sequence

<400> 142
ctgtgagtta attccacaag aacaagaata tagtgtctgg tcatgaacac tatatatgta 60

cgcggggcat ccataactaa taatattact gattaaatat ctcataaaaa taagaaaaat 120

gaaaatgatg aaattttttg tgcttgttgt aactatttta gcattgttgc taagtgtagc 180

aaatgcacaa caatgtggaa gtcaagctgg tggagctttg tgtgccaatg ggttatgttg 240

tagtcaatat ggctattgtg gcactactcc tgattactgt ggacagggat gccagagtca 300

atgcaactaa attatgtctt gtgtgtacgc ggggacacat tctcacacac aacattttat 360

atatcctctt ttttttttcc taataacatc atgggtgtca ctagctatac acatgaaacc 420

acaacaccag ttgcccctac taggttgttc aaagctttgg ttgttgattc tgataatctt 480

attcctaagt tgatgccaca agttaaaaat attgaggctg agggagatgg aagtatcaaa 540

aagatgaact ttgttgaagg ttcaccaatc aagtacttga agcacaagat tcatgttgtt 600

gatgacaaga atttggtgac caaatattca atgattgaag gagatgttct tggagacaaa 660

cttgagtcaa tttcctatga cctaaaattt gaagctcatg gaaatggagg atgtgtttgc 720

aagtctataa ctgagtatca cacaaaaggt gattatgtgt tgaaggatga agaacataat 780

gaaggcaaaa aacaagccat ggaacttttc aagattgttg aagcatacct cctcgagaat 840

ccttctgtct acgcttaagt gatgaaataa gaatcaggtc cacacgtgtt atataacgtg 900

tttgtgaca 909

<210> 143
<211> 268
<212> DNA
<213> unknown

<220>
<223> tomato indicator gene Fnr323 - downregulated sequence

<400> 143
acgcggggac atccataact aataatatta ctgattaaat atctcataaa aataagaaaa 60

atgaaaatga tgaaattttt cgtgcttgtt gtaactattt tagcattgtt gctaagtgta 120

gcaaatgcac aacaatgtgg aagtcaagct ggtggagctt tgtgtgccaa tgggttatgt 180

tgtagtcaat atggttattg tggcactact cctgattact gtggacaggg atgccagagt 240

caatgcaact aaattatgtt ttgtgtgt 268

<210> 144
<211> 169
<212> DNA
<213> unknown

<220>
<223> tomato indicator gene Fnr349 - downregulated sequence

<400> 144
acatacatgt tcccacttta ttctagtaat acaaatttat atattttaaa cacacaacca 60

catggatata tattacctta ttattatcat agtcttcaag ctagaacgta cttatagact 120

ggaggtggtg gagacttgta tttgtaaacg ggtggaggtg gagacttgt 169

<210> 145
<211> 413
<212> DNA
<213> unknown

<220>

<223>  tomato indicator gene 1Fnr224 - upregulated sequence

<400>  145
acttctactg gtggctcatt taaatgtgcc caattgctac ttatccacca tccatttttc      60

ctgattagtt gatccaaact ttcctcattt tccaaattag aaagactttc ttccgctgtt     120

ttagttgcct cctcaaatac aatattcgcc tgtttttggt ttcgagggga taacgtagag     180

atatattttt gatgctcttc ttcaattagt ttttttgatc tccaatatgc atctatctct     240

ttctttgtta atgacttatt tgttcgtaat ttcatagctt gaggatcaga aacaggagag     300

tcccaacctg ccataagaga acccataatt atctcttttt gttgctaatt aacttcttaa     360

ttaaacttaa cacacagttg attaatttca ctcaacttgt tatatccccg cgt            413


<210>  146
<211>  340
<212>  DNA
<213>  unknown

<220>
<223>  tomato indicator gene 3Fnr100 - upregulated sequence

<400>  146
acaatagagc attggctagc aaacaacaac aaaaactaat gtctttgttg tattcatata      60

gcctcatatt catattggtg ggtcaatatt cattaagatc caacaaaagt ccaccacaaa     120

tcacttcttt cttcttcttc cattcttttc tggtccaaaa tttttgactt tttttgagtc     180

tttactcaca ctagttagta gtaggatctg atacgagtgc aacaatattt ctgaagagtc     240

tgagggatat aggctagtag tattcaactg aggtatcagg gcaacttcca tcagagagaa     300

attctttact gaatggagga ggaatgaatg gtggtgaagt                           340


<210>  147
<211>  122
<212>  DNA
<213>  unknown

<220>
<223>  tomato indicator gene 7Rnr298 - upregulated sequence

<400>  147
acggccttct gcgccgaaat tgtggaatca gctgatgctt acttgtttgc tggacctatt      60

tttaatgact atagctctgt tgggtattct ctgcttctca aaagagagaa agctatcatt     120

gt                                                                    122


<210>  148
<211>  629
<212>  DNA
<213>  unknown

<220>
<223>  tomato indicator gene 7Rnr310 - upregulated sequence

<400>  148
acaggctgca gaagggaggg tctacgtgga tgttggtttc tggggaggtt tggttccaga      60

aaatgcagaa aatacaagtt ctctggaaag acttctaaat gcaggggttc tgggattgaa     120

gtcttttatg gtgccatcag gcatcaatga ctttcccatg acaactgcat cccacataaa 180

ggaggcactc ccaactctgg ctcgatacaa aagaccccta cttgttcatg ccgaagtgct 240

agtagatctt gatgaaaaag tggagctgga ggatggggta gagaatgcta gatcatactc 300

tacatatctc aagaccaggc ctgcttcaat ggaagaggct gctatcaatc agctcataac 360

actgtcaaag gacgcgaggg ctggtggttc tgctgaagga gctcatctcc atattgttca 420

tctatctgat gctagaactt cactaaacct cattaaggaa gccaaacaaa ggggtgatag 480

catcaccgtt gaaacttgcc cccattatct cgcttttgca gctgaagata ttcctgatgg 540

agatactcgg tttaagtgtg ctccacccat tcgtgatgca gccaataaag aaaaactatg 600

ggatgcgtta ctggatggag atattgaca 629

<210> 149
<211> 387
<212> DNA
<213> unknown

<220>
<223> tomato indicator gene 7Rnr355 - upregulated sequence

<400> 149
cccacatcaa tggtaacggg caaacaagct gaaggacgaa tgccccccag agcagtgtat 60

aaagagagct tgcccactgg tatccccatt ccctggcaac caaggtcccc aaggcccaga 120

attcgttctc catcagtaac aacaataact tgaattttct tctcgggcca attttttaat 180

acctcgtgaa ttttgccttt ttctttcaag ctgataaaaa gaccttgagg acgcctgaag 240

atgctaccat atttctggca tgcttcacca acagttggag tgtaaactat cggaagaagc 300

tcctcaacat tgtcaataag aagcttgtag aatagccgct cattcatttc ctgaagatcc 360

atcatggcca tgtatctttg aagtggt 387

<210> 150
<211> 255
<212> DNA
<213> unknown

<220>
<223> tomato indicator gene 7Rnr478 - upregulated sequence

<400> 150
gcccgacgtc gcatgctccc ggccgccatg gcggccgcgg gaattcgatt tcgagcggcc 60

gcccgggcag gtacgcgggg aaccaaaatt tgtgttctat aaaaagtttt catatttagt 120

gatcactaaa aaaaaaatca agaagatgtc gactactgta ggccaagcca ttcgttgcaa 180

aactgctgtg gcatgggaag ctggtaagcc attactgatg gaggaagtag atgttgctcc 240

tccacaaaaa atgga 255

<210> 151
<211> 503
<212> DNA
<213> unknown

<220>

<223>  tomato indicator gene 7Rnr563 - upregulated sequence

<400>  151
ccaattgact cgaaaacagc tacagaggac aatgttttac cagatggaac ttttgttaaa      60

aagggaacaa gagtaagtta tcatccctac gcaatgggga gagcagagga actatgggga     120

agtgattgga acaagtttaa accagaacgg tggttggata aagatgaagt tactggaaat     180

tggaattttg ttactaaaga catgtataca tatcctgttt ttcaggcggg gccaaggatt     240

tgtttgggga aagaaatggc gtttttgcag atgaagaggg tggttgctgg tgttttacgg     300

cggttcaagg tggttccggt cacggaaaaa ggggtggaac cggtgtttat ttcatacctc     360

acggcgaaaa tgaaaggtgg tttccatgtg acaattgagg aaagggacca tgagtagtgt     420

tgttattttt ctacttgtct taaactattt tggatggata aaaaattact tttaattatg     480

aatataataa gaatatctgg ttc                                             503

<210>  152
<211>  482
<212>  DNA
<213>  unknown

<220>
<223>  tomato indicator gene Rnr567 - upregulated sequence

<400>  152
acgcggggat acataaaata aaaatatata tgttcataat tgttagaagt ggagatggga      60

aattgtttga gacatggaga atgcgtggca ccaatgacaa gtttagatgt agaaaaagaa     120

agattattat ggattaatac tggtaatagt aagattagag ggacaaaaat cagaataaca     180

aagaaggagc tagaggacat gttaagagga ttgaacatgc aagatgttac ggtagatcaa     240

gttttatcta caatattgat caattcaagg agccatggct atcatcatcg gaatcaacgt     300

caatggcgtc ccgttctcca aagaattcct gaggtttatt gaagaagtaa gaagattatc     360

gtttaggttc agaagaaatg aatttaagat ttaaattgta aatgattgat aagttagatt     420

gagaacatat gtaatagcag aactactttt ttgtggtaat gaatgaatgc taattttaaa     480

gc                                                                    482

<210>  153
<211>  527
<212>  DNA
<213>  unknown

<220>
<223>  tomato indicator gene 9Fnr019 - upregulated sequence

<400>  153
acgcgggaag aatataaaat cagcaacaaa atcgaaaatt cctccgtaca caacgacgaa      60

tacggtgacg acgatagagt aattgacggt agcagtggag ctgagagttt acgataatca     120

aagacgggag ctttaatttc agaaacccta gctcatccta atgcagaatc atccgtacaa     180

attgaaattg atggccaatc cgaattcgat tcagagtttg ctccctctta tcaactttct     240

gaagtaccag tggaatatga actgccctcg tttggtttct cggaaaaaat caaggataga     300

attagtgtta ccaaacctgg aactttgaat gctcaagcac gaacagaaca tcaacgacgt 360

gttcctgatg cagcatcctc attggagctg tcctctttat ctgttgcaca gtccatttca 420

tcagtgccaa gtgcaactct agcagaaaga cgatcagcag cagcagtaaa ttgtattaca 480

ggagaagtgg tttaacagag ttctgacgcc caggttctaa ctcttgt 527

<210> 154
<211> 388
<212> DNA
<213> unknown

<220>
<223> tomato indicator gene 9Fnr248 - upregulated sequence

<400> 154
acgcgggggg tgtacgcgct ggtgcattag gattactgtt gcaatctgtt gttctagggt 60

ttatgtcact tggggttgaa tttttaggga agaagatcgg tggtgctaag agactatggg 120

ggattttgaa cttcgtttta gctatttgct tagctatgac tattttgata accaaaatgg 180

ccgagaaatc tcgccggcac gacgccgccg gtacactcat gggcccgacg cctggtgtta 240

aaatcggtgc cttgcttctc tttgccgccc ttggtattcc tcttgcggta acttttagta 300

ttccatttgc tttggcatct atattttcta gtaatgctgg ttcaggacaa ggtttgtcac 360

taggagtgct caatcttgca attgttgt 388

<210> 155
<211> 633
<212> DNA
<213> unknown

<220>
<223> tomato indicator gene 3Fnr244 - constant expression

<220>
<221> misc_feature
<222> (581)..(581)
<223> n is a, c, g, or t

<400> 155
cgcgggggag gaggaagaaa tgggaggagg agaagaagat caacagaaat tgaagaacac 60

agcagcagca gcgtatggct acgagaatga tccgagatgg gcagattatt ggtccaatat 120

actcattcct cctcacatgg cttctcgttc tgatgtcgtc gaccatttca agcgcaagtt 180

ttatcaacga tacatagacc ctggtctagt ggttgagcca atggctacca gcagttcgtc 240

ccaaccagca aaaccatcag ttgctcaacc atcatcatca tcgtccacac cttctaataa 300

ccagccgtca cagcgtaact ctggtcaagc tagtagaact tcaggcacgc caactactcc 360

aacttcaaat ccgacatcac ttcgctggga ccgaaatact atacaatttt cagtgaatgc 420

ttgggttttt gttgttgcag tcctctcaat atttccgctt acacctgcaa atctgtcgaa 480

cagagcatat cgactgtcct tcttgggaac tgcatgttct tcgctatatt cattatactc 540

tgtatatggg aaaccgaggg cttggaattt acaagcagtg ncagtttggt tccagtcagt 600

tattgtgacg aaggatttca tctccttcat tta 633

<210>  156
<211>  216
<212>  DNA
<213>  unknown

<220>
<223>  tomato indicator gene Fnr268 - constant expression

<400>  156
atgttacagc ccgtctgacc tacaagaatg tccctacatg gcatagagat ctttgtaggg      60

tttgtgaaaa catccccatt gttctctgtg gaaacaaagt tgatgtcaag aacaggcaag     120

ttaaggctaa gcaagttacc ttccacagga agaaaaattt gcaatactat gagatctcag     180

caaagagtaa ctagcacttt gagaagcctt ttttgt                               216


<210>  157
<211>  408
<212>  DNA
<213>  unknown

<220>
<223>  tomato indicator gene L08255 - constant expression

<400>  157
atggaggagg agaaacacca ccaccaccac ctgttccacc acaaggacaa ggcggaggag      60

ggccccgtcg actacgaaaa agaaatcaaa caccataaac atctcgagca aatcggtaaa     120

cttggcactg ttgctgccgg tgcctacgcc ttgcatgaga aacatgaggc aaagaaagat     180

ccagaacatg cacacaaaca caagatagag gaagagatag cagcagctgc tgcagttggg     240

gcaggtggat ttgcattcca tgagcatcat gagaaaaaag atgccaagaa agaagaaaaa     300

gcaggtggat ttgcattcca tgagcatcat gagaaaaaag atgccaagaa agaagaaaaa     360

aaaaagctga gggggacac caccatctct tctaaattgt tattttag                  408


<210>  158
<211>  488
<212>  DNA
<213>  unknown

<220>
<223>  pear ACS1-6

<400>  158
aagtgacctt tgatcccaac cacttagtgc tcaccgccgg tgcaacttca gccaatgaga      60

cctttatttt ctgccttgct gaccccggcg aagccgttct tattcctacc ccatactacc     120

caggatttga tagagacctc aagtggcgaa ctggagtcga gattgtaccc attcactgca     180

caagctccaa tggcttccaa attactgaaa ccgctctgga agaagcctac caagaagccg     240

aaaaacgcaa tctcagagtc aaaggcgtct tggtcacgaa cccatcaaat ccattgggca     300

ccacaatgac cagaaacgaa ctctacctcc tcctttcctt cgttgaagac aaaggcatcc     360

acctcattag cgatgaaatt tactccggca cggcttttag caccccatcc tttataagcg     420

tcatggaagt tctcaaagac agaaactgtg atgagaattc cgaagtttgg cagcgagttc     480

acgttgtc                                                              488

<210>  159
<211>  456
<212>  DNA
<213>  unknown

<220>
<223>  pear ACS2

<400>  159
aactggggtt caaagggatc aaaaggagtg tctggcttta gagaaaatgc cttatttcaa      60

gactaccatg gccttttgtc tttcagaaag gcaatggcaa gttttatgga acaaattaga     120

ggaggaagag ccaaatttga ccctgctagg gtagtcttaa cggcgggtgc aactgcagca     180

aatgagctat tgactttcgt catagctgat cccggtgatg ctttgcttgt tccaacccca     240

tattatccag ggtttgatag agatttgagg tggaggactg gtgtgaacat tgtgccaatt     300

cactgtgaaa gctcaaacaa cttccagatt actcctcaag ctttagaagc tgcatacaaa     360

gaggcagaag ccaagaacat gagagtgaga ggggtactat tcacaaatcc atcaaaccca     420

cttggtgcaa caatccaaag gacagtcctt gaagag                               456

<210>  160
<211>  641
<212>  DNA
<213>  pear ACS6

<400>  160
ctgctcaaac cttctggatg atgatgaaaa gctcagcctt agattgctct gccaacgctt      60

cttaggactt ttcacttgtg cttgttcttg agctttcttt ccttgcccta caaatgccct     120

aatccttttg agtgcaactt ccactgtgtc atcatccatg ttggcgaagc aaaccctaaa     180

ccaaccaggc tcgacacatt taaatgaaga gcccggagaa acgttgagct tcacttcatt     240

aacgattaca cgccacaaca ccatttcgcc atcaaacgtt tgatctttca atagcctcct     300

taagtccatc caacagaaaa ggccggcatt gctcttcaag cagttgattc ccacttcctc     360

aagcccatca gtgaagaccc cgtgcctctt tgcgagcctt tttgcgcttg tggtgaggaa     420

cttctcgaca aaatcttcgt ccgaaagcat ggacgcaagc atgtgctgag tttgggatga     480

gaccagccca aaacttgaca tttttcggcc aatgttcacc acgtcatcgt tgtaggagta     540

aacgatgccg actctcaatc ccgggaaccc catgtccttg gacaaactga agacaatgtg     600

gattaggttg gggttgcaat tcatgttttg tataacctcg g                         641

<210>  161
<211>  465
<212>  DNA
<213>  unknown

<220>
<223>  pear ACS5-4

<400>  161
tatccaggat ttgatcgaga tttggggtgg cgaacgggag tgcaactgat accagttgcc      60

tgtgacagct ccaacaattt caaagtcacc agagcagctt tggaagctgc ctatgagaaa 120

gctcagaagg caaacatcag agtaaagggc ttgctcatta ccaacccctc aaatcccttg 180

ggcactgtcc ttgacagaga caccctcaga agtctagtga cattcatcaa cgaaaagaaa 240

atccacctag tgtgcgatga aatctacgct gccaccgtgt tcagccagcc aagtttcata 300

agcatagccg agatcataga ggaagacatt gaatgcaacc gcaaccttat tcacattgtg 360

tacagtcttt ctaaggacat ggggttccct ggcttcagag ttggcattgt atattcctac 420

aatgatgatg tcgttaattg tgcacgaaag atgtcgagtt ttgga 465

<210> 162
<211> 197
<212> DNA
<213> unknown

<220>
<223> pear GAPDH-7

<400> 162
caggttcgga attgttgagg gtctcatgac cacagtgcat tccatcactg gtgagctttg 60

actcgttgag ttttgaagtt gtaggttttt ttataacatg aattgaattt gaaactaacc 120

taagtttatg tttaccttgt tattcagcca cccaaaagac tgttgaggtc catcacagaa 180

ggactggaga ggtggac 197

<210> 163
<211> 2476
<212> DNA
<213> unknown

<220>
<223> pear beta galactosidase

<400> 163
atgggagttg gaattcaaac aatgtggagc attctgctat tgttttcctg cattttttct 60

gcagcttcgg cttctgtgag ttacgaccac aaggctataa taattaatgg gcagaaaagg 120

attttaattt ctggctccat tcactatccc agaagcactc ctgagatgtg gccggattta 180

attcagaagg ccaaagatgg aggcttggat gttatacaga cctatgtgtt ttggaatggc 240

cacgaacctt ctccgggaaa atattatttc gaggacagat atgatttggt caagttcatc 300

aagctggtgc aacaagcagg cctatttgtt aatctccgga ttggccctta tgtttgcgct 360

gaatggaact tcgggggatt cccagtttgg ctgaaatatg tccctggaat cgcttttcga 420

acggacaatg agcctttcaa ggcggcaatg caaaaattta cagagaagat tgtcagcatg 480

atgaaggcag agaagctgtt tcaaagtcaa ggaggtccta taattctctc tcagatagaa 540

aatgaatttg gacctgtgga atgggaaatt ggtgcacctg gaaaagctta caccaaatgg 600

gcagctcaga tggctgtagg tctagacact ggagttccat ggattatgtg caagcaagag 660

gatgcccccg atcccgttat tgacacttgc aatggtttct actgtgagaa tttcaagcca 720

aataaggact ataagcccaa aatgtggaca gaagtctgga ctggttggta tacagaattc 780

ggtggggcag ttcccactag acctgcagaa gatgtggcat tttcagttgc taggttcata 840

caaagcggcg gttcgttttt gaactattac atgtaccacg gaggaacgaa ttttggccga 900
acagccggag gtcccttcat ggccactagc tatgactatg acgccccctt agacgaatat 960
ggactacccc gggaaccaaa gtggggacat ttgagagatc tgcacaaagc cattaaacca 1020
tgtgagtctg ctttagtgtc cgttgatcct tcagtgacta aactcggaag taatcaagag 1080
gctcatgtat tcaaatcaga gtcggattgc gctgcattcc tcgcaaatta tgacgcaaaa 1140
tactctgtta aagtgagctt tggaggcggg cagtatgacc tgccgccatg gtccatcagc 1200
attcttccgg actgcaaaac cgaagtttac aacactgcaa aggttggttc gcaaagctcg 1260
caagttcaga tgacaccagt acatagtgga tttccttggc agtcattcat cgaagaaacc 1320
acctcttctg atgagaccga tacaacttac atggacgggt tgtatgagca aataaatatc 1380
actagggata ctacagacta cttgtggtac atgacagata tcacaatagg ttctgatgaa 1440
gcatttctaa agaacggaaa gtccccgctt cttacaatct cttcagcagg tcatgccttg 1500
aatgttttca tcaatggtca gctctcagga accgtgtatg ggtcgttgga gaatcctaaa 1560
ttatcattca gtcaaaacgt gaacctgaga tctggcatca acaagcttgc attgcttagc 1620
atttccgttg gtctgccgaa tgttggtact cactttgaga catggaacgc gggagttctt 1680
ggcccgatca cattgaaagg tctgaattca ggaacatggg acatgtcagg gtggaaatgg 1740
acatacaaga ctggtctgaa aggtgaagct ttaggcctcc atactgttac tgggagttct 1800
tctgttgaat gggtagaagg accatcgatg gctaaaaaac aaccccttac gtggcacaag 1860
gctactttta atgcaccacc aggtgatgct ccattagctt tagatatggg aagcatggga 1920
aaaggtcaga tatggataaa tggacagagc gtgggacgcc actggcctgg atacattgca 1980
cgcggcagct gtggcgattg ttcttatgcc ggaacttatg atgataagaa atgcagaact 2040
cattgcggcg agccctctca gagatggtac cacattcctc gatcgtggtt gaccccgact 2100
ggaaatcttt tggtggtgtt cgaagaatgg ggcggtgatc cgtcagggat ttcgttggtt 2160
gaaagaggta cagccctcga cgcgaagaag ctctaggttg aggctgtctg cagctaaaga 2220
tcgagcagat acgtagatta ctaaatacgt gaagtggttg tgtacataga caatctatta 2280
attgtcgaaa aaaaatatag ctccacatga tatacgaagg gttacataca aagtttgtag 2340
tcagtagatt tgcgcaagca ttttccattg taagtttgta acaacttatg gaaaagattt 2400
ccttttcctt tacaagaata aatggaaaac taatagagac tactttatcc ttgtctttct 2460
aaaaaaaaaa aaaaaa 2476

<210> 164
<211> 1764
<212> DNA
<213> unknown

<220>
<223> pear polygalacturonase 1

<400> 164
cttgaacaca ccaatcctta catttctagc tacaattcta agtttccatt ttccaacatc 60

ccatcacatt gttcaaaaat attatcagcc tcgagttagg gtttattatc cttcgtgacc 120
ctccttttag tatttggttc tttttgaaag acgatttgct aggtgtttct aggcctcgtg 180
ccatcttgta tctcaccaag agaatatagt accagccttt tacaccaaat taaatagtaa 240
aaagaaagca tcaatggctt taaaaacaca gttgttgtgg tcatttgttg ttgtttttgt 300
tgtttccttc agtacaactt catgttctgg tagtagtttc caggaggtca acgcgcttca 360
tagttacgtt gaccatgttg atgatagggt gtccggctac aattctaggg cttatccttc 420
atacatggac accattgaag gtttaaagtt catggaattg atcaggccaa gaactcagct 480
ctccagttca aggaagctca acacaatcac cggtgggata gcaacatcat cagctccggc 540
caaaaccatt agcgtcgacg attttggagc taaagggaat ggtgctgatg acacacaggc 600
atttgtgaag gcatggaagg cagcttgttc ttccagtgga gcaatagttc ttgtggtacc 660
acagaagaaa tatcttgtta ggccaattga tttctcaggc ccatgcaaat ctcaacttac 720
agtgcagatt tatggaacca tagaagcatc agaagaccga tcaatctaca aagacataga 780
ccactggctc atctttgaca atgtccaaaa cttgctagtt gttggtcctg gaaccatcaa 840
tggcagtgga aacatctggt ggaaaaactc atgcaaaaga aaacctcagc ccccttgcgg 900
tacacacgcc cccacggctg tgaccttcaa caggtgcaat aacttggtgg tgaagaatct 960
gaagatccaa gacgcacaac aaatgcatgt cagattccaa aactgcatca acgtccaagc 1020
ttcccgtctc acggtaaccg caccggagga tagccctaat acggacggaa ttcatgtgac 1080
aaatacccag aacatcacta tctcgagctc ggttatagga acaggtgatg actgtatttc 1140
tattgtaagt gggtcccaaa gagttcaagc cacagacatt acttgtgggc caggccatgg 1200
aatcagtatt ggtagcttgg gagaagacgg ctcaaaagat catgtttcag gagtgtgtgt 1260
gaatggagct aagctttcag gaacctccaa tggactccgg atcaagacgt ggcagggagg 1320
ctcaggcagt gcaaccaaca ttgttttcca gaatgtgcaa atgaacaatg tcaccaaccc 1380
catcatcatc gaccagaact actgtgacca caaaaccaaa gattgcaaac aacagaaatc 1440
ggcggttcaa gtgaaaaatg tgttatacca aaacataaga ggaacgagtg cttccggcga 1500
cgcgataacg ttgaactgca gccaaagtgt tccttgtcgg gggatcgcgc tgcaaagtgt 1560
tcgactgcag aatggaagag ctgaatgcaa caatgttcag cctgcttaca aaggagttgc 1620
ctcccctaga tgttaaaacc tagggttcat aattatgggc attgtgaaat agattatgca 1680
attcttgtac caatcagcac ataaataatt gtttgtttgt catttttatg tttaaaaaaa 1740
aaaaaaaaaa aaaaaaaaaa aaaa 1764

<210> 165
<211> 1673
<212> DNA
<213> unknown

<220>
<223> pear polygalacturonase 2

<400> 165
cctttcaact actcgttcta aagtaattaa gacaagtagc ctctttattt ctcctacatc 60

tccatctcac tcctttttca aatcagaaaa tcctaaaacc agccagcaca aatggcaaac 120

cccaaaagcc tctcatatcc agcagctgca gtttttgcgt tgttgatgat ggctataagc 180

attactaatg tggatgctgc agccgtcact ttcagtgtga gcagtttagg agccaaagca 240

gatggcagta ctgactccac caaggccttc ctctctgcgt ggtccaatgc ttgtgcctcc 300

gtcaaccctg ctgtcatata tgtccccgca gggaggttct tgcttggcaa tgccgtgttc 360

tctgggccat gcaagaacaa cgccatcacc ttccgcattg ccggcactct cgtcgccccg 420

tctgattacc gggtcattgg aaatgccggt aactggcttc tctttcagca tgtcaatggg 480

gtcacgattt ccggtggagt tctcgacggt cagggcaccg gattgtggga ttgcaagtcc 540

tcgggcaaga gttgccccag cggagcaact acactgagct tttcgaactc caacaacgtt 600

gtggtgagtg gattaatatc actaaacagc caaatgttcc acattgtcgt caacggctgc 660

caaaatgtga aaatgcaagg tgtcaaggtt aacgcggccg gcaacagccc caacaccgat 720

ggcatccatg tccaaatgtc atctggagtc accattctcg actccaaaat ttcaaccggt 780

gacgactgtg tctcagttgg ccccggcact accaatttgt ggattgaaaa cgtcgcatgt 840

ggacccggcc acggaatcag cattgggagt ttagggaagg accaacaaga agccggtgta 900

caaaatgtta cagttaaaac agttacattc actggtactg aaaacggcgt cagaattaag 960

tcttggggga gacctagcac tggatttgct aggagcattc ttttccaaca tattgtgatg 1020

accaacgttc aaaatccaat cgttattgat caaaattact gccctaatga caaaggttgc 1080

cctggccaag cttctggagt taaggtcagc gatgtgacgt atcaagacat tcatggtaca 1140

tcggcgacgg aagtggcggt gaaattcgat tgtagttcca tgtatccttg caacgggatc 1200

agactgcaag atgtgaagct cacttacaat aaccaagcag ctgaagcttc ctgcatccat 1260

gcaggcggaa caactgccgg tacggttcag ccgacaagtt gtttctaact cgagttgtag 1320

ttttttccat ctactcctcc tcactcggag tctcgtagta ctagttggga taaaaaagaa 1380

gggactagtc atactataaa ctatatatat atatatatat ataagaatta aagaatattt 1440

ctagagtagt aggtctaggt ctagctctag ctctacgtag ttgatgtatt gagatgtatt 1500

ttgcttgagc ctgccgtgtt ggcagcctat tgggcttcct tagagcctgg cgctgcatca 1560

tccaaaccca cttcatggag agattctctt ttgcattggg tgctttgtat tatggaatgt 1620

tgtaacttga aagtgataaa tgcaatatga attaaaagta aaaaaaaaaa aaa 1673

<210> 166
<211> 1190
<212> DNA
<213> unknown

<220>
<223> pear actin

<400> 166
ggtgtcatgg ttggtatggg tcagaaggat gcctatgtag gggatgaggc tcaatcaaag 60

agaggtatct tgaccttgaa atatccaatt gagcacggta ttgttagcaa ttgggatgac 120

atggagaaga tctggcatca cacattctac aatgagctcc gtgtggctcc agaagagcat 180

ccagtcctcc tgacagaagc accccttaac cccaaggcca atcgtgaaaa aatgactcaa 240

attatgtttg agacattcaa caccccggct atgtacgttg caattcaggc tgtcctttcc 300

ctatatgcta gtggacgtac aactggtatt gttctcgact caggagatgg tgtgagctac 360

acagttccaa tttatgaagg gtatgccctc ccacatgcca tccttcgttt ggacctggca 420

ggtcgtgatc ttacagatgc tctcatgaaa attttgactg aacgtggtta ctctttcacc 480

acaactgctg agcgggaaat tgtgagggac atgaaggaga agttagccta tattgctctt 540

gactatgaac aagagctaga gacatcaaag accagctcgt ctgtggagaa gagttacgag 600

ctacctgatg gacaggtaat cactatcgga gctgagagat tccggtgccc tgaagtcctc 660

ttccagccat ccatgattgg aatggaagct cccggtatcc atgaaaccac atacaactct 720

atcatgaagt gtgatgttga tattaggaag gatctgtatg gaaatattgt cctcagtgga 780

ggtcccacta tgttccctgg aattcgtgac aggatgacga aagagattac agccttggct 840

ccaagtagca tgaagattaa ggtcgtagca ccacctgaga ggaaatacag agtctggatt 900

ggaggctcca ttttagcgtc cctcagcacc ttccaacaga tgtggattgc aaaggcagag 960

tacgatgagt ctggcccctc aattgttcac aggaaatgct tctaatcgcg caaacaaagc 1020

aagcggggaa caaagaatct acacaacaga ttttgtacta cagaaaggct tcttctattc 1080

aagcggttgc ggaatttctt tttgtattca ttttcgaatt tcattcatgt cgatttctct 1140

gtgtttgtta tgacgggaca tgagcatgga ggcgagcaaa ttatgattaa 1190

<210> 167
<211> 1384
<212> DNA
<213> unknown

<220>
<223> pear beta xylosidase

<400> 167
atttcgtgtg aaatgtgcag gttagtaagc aggacttgga agacacatat aacgtgccct 60

tcaaagcctg tgtggtggat gggaatgttg ctagtgttat gtgctcctac aaccaggtca 120

acggaaagcc tacttgtgct gaccctgatc tcctcaaagg cacaatccgt ggtcaatgga 180

aactcaatgg gtgagtccca ttgatttgaa ttatcacttg aattgcttat gtataatttt 240

cttaatttat atttgtagcc tctaattaat tactgattaa gcatttggtt tatgtgtcca 300

aaataggtat atcgtctcgg attgtgattc agttggtgtt tattatgata accaacatta 360

caccaaaaca ccagaagcag cagctgcata cgcaattaaa gcaggtcatt agcgatttaa 420

ccattttata tgagtttaat gttatctagt gcgtctcaaa agaggaagtt aagaaaatct 480

aagcccaatt ttgtaataag caggtttgga tttggattgt gggccattcc ttggcatcca 540

cacagaggca gccatgagga ctggccaggt aaacgagatc gacattaact atgcgttggc 600

taacacaatc acggtccaga tgagattggg aatgtttgat ggtgaaccgt caactcaacg 660

atatggaaac ttaggcctag cagatgtgtg caaaccatcc agcaatgagc tagcccttga 720

agctgccaga caaggcattg ttctgcttga aaaccgtggg aattcgctac ccctctcaac 780

catacgccac cgtactgtgg cagtaatcgg gcccaattct gatgttacag aaacaatgat 840

tggaaattat gctggtaagt ctactaaaaa tgtaaacctg tacatatata tatattttgt 900

cactctagtc caatttctta gtaaagcttt aaaatttgat ccattggata tttttgtaat 960

tgtatgtagg agttgcatgt ggttacacta cgccactaca aggaattgca aggtacacaa 1020

ggaccataca ccaagctggg tgcacagatg ttcattgcaa tggaaatcaa ctaattggtg 1080

ctgctgaggt tgcagctaga caagctgatg caaccgtctt agtaataggc cttgaccaat 1140

caattgaagc agagttcaga gaccggacta accttctctt gcccggacac caacaagagc 1200

tggtgtctag ggtggccagg gcttctaggg ggccgaccat tctggtcata atgtcaggcg 1260

gccctattga tgtgatgttc gcaaagaatg accctcgcat tggagcgatt atttgggttg 1320

ggtatccagg ccaagctgga ggaaccgcca ttgctgatgt tctattcggc actacaaacc 1380

caag 1384

<210> 168
<211> 1153
<212> DNA
<213> unknown

<220>
<223> pear expansin 2

<400> 168
ctcaatactt cctcctcctc ctctctctcc ttagtttctc tctctaaaaa cgcaaacaat 60

ggcttttact tctcactcca ccattgctct tctgttcttt gtcctcaatc tatgtcttca 120

aggtactttt ggtgactatg gaggtggatg ggagggcggc catgccacat tttatggtgg 180

tggtgatgcc tctggcacaa tgggaggtgc ttgtggatat gggaacttgt acagccaagg 240

gtacggaacc aacactgcag cactgagcac agctctcttc aacaatggct tgagctgcgg 300

gtcttgctat gagatgaaat gtggcagtga ccccaaatgg tgcctccccg gcagcatcat 360

cgtcaccgcc accaacttct gccctcccaa ctttgcgcag gccaacgaca atggtggctg 420

gtgcaaccct cctctccagc actttgattt ggctgagcct gctttcttga aaattgccca 480

atacagagct ggaattgtcc ccatctcctt cagaagggtt tcgtgtgtga agaagggagg 540

aataaggttc acaatcaacg gccactctta cttcaacttg gttttgatca cgaacgtcgg 600

aggagcagga gacgtccact ctgtgtcgat caagggttcc aagacagggt ggcaacccct 660

gtcaagaaat tggggccaga actggcagag caactcttac ctcaacggcc agagcctctc 720

cttccaggtc accaccagcg acggcagaac tctcaccgcc aacaatgttg cgccgggaaa 780

ctggcagttc ggacaaacat ttgagggcag tcaattctga gactcctccg gcggtaaaac 840

agttaaaaag tctggtgaat ggtgaatggt gaatggtgaa gggtgaattg tatatggtaa 900

tttgtacgtg gatgggaaga ggagagtttg gggggtagtt ttaggagagg caactgattg 960

ctgaggtggc taactggcac ccgctagtcc tatatatata aataataata ataatattta 1020

tttatatata gtgaggagtt tgtgagttta tagttaattt attaaaaatt tgttaatcat    1080

aagtagaggg acatgtaagt tctcgtatga aaggaacaca agtactatca tttccaaaaa    1140

aaaaaaaaaa aaa                                                       1153


<210>  169
<211>  1118
<212>  DNA
<213>  unknown

<220>
<223>  pear expansin 3

<400>  169
gccctacaca aaaactaaaa ctcctctctt tcttttccct attgaaatca aaacccacca      60

aaaagccaca aaaatggcag ctcatgcatt gtcttttgct cctatagccc tctctgttgt     120

tctctttaat ctacatctgc atggtgtatt tgctgtttat ggtagctggg aaggcgctca     180

tgccacattt tacggtggcg gtgatgcttc tggcacaatg ggaggagcat gtggttatgg     240

gaatttgtac agccaggggt atggaaccaa cactgcagct ttgagcacag cattgttcaa     300

caatggctta agctgtgggt cttgttatga aatgagatgc gacaatgacc cgagatggtg     360

ccgtcctgga tccatcattg taactgctac aaacttttgc cctcctaact ttgctcagtc     420

caacgacaat ggcggatggt gcaatcctcc tctccagcat ttcgatttgg ctgagcctgc     480

tttcttgcaa attgcccaat accgtgctgg aatcgtgccg gtttccttca gaagagtacc     540

ttgtgtgaag aaaggaggaa taagattcac catcaacggc cactcctact tcaacctggt     600

tttgatcacc aacgtggctg gggcaggaga cgtccattca gtttcaatca aggggtccag     660

aacagggtgg caacccatgt caagaaactg gggtcaaaac tggcagagca actcttacct     720

caatggccaa gccctctcct tccaagtcac caccagtgac ggtagaaccg tcacgagcta     780

caacgtcgcg cctggtaatt ggcagtttgg tcagacattc tccgggggtc aattttagag     840

atattcctct acattattgg taaaaatttg tatatctatc tgtcattttt ttcccgtaaa     900

cttttctgag tgtaaaagca aagagtagtt gtgaagtgga ggtttgctga ggtgagctaa     960

aaaaacaccc gctgggcctt tcacatttga gttttcctgg agaaatgata ttcacctcat    1020

tcaggttgta accaatttct cagttgtact tgtaacctta atgatatata tatttataaa    1080

aaacgagaaa gctttatcaa aaaaaaaaaa aaaaaaaa                            1118


<210>  170
<211>  1355
<212>  DNA
<213>  unknown

<220>
<223>  pear expansin 5

<400>  170
atttcctctc ccctgttttt tcaagcctca ttcgtttcca atccaaatga aaatggcttc      60

ttcatctggg tttttcatgg cgggtcttct tgcaatgttg gtggcgtctg cgcatgctta     120

cggtggaggc gggtgggtta atgctcgcgc cactttctac ggcggcggtg acgcttctgg     180

cacaatgggt ggagcttgtg gttatggcaa cctctacagc caggggtacg gaaccaacac 240
ggccgcgctg agcactgctc tgttcaacaa tgggctgggg tgcggatctt gctatgagat 300
taggtgtgtg aacgacccca aatggtgtct gccgggctcc attgtggtca ccgccaccaa 360
tttctgcccg ccgaacaacg ccctccccaa caatgccggc ggctggtgca accctcccca 420
gcaccacttc gacctctctc agcctgtctt ccagcacatt gcccaataca aagccggagt 480
tgtgcctgtg tcctacagaa gggtaccctg catgagaagg ggaggcatca gattcacaat 540
caacggacac tcctacttca acctggtcct tatcacaaac gttggtggtg ccggtgatgt 600
gcactctgtt tccgtcaaag ggtccagaac tggctggcaa gcaatgtccc gaaactgggg 660
gcaaaactgg caatccaaca gctacctcaa cggccaagct ctctctttca aggtcacaac 720
aagtgacggc aggactgttg tgtcctacaa tgctgcccct gctggatggt cctttgggca 780
gacctacagc ggtgcccaac tccgctaggg cacccccacg ccacctccta atttaccctc 840
catatttagc catttcctag gtcattaaat atatactgaa gtataattaa gattagtgtg 900
actcttaagt atactaggat attagttagt attctgagtc actgagtcaa ggtctttggc 960
ctctgcggtc aaaagtgagt agggcttatt ttataagggc tctcccatca agttttaatt 1020
tttgaattat atgtgtctta ggtgtagggt accctactat ctttgggacc ctaactcctt 1080
gggtattgtt tctttttact tctgcagtat ttgtaaaatc agaagttggc agaggtggtg 1140
gactttgacc acccgccatt agtgttttga aatattacta gcatcttggg cttgttttgg 1200
aagggcatta ttgtaattca gctgaggctg atggttaatt cagtgttgtc cgtggttgtt 1260
atatttgaat gttgttgaag gactaaatgt aaattttcct ttctctacta agtaatggat 1320
tttctaattt actatttaaa aaaaaaaaaa aaaaa 1355

<210> 171
<211> 1272
<212> DNA
<213> unknown

<220>
<223> pear expansin 6

<400> 171
catcactctc tctcctctct ctgctttctc actccccttt ctctctctcc ggcaatggcc 60
tcccttcgcg tgctctacat tgctttcatg ctctcactct tcatggcggc caacgctaga 120
attccaggag tttacaccgg tggctcatgg gagggcgccc acgccacctt ctacggtggc 180
aacgacgcct ctggaaccat gggtggcgct tgcgggtacg gaaacctcta cagccaaggc 240
tacggcgtga acacggcggc actgagcact gctctgttca acaatggcct tagctgcggc 300
gcctgcttcg agattaagtg cggcgacgac cccaggtggt gccacccagg caacccctcc 360
atcttagtca ccgccaccaa cttctgccct cctaacttcg ctcagcccag cgacgacggc 420
gggtggtgca accctccccg cacccacttc gacctcgcca tgcccatgtt cctcaagatc 480
gccgagtaca aggccggcat cgtccccgtc tcttaccgcc gagttccgtg cagaaagcaa 540

ggcggagtga gattcacaat taacggtttc cgttacttca acctggttct gatcaccaac      600

gtcgcgggcg caggggatat cgtgagggtg agcgtaaaag gcgggaacac tgggtggatg      660

ccgatgagtc gcaactgggg acaaaactgg caatccaacg cagacctggt gggccagacc      720

ctgtcgtttc gagtcacggg cagtgacagg cgcacatcca cctcccacaa cgtggcaccc      780

gctgattggc agttcggaca aactttcacc ggcaagaatt tccgggtcta aaattaagaa      840

gggaaaaaaa agtttatcca ctatctttaa ttttcctttt gggtttttaa cttttttttt      900

aaattatcaa agtttaattt cccgccatct gattttcctt aattttcccg ggaaaatttg      960

gaagcggtgg gagtataaaa gtaaagtgtt agatgatatg gggttaaaag ttaaaattgg     1020

gtggtaagat aggtcgaaaa gcgacttctt ttgcaagtgt ggtgtgcggc aactttttac     1080

ttttggtgct tttttttttt aggtttgagt gggaggctgg taaaaattta ggtgatccgg     1140

ccaaatagtg cgtgtaaaag gagtcgaagc ggctgcaaag aaccaacatg cagcctgcag     1200

ctctacccat atcttttcta gaattttata tgatatatat ttcagttagt ataaaaaaaa     1260

aaaaaaaaaa aa                                                         1272

<210>  172
<211>  560
<212>  DNA
<213>  unknown

<220>
<223>  rose OProseR1663 (Unknown function)

<220>
<221>  misc_feature
<222>  (2)..(2)
<223>  n is a, c, g, or t

<220>
<221>  misc_feature
<222>  (22)..(22)
<223>  n is a, c, g, or t

<220>
<221>  misc_feature
<222>  (412)..(412)
<223>  n is a, c, g, or t

<400>  172
angattaccc cattctattt angggtgaca ctatagaata ctcaagctat gcatccaacg       60

cgttgggagc tctcccatat ggtcgacctg caggcggccg cgaattcact agtgatttcg      120

agcggccgcc cgggcaggtg gcagcggtgc tgctggtggt tgcttgcatt aatgtagagg      180

tctccgatgc gcagaccatt tgcaatgtgt ctgtgaataa cttgatgtcc tgtaagccgg      240

ccgtaacaaa acctaaccct tccaggccga ccaagacctg ctgctcggtg ctgtcgcacg      300

ccgacttgaa gtgcctctgc tcctacagga actcgaacct gttgccttct ctggggattg      360

accctaacct tgccatgcag ctccctgcca agtgcaagct tcctcaccct gnccaattgc      420

tagagaggcc cttaatctac agctacactg tcagatgtgg ctagctagct gaagttaaat      480

aaaagctcta tatgtatgta tgattaagat gggattaatc ttggagtgaa atgcttaatt      540

aatttccaaa aaaaaaaaaa                                                560

<210>  173
<211>  810
<212>  DNA
<213>  unknown

<220>
<223>  rose OProseR0948  - HHG4 nucleoid DNA binding proteint cnd41


<220>
<221>  misc_feature
<222>  (2)..(2)
<223>  n is a, c, g, or t

<220>
<221>  misc_feature
<222>  (4)..(4)
<223>  n is a, c, g, or t

<220>
<221>  misc_feature
<222>  (6)..(6)
<223>  n is a, c, g, or t

<220>
<221>  misc_feature
<222>  (11)..(11)
<223>  n is a, c, g, or t

<220>
<221>  misc_feature
<222>  (20)..(20)
<223>  n is a, c, g, or t

<220>
<221>  misc_feature
<222>  (23)..(23)
<223>  n is a, c, g, or t

<220>
<221>  misc_feature
<222>  (25)..(25)
<223>  n is a, c, g, or t

<220>
<221>  misc_feature
<222>  (32)..(33)
<223>  n is a, c, g, or t

<220>
<221>  misc_feature
<222>  (37)..(37)
<223>  n is a, c, g, or t

<220>
<221>  misc_feature
<222>  (77)..(77)
<223>  n is a, c, g, or t

<220>
<221>  misc_feature
<222>  (620)..(620)
<223>  n is a, c, g, or t

<400>  173

antnanttac ngcgaattcn ctngngacct gnncgcntct acataaaggg aacaaaatgc 60

tggagctcca ccgcggntgg cggccgctct agaactagtg gatcccccgg gctgcaggaa 120

ttcggcacga ggcgccagtc tgctctctac tcacatcagc cacaggtaat acacctgggt 180

tgctcaagcg gcacatcaac ttgcatatat gccatacaat acggagataa ctccttctcc 240

gtgggatact ttagcaaaga taggttgaca ctaacgtcaa ctgactattt cgacgggttt 300

ctcttcggtt gtggccaaaa caatcaaggc ctttttggcg ggtctgcagg gttattgggc 360

ttgggccgca acaaaatctc cctcgatcga acagagcgcg caaaagtaca gccgcttctt 420

ctcctactgc ctaccctcaa cttccagcgc cactggttac ctcagcttcg gaaaaggcgg 480

cggaccttcc aacgccgtca agttcacgcc gctatccact gtatctgaag gtgggtcctt 540

ttacggcctg gatgtcgttg gaatcaatgt cggcggacgt cgggtatcga ttcccgcttc 600

agttttttcg tcctcagggn acgatcatcg actcagggac ggtgataacg cggcttccgg 660

caacagcgta cagtgctttg agggatgcgt ttaagcaagg aatgaaaagc tatccacaag 720

ctgaggagct ttcgatattg gacacgtgct acgacctgag cgggagcagc acagtgtcgt 780

atccccaaaa tagcgttcgc tttcagcggt 810

<210> 174
<211> 478
<212> DNA
<213> unknown

<220>
<223> rose OPRoseR0049 (actin)

<400> 174
tcggcacgag gggaaatcac tgctcttgct ccaagcagca tgaagatcaa ggttgtggct 60

ccaccagaga ggaagtacag tgtctggatc ggagggtcca ttcttgcttc cctcagtacc 120

ttccaacaga tgtggatttc taagggagag tatgatgagt ctggtccatc cattgtccac 180

aggaagtgct tctgagttct ataaatgttt tggtggtgag ttattttttc ttcaatttag 240

ttggattttc gtgtcaagtg tcatgtggtt gggatgtttg tagtggagag tgattgggat 300

cttttttttt ccgtccttaa ttcccttttt gttctatatt tgacggttct ttttctttct 360

tttttcttgg gaacattaat attactagct ttattgtatc tgaatgatag tgtcagtttg 420

tggtcataaa ctgctatgga aattatattt aaataaaaaa aaaaaaaaaa aaaaaaac 478

## Description

### FIELD OF THE INVENTION

The present invention relates to the field of quality testing of fresh plant-based and mushroom based products, such as food or feed products and ornamental products. Provided are methods for quality testing and quality prediction and diagnostic kits for quality screening and selection of high quality products. In particular, relative or absolute mRNA expression levels of defined sets of gene transcripts are determined, whereby a specific stage or category of a quality trait is determined and an advice for subsequent distribution or processing chains is given. Thus this invention describes a new support tool for stakeholders in agro-production, agro-distribution and agro-processing.

### BACKGROUND OF THE INVENTION

Fresh plant and mushroom products that are generated in agricultural production chains differ in intrinsic quality (phenotype) in a batch-dependent way. Partly this is due to differences in growth conditions, but even after harvest the products are actively metabolizing and responding to environmental triggers, such as temperature, light, humidity, etc.

The batch quality has a strong influence on the type of application that the product can have in downstream distribution and/or processing chains. Batch quality is the main parameter in decisions concerning (international) market choice. In addition high quality batches can be assigned A-status, which will increase the added value that can be obtained. At the moment these batch-to-batch differences in quality are only marginally determined and consequently hardly exploited. Present quality tests usually involve measurements of a physiological parameter such as color, firmness or pH that is always secondary in nature and gives no information on the nature or status of the biological process that is causing the effect.

Another problem associated with present quality tests is that no prediction of future quality can be made, because the tests only allow the present status to be determined.

The present inventors found that genomics technology offers a complete new spectrum of possibilities to assess the quality of fresh agricultural products during all stages (from production, to harvest, to processor or consumer) of the diverse production chains in which they are used. At present in plant production, genomics technology is only used for generating scientific knowledge and for breeding purposes. However, the present inventors found that the high information content of genomics data, makes it eminently suited for use in quality diagnostics. They have shown that genomics-based agro-diagnostics even based diagnostics even allows prediction of future quality and can thus be used as support tool, for decisions concerning applications, treatments or destinations for specific batches.

The herein provided genomics based diagnostic methods and kits facilitate the implementation of precisely controlled agricultural production and distribution chains, and allow for batch differentiation at auctions, before storage and for processing industry. Robust quality assays are provided herein, which were developed based on a combination of expertise in molecular biology, post-harvest physiology, chain knowledge and quality dynamics modeling.

### GENERAL DEFINITIONS

The term "gene" means a DNA fragment comprising a region (transcribed region), which is transcribed into an RNA molecule (e.g. an mRNA, or RNA transcript) in a cell, operably linked to suitable regulatory regions (e.g. a promoter). A gene may thus comprise several operably linked fragments, such as a promoter, a 5' leader sequence, a coding region and a 3'nontranslated sequence comprising a polyadenylation site.
"Indicator genes" refers herein to genes whose expression level is indicative of a certain quality stage of a fresh agricultural product.
"Expression of a gene" refers to the process wherein a DNA region which is operably linked to appropriate regulatory regions, particularly a promoter, is transcribed into an RNA molecule.
"Upregulation" of gene expression refers to an amount of mRNA transcript levels of at least about 2 times the level of the reference sample, preferably at least about 3x, 4x, 5x, 10x, 20x, 30x or more.
"Downregulation" of gene expression refers to an amount of mRNA transcript levels of at least about 2 times lower than the level of the reference sample, preferably at least about 3x, 4x, 5x, 10x, 15x lower.
"Constant" refers to an essentially equivalent mRNA transcript level as in the reference sample. Generally, housekeeping genes (such as glyceraldehydes-3-phosphate dehydrogenase, albumin, actins, tubulins, 18S or 28S rRNA) have a constant transcript level.
"Relative" mRNA expression levels refer to the change in expression level of one or more indicator genes relative to that in another sample, preferably compared after "normalization" of the expression levels using e.g. housekeeping genes. The fold change (upregulation or downregulation) can be measured using for example quantitative real-time PCR. The fold change can be calculated by determining the ratio of an indicator mRNA in one sample relative to the other. Mathematical methods such as the 2(-Delta Delta C(T)) method (Livak and Schmittgen, Method 2001, 25: 402-408) or other mathematical methods, such as described in Pfaffl (2001, Nucleic Acid Research 29: 2002-2007) or Peirson et al. (2003, Nucleic Acid Research 31: 2-7) may be used.
"Absolute" mRNA expression levels refer to the absolute quantity of mRNA in a sample, which requires an internal or external calibration curve and is generally more time consuming to establish than relative quantification approaches.
The term "training sample" or " training batch" refers herein to a reference batch of the same type of plant material (e.g. same tissue type and cultivar), having a predetermined quality status (i.e. the expression profile of indicator genes of the training batch is correlated with the quality stage or predicted/future quality stage). The expression profile of the indicator genes in a "test batch" can then be analyzed and thereby correlated with the quality stage (or predicted/future quality stage) of one of the training batches.
The term "substantially identical", "substantial identity" or "essentially similar" or "essential similarity" means that two peptide or two nucleotide sequences, when optimally aligned, such as by the programs GAP or BESTFIT using default parameters, share at least a certain percent sequence identity. GAP uses the Needleman and Wunsch global alignment algorithm to align two sequences over their entire length, maximizing the number of matches and minimizes the number of gaps. Generally, the GAP default parameters are used, with a gap creation penalty = 50 (nucleotides) / 8 (proteins) and gap extension penalty = 3 (nucleotides) / 2 (proteins). For nucleotides the default scoring matrix used is nwsgapdna and for proteins the default scoring matrix is Blosum62 (Henikoff & Henikoff, 1992, PNAS 89, 915-919). It is clear that when RNA sequences are said to be essentially similar or have a certain degree of sequence identity with DNA sequences, thymine (T) in the DNA sequence is considered equal to uracil (U) in the RNA sequence. Sequence alignments and scores for percentage sequence identity may be determined using computer programs, such as the GCG Wisconsin Package, Version 10.3, available from Accelrys Inc., 9685 Scranton Road, San Diego, CA 92121-3752 USA. or using in EmbossWIN (version 2.10.0) the program "needle", using the same GAP parameters as described above. For comparing sequence identity between sequences of dissimilar lengths, it is preferred that local alignment algorithms are used, such as the Smith Waterman algorithm (Smith TF, Waterman MS (1981) J. Mol. Biol 147(1);195-7), used e.g. in the EmbossWIN program "water". Default parameters are gap opening penalty 10.0 and gap extension penalty 0.5, using Blosum62 for proteins and DNAFULL matrices for nucleic acids.
"Stringent hybridization conditions" can also be used to identify nucleotide sequences, which are substantially identical to a given nucleotide sequence. Stringent conditions are sequence dependent and will be different in different circumstances. Generally, stringent conditions are selected to be about 5°C lower than the thermal melting point (Tm) for the specific sequences at a defined ionic strength and pH. The Tm is the temperature (under defined ionic strength and pH) at which 50% of the target sequence hybridizes to a perfectly matched probe. Typically stringent conditions will be chosen in which the salt concentration is about 0.02 molar at pH 7 and the temperature is at least 60°C. Lowering the salt concentration and/or increasing the temperature increases stringency. Stringent conditions for RNA-DNA hybridizations (Northern blots using a probe of e.g. 100nt) are for example those which include at least one wash in 0.2X SSC at 63°C for 20min, or equivalent conditions. Stringent conditions for DNA-DNA hybridization (Southern blots using a probe of e.g. 100nt) are for example those which include at least one wash (usually 2) in 0.2X SSC at a temperature of at least 50°C, usually about 55°C, for 20 min, or equivalent conditions.
The term "comprising" is to be interpreted as specifying the presence of the stated parts, steps or components, but does not exclude the presence of one or more additional parts, steps or components. A nucleic acid sequence comprising region X, may thus comprise additional regions, i.e. region X may be embedded in a larger nucleic acid region.
In addition, reference to an element by the indefinite article "a" or "an" does not exclude the possibility that more than one of the element is present, unless the context clearly requires that there be one and only one of the elements. The indefinite article "a" or "an" thus usually means "at least one".
The term "plant" refers to any organism of which the cells, or some of the cells contain chloroplasts. It may refer to the whole plant (e.g. the whole seedling) or to parts of a plant, such as cells, tissue or organs (e.g. pollen, seeds, gametes, roots, leaves, flowers, flower buds, anthers, fruit, etc.) obtainable from the plant, as well as derivatives of any of these and progeny derived from such a plant by selfing or crossing. "Plant cell(s)" include protoplasts, gametes, suspension cultures, microspores, pollen grains, etc., either in isolation or within a tissue, organ or organism.
"Mushroom" or "fungus" refers to members of the kingdom Fungi, including parts thereof, such as hyphae, fruiting bodies, spores, etc., as well as progeny or derivatives thereof. Generally throughout the description, reference to plants above will equally apply to mushrooms and it is understood that, even if mushrooms are not mentioned, they are encompassed in the embodiments.
The term "batch" refers to a collection of harvested plant or mushroom products that share a considerable part of their history in the production and or distribution chain. For example the term "batch" is used to describe a group of plant or mushroom products grown in the same greenhouse in the same period and harvested at the same time.
The term "quality trait" refers to a specific physiological characteristic of a plant or mushroom product that is important for determining the economic value. For example the term may be used to refer to colour or taste or firmness or tenability of a product. The term "quality stage" or "quality trait stage" refers to a predefined moment in the development of a quality trait, described by a specific set of physiological of morphological characteristics. For example, the term may be used to refer to a specific level of firmness of fruits, defined by the level of resistance to penetration by a metal rod. Or it may refer to a specific level of ripeness of tomato, determined by the color of the fruit as compared to a standard color-card. "Predicted or future quality (trait) stage" is the quality stage which is predicted to develop in the batch after time, as determined by the expression profile of a set of indicator genes. This is encompassed by the broader term "quality stage".
The term "fresh" refers to plant products that have not (yet) been processed, or only minimally processed (e.g. cut or sliced and/or packaged) after harvest and which are still actively metabolizing and responsive to the environment.
"PCR primers" include both degenerate primers and non-degenerate primers (i.e. of identical nucleic acid sequence as the target sequence to which they hybridize). "Oligonucleotides" refer to nucleic acid fragments suitable for use as PCR primers or hybridization probes, e.g. coupled to a carrier in a nucleic acid microarray.
"DNA Microarray" or "DNA chip" is a series of known DNA sequences (oligonucleotides or oligonucleotide probes) attached in a regular pattern on a solid surface, such as a glass slide, and to which a composition consisting of or comprising target sequences are hybridized for identification and/or quantification.

### DETAILED DESCRIPTION

A genomics-based method, exemplified by six specific examples, is provided that can be used for measuring (and predicting) specific quality characteristics (or quality stage) of a fresh product. The tests are based on the combined expression profiles of a carefully selected set of indicator genes. In living organisms, each developmental step and every interaction with the environment is orchestrated by DNA encoded genes. The history and actual condition of a plant, animal or microorganism is accurately reflected in the activity profile of its genes. The indicators were selected by combining gene expression analysis (using microarrays) and thorough physiological analyses with knowledge of distribution chain logistics. The information was used to select those genes that are most strongly correlated to the trait(s) of interest. The selected set of indicator genes was translated into a reliable and robust assay for use in practice.

Quality assays and kits are provided for 1. the determination / prediction of fungal incidence or susceptibility to the development of fungal disease symptoms in *Rosaideae,* especially of *Botrytis* in cut roses (genus *Rosa*), 2. the determination of cold tolerance in tree seedlings and the stage at which tree seedlings are cold-hardened sufficiently to be lifted, 3 the determination of the ripening stage of fruit, especially pears after harvest, 4. the determination of sensory stages or the deterioration (sensory decay) of fruit, especially apples after harvest, 5. the determination of quality stage (degree of browning) and especially prediction of brown discoloration in edible mushrooms, especially harvested basidiomycetes such as *Agaricus* species and 6. the determination and prediction of firmness development (in particular of loss of firmness during post-harvest storage) in Solanaceous fruits, especially tomatoes (genus Solanum).

In its broadest term, the method for determining the quality stage and/or predicting quality traits according to the invention comprises:
(a) providing a nucleic acid sample (comprising RNA or corresponding cDNA) of a plant or plant part, or a plurality of plants (batch), or of an edible mushroom (or batch),
(b) analysing the sample by determining the level of a set of indicator mRNA transcripts in the sample, which are indicative of the present stage and/or of the future status of a quality trait of the plant or plant part, or the batch of plants, or mushroom(s) or mushroom batch, and optionally
(c) identifying and selecting the plant (or mushroom) or the batch, which comprises a certain level of the indicator mRNA transcripts (i.e. a certain relative or absolute amount of mRNA or corresponding cDNA) for further use.

Optionally a step (b') is inserted between steps (b) and (c), or replaces step (c), whereby said step comprises:
(b') feeding the result obtained in (b) into a quality determination model or quality dynamics model that builds on a database of previously analysed samples and that positions the sample at hand in the quality spectrum of interest and translating the outcome of the model into a practical advice for stakeholders in agro-production and agro-distribution, such as step (c) above.

In one embodiment steps (a) and (b) are repeated at regular time intervals, until the mRNA transcript levels are such that the plants, plant parts or batch is at a certain quality stage for step (b') or (c).

For simplicity herein below reference to plants and plant parts or plant batches is understood to apply equally to edible mushrooms or mushroom parts or mushroom batches. In certain embodiments the plants or plant parts (or batches) are harvested parts, such as severed plant parts (e.g. cut flowers), harvested fruit (e.g. apples, pears, strawberries, etc.). The quality stage of the harvested product is determined once or more times. A harvested product may also refer to harvested plants or plant parts which have been further processed, such as sliced, diced, etc. and packaged into batches, but which are preferably still regarded as "fresh" (as defined above). In other embodiments, the quality stage is determined for the living, developing plant, such as plant seedlings.

In step (a), the plant or mushroom tissue, from which the nucleic acid sample is to be obtained, is collected and optionally processed and/or stored. Many methods for extracting nucleic acids from plant or mushroom material are known in the art. In a preferred embodiment, a tissue homogenate is made and some of the homogenate is placed onto FTA cards (Whatman FTA® Technology). The FTA cards capture the nucleic acids present in the homogenate and contain agents which protect the nucleic acids from degradation and damage. The homogenate on the FTA cards is allowed to dry (e.g. at least one hour, preferably at room temperature and preferably without assisting the drying period by heating or other means). The FTA cards comprising the tissue homogenate can then be stored (preferably in a desiccated environment) until the nucleic acids are captured therefrom for downstream processing.

In one embodiment steps (a) and (b) of the method are carried out at regular time intervals (e.g. once a week, once every two weeks, once a month, etc), so that a change in the level of mRNA transcripts (or the corresponding cDNAs) of the indicator genes can be determined, relative to the earlier level of transcripts. The relative change in mRNA transcript abundance (up-regulation down-regulation, no change in mRNA levels) is then used to select plants, plant parts or batches in step (c) and/or the change in transcript abundance is entered into the model in step (b'). Alternatively, the relative or absolute mRNA level of the indicator genes is compared to the level of the indicator RNAs in a suitable control sample. Such a control may for example consist of one or more nucleic acid samples of known quality stages (e.g. training batches or batches obtained at earlier time points) so that the indicator mRNA abundance is compared relative to that of the control sample. It is understood that the control expression data does not need to be produced at the same time as the sample data, but can have been produced previously, such as one or more training batches.

The nucleic acid sample of step (a) may be provided for several individual plants (or parts), or preferably for batches of several plants (or parts). cDNA samples of batches may be made by either first pooling tissue from several individuals and then obtaining the nucleic acid from the pooled tissue sample or by directly pooling the nucleic acid obtained from individual plants. Preferably, the nucleic acid sample in step (a) comprises or consists of total RNA, total mRNA or total cDNA. For example, the total mRNA is isolated (e.g. using polyA⁺ selection) and is used to make corresponding cDNA by reverse transcription.

The mRNA level (or corresponding cDNA level) of a set of defined indicator genes can be detected and quantified using various methods generally known in the art, such as (but not limited to) quantitative PCR methods, preferably quantitative RT-PCR, or nucleic acid hybridization based methods (for example microarray hybridization). Quantitative PCR (qPCR) may be carried out by conventional techniques and equipment, well known to the skilled person, described for instance in S.A. Bustin (Ed.), et al., A-Z of Quantitative PCR, IUL Biotechnology series, no 5, 2005. Preferably, labeled primers or oligonucleotides are used to quantify the amount of reaction product. Other techniques capable of quantifying relative and absolute amounts of mRNA in a sample, such as NASBA (Nucleic Acid Sequence Based Amplification), may also be suitably applied. A convenient system for quantification is the immunolabeling of the primers, followed by an immuno-lateral flow system (NALFIA) on a pre-made strip (references: Kozwich et al., 2000, Applied and Environmental Microbiology 66, 2711-2717; Koets et al., 2003, In: Proceedings EURO FOOD CHEM XII - Strategies for Safe Food, 24-26 September 2003, Brugge, Belgium, pages 121-124; and van Amerongen et al., 2005 In: Rapid methods for biological and chemical contaminants in food and feed. Eds. A. van Amerongen, D. Barug and M. Lauwaars, Wageningen Academic Publishers, Wageningen, The Netherlands, ISBN: 9076998531, pages 105-126).

As a positive control for the RNA isolation, reverse transcriptase reaction, amplification reaction and detection step, amplification and detection of a constitutively expressed housekeeping gene may be included in the assay, such as ribosomal (18S or 25S) rRNA's, actin, tubulin or GAPDH. Primers may be labeled with direct labels such as FITC (fluorescein), Texas Red, Rhodamine and others or with tags such as biotin, lexA or digoxigenin which may be visualized by a secondary reaction with a labeled streptavidin molecule (for instance with carbon or a fluorescent label) or a labeled antibody (labeled with fluorescent molecules, enzymes, carbon, heavy metals, radioactive isotopes or with any other label).

In another embodiment, comparative hybridization is performed on mRNA or cDNA populations obtained from a plant or sample thereof, to a set of indicator gene sequences, which may optionally be tagged or labeled for detection purposes, or may be attached to a solid carrier such as a DNA array or microarray. Suitable methods for microarray detection and quantification are well described in the art and may for instance be found in: Applications of DNA Microarrays in Biology. R.B. Stoughton (2005) Annu.Rev.Biochem. 74:53-82, or in David Bowtell and Joseph Sambrook, DNA Microarrays: A Molecular Cloning Manual, Cold Spring Harbor Laboratory Press, 2003 ISBN 0-870969-625-7. To construct a DNA microarray, nucleic acid molecules (e.g. single stranded oligonucleotides according to the invention) are attached to a solid support at known locations or "addresses". The arrayed nucleic acid molecules are complementary to the indicator nucleotide sequences according to the invention, and the location of each nucleic acid on the chip is known. Such DNA chips or microarrays, have been generally described in the art, for example, in US 5,143,854, US 5,445,934, US 5,744,305, US 5,677,195, US 6,040,193, US 5,424,186, US 6,329,143, and US 6,309,831 and Fodor et al. (1991) Science 251: 767-77, each incorporated by reference. See also technology providers, such as Affymetrix Inc. (www.affymetrix.com). These arrays may, for example, be produced using mechanical synthesis methods or light-directed synthesis methods that incorporate a combination of photolithographic methods and solid phase synthesis methods. Also methods for generating labeled polynucleotides and for hybridizing them to DNA microarrays are well known in the art. See, for example, US 2002/0144307 and Ausubel et al., eds. (1994) Current Protocols in Molecular Biology, Current Protocols (Greene Publishing Associates, Inc., and John Wiley & Sons, Inc., New York; 1994 Supplement).

Herein, for each quality trait, a specific "set of indicator genes" is provided, whose expression level correlates with and is indicative of the present and/or future quality stage of the plant, plant part or batch. A "set of indicator genes" refers, therefore, to a defined number of genes whose expression level (mRNA abundance, or corresponding cDNA abundance) is being determined. A distinction can be made between the "main set", which refers to a larger number of defined genes, and "sub-sets", which refer to smaller numbers selected from the main set. Thus, either the main set of indicator transcripts may be detected or, preferably, a subset is detected. For example, the upregulation of one indicator mRNA transcript and the down regulation of another indicator mRNA transcript may already be sufficient to determine the quality of the plant or plant part (or batch). Thus, although in some examples herein below up to 30 indicator genes (and indicator transcripts) are provided, any subset thereof, such as 20, 10, 5, 4, 3, or 2 may already be sufficient. It is clear, that the robustness of the method is inversely related to the number of indicator transcripts being detected.

When referring to "indicator genes", not only the specific nucleic acid sequences (mRNA or cDNA) of those genes (as depicted in the Sequence Listing) are referred to, but also "variants" of these sequences and fragments of the indicator genes or of the variants. A "variant" refers herein to a nucleic acid sequence which are "essentially identical" to the indicator genes provided, i.e. they comprises at least about 70, 75, 80, 85, 90, 95 98, 99% or more, nucleic acid sequence identity to the sequences provided herein (determined using pairwise alignment with the Needleman and Wunsch algorithm, as defined).

As mentioned, also fragments (e.g. oligonucleotides) of indicator genes (or of the variants of indicator genes) are encompassed and may be detected, or may be used for detection of the indicator transcript in a sample or batch. Fragments comprise any contiguous stretch of at least 8, 10, 12, 14, 15, 18, 20, 22, 25, 30, 40, 50, 100, 200, 500, 800, 900, 1000 or more nucleotides of an indicator gene or a variant thereof. Such fragments may be used as PCR primers or probes for detecting indicator genes by selectively hybridizing to the indicator mRNA or cDNA.

Variants may be isolated from natural sources, using for example stringent hybridization conditions or can be easily generated using methods known in the art, such as but not limited to nucleotide substitutions or deletions, *de novo* chemical synthesis of nucleic acid molecules or mutagenesis- or gene-shuffling techniques, etc.

Also provided are kits for carrying out the methods and nucleic acid carriers comprising sets of indicator genes, and/or variants and/or fragments of indicator genes, e.g. oligonucleotides of the indicator genes or of variants thereof. The kits may optionally also contain material and instructions for tissue/batch sampling, such as FTA cards and instructions for use or FTA cards onto which tissue homogenates have already been applied. Obviously, other material for sampling include other carriers for sample material (e.g. containers such as Eppendorf tubes or microtitre plates) and reagents, such as solvents, buffers, etc.

Nucleic acid carriers may for example be arrays and microarrays or DNA chips, comprising nucleotides on a glass, plastics, nitrocellulose or nylon sheets, silicon or any other solid surface, which are well known in the art and for instance described in Bowtell and Sambrook, 2003 (*supra*) and in Ausubel et al., Current protocols in Molecular Biology, Wiley Interscience, 2004. A carrier according to the current invention comprises at least two (or more, such as at least 3, 4, 5, 10, 15, 20, 25, 30, or more) (oligo-)nucleotide probes capable of selectively hybridizing with two indicator genes (mRNA or cDNA) present in a sample.

A kit for determining and/or predicting the quality stage of a sample comprises elements for use in the methods of the invention. Such a kit may comprise a carrier to receive therein one or more containers, such as tubes or vials. The kit may further comprise unlabeled or labeled (oligo)nucleotide sequences of the invention, e.g. to be used as primers, probes, which may be contained in one or more of the containers, or present on a carrier. The (oligo)nucleotides may be present in lyophilized form, or in an appropriate buffer. One or more enzymes or reagents for use in isolation of nucleic acids, purification, restriction, ligation and/or amplification reactions may be contained in one or more of the containers. The enzymes or reagents may be present alone or in admixture, and in lyophilized form or in appropriate buffers. The kit may also contain any other component necessary for carrying out the present invention, such as manuals, buffers, enzymes (such as preferably reverse transcriptase and a thermostable polymerase), pipettes, plates, nucleic acids (preferably labeled probes), nucleoside triphosphates, filter paper, gel materials, transfer materials, electrophoresis materials and visualization materials (preferably dyes, labeled antibodies or -enzymes) autoradiography supplies. Such other components for the kits of the invention are known *per se.* The kit may also comprise tissue samples and/or nucleic acid samples, such as suitable control samples.

### Assays and kits for the determination / prediction of Botrytis incidence or susceptibility to Botrytis infection in Rosoideae

Harvested flowers can suffer from *Botrytis* disease (grey mold, especially *Botrytis cinerea*) during its vase-life / post-harvest. Batches of roses can vary enormously in the percentage of flowers that show disease symptoms during vase-life. Using a standard visual screening method, it has been shown that there is no clear correlation between observed disease spots directly after harvest and the percentage infected flowers after a post-harvest chain simulation. It is a major problem for roses grown in African countries because transportation is expensive. Part of the biological variation in susceptibility is genetic, some cultivars are less susceptible to the fungus than others. However, non-genetic or phenotypic variation is of equal or even larger importance. Within-cultivar variation, e.g. caused by different growing or post-harvest conditions, is the main reason for quality miss-estimations.

So far no tests were available for determining the likelihood that roses will develop *Botrytis* symptoms after harvest. A visually screen does not give a conclusive prediction of *Botrytis* incidence. Detection of the pathogen itself is not sufficient because almost all roses contain spores of the fungus, but whether or not this will result in serious infection/disease symptoms is determined by several parameters, such as humidity, spore dose, temperature and, most of all, susceptibility (or sensitivity) of the plant to *Botrytis* infection and development caused by genetic differences (cultivar) and growth conditions.

Herein a method is provided which uses a set of 36 indicator genes to predict the susceptibility/resistance of *Rosideae,* especially roses, to *Botrytis.* Based on the expression level of the indicator genes conclusions can be drawn about the predicted quality class of a batch of roses (i.e. the predicted severity of *Botrytis* symptom development in the batch). *Botrytis* symptom development is assessed after 7 days at 21°C, 60% relative humidity, and under a light regime of 10h light / 14 h dark. The quality class labeled 'good' refers to batches of flowers with less then 10% of the flowers showing disease symptoms after this 7 days vase-life. Quality class labeled 'moderate' is used for batches having between 10-30% of the flowers showing *Botrytis* disease symptoms. Batches labeled as 'bad' refer to batches of which 30% or more of the flowers show disease symptoms. The expression level of the indicator genes can be used to predict in which class a 'test' batch falls.

In one aspect of the invention a method is provided for detecting the susceptibility / resistance of *Rosideae* (preferably *Rosa,* especially *Rosa hybrida*) to *Botrytis* infection and to the predicted development of *Botrytis* disease symptoms. The mRNA levels of the indicator genes, thus, serve as an indicator of the quality of the plants/plant tissue with respect to *Botrytis* resistance/susceptibility and thus with respect to the predicted severity of *Botrytis* symptoms after 7 days vase-life.

In one embodiment a method for determining the *Botrytis* susceptibility of plants or plant tissue of the family *Rosideae,* especially of the genera *Rosa, Rubus* and *Fragaria,* is provided.

The method provided herein uses a set of 36 indicator genes whose expression profile can be used as measurement of the likelihood that the plant tissue will develop no, mild, or severe Botrytis symptoms (i.e. belongs to the quality class labeled as good, moderate or bad, as described above). Based on the relative or absolute expression level of the described indicator genes conclusions can be drawn about the quality of plants or plant parts regarding *Botrytis* disease susceptibility/resistance.

As shown in the Examples, comparison of expression levels of a set of 36 genes in various batches of roses provided an indication of the susceptibility of a plant or batch to *Botrytis* infection and development subsequently during 1 week, under conditions similar to indoor vase-life. Thus, discrimination between batches which are of bad quality (susceptible and likely to develop severe *Botrytis* symptoms) and good quality (resistant and likely to develop no *Botrytis* symptoms) and moderate quality is possible.

The method for determining the *Botrytis* susceptibility of plants or plant parts (especially cut flowers) of the family *Rosaideae,* or in other words, for predicting the severity of *Bortytis* symptoms that will develop subsequently, comprises the following steps:
(a) providing a nucleic acid sample (comprising mRNA or cDNA) of a plant tissue (or a plurality of plant tissues; batch or batches),
(b) analysing the sample by determining the level of a set of indicator mRNA transcripts in the sample, which are indicative of the *Botrytis* susceptibility/resistance of the plant or batch(es), and optionally
(c) identifying and selecting the plant or plant parts or batch(es) which comprises a certain level of the indicator mRNA transcripts, relative to suitable controls, for further use, e.g. good quality batches can be transported or sold, while bad quality batches can be destroyed.

Optionally, between step (b) and (c), or replacing step (c), the following step may be present:
(b') feeding the resulting data obtained in (b) into a ripening model that relates expression of the indicator genes to ripening stage. This can be done using computer programs. An analogous step (b') can be applied in any of the other embodiments of the invention.

Thus, plants or plant parts which comprise an "indicator mRNA profile" which is indicative of the *Botrytis* susceptibility / resistance level (i.e. the predicted severity of Botrytis symptoms which will develop) can be differentiated and handled differently. Preferably, the method is carried out once (or several times, e.g. at regular time intervals, such as once every two days, once a week, etc.) after harvest, in order to sort plants or batches into different groups based on prediction of *Botrytis* susceptibility.

Any tissue of the plant may be used in the method, for example leaf, flower, stem, root, twigs, fruit, seeds, embryos, pollen, whole seedlings, etc., although preferably, the petals of the flowers are used to prepare the nucleic acid sample. For roses, especially the outer petals are preferred. To have a good coverage of the potency of the whole batch, 20 outer petals are preferably sampled randomly from the batch. Definition of a batch is a product, sampled on the same day from the same greenhouse that have been treated the same from harvest until sampling. Thus, first suitable tissue is sampled for nucleic acid extraction. In the present method, it is preferred that in step (a) nucleic acid samples are prepared by harvesting petal samples of a plant, grinding and mixing sample material and extracting the total RNA or total mRNA from the sample. The sample can be prepared using known nucleic acid extraction methods, e.g. total RNA or mRNA purification methods and kits provided in the art (e.g. RNAeasy kits of Qiagen, kits of SIGMA, Clonetech, etc.). The mRNA may be reverse transcribed into cDNA, using known methods. Expression levels of the genes are preferably analyzed relative to the levels of a training set of batches (same materials and same cultivar) with known occurrence of *Botrytis* infection in the flowers in a vase-life test. Using a training set of at least about 30, more preferably at least about 45 samples, preferably at least about 10, more preferably at least about 15 from each of the three quality classes, the genes expression of new 'test' batches are studied relative to the gene expression of the training set batches in order to predict in which quality class the new 'test' batches fit best.

In step (b), the nucleic acid sample is analyzed for the presence and the level (abundance or relative level) of indicator RNA transcripts (mRNA) in the sample. When referring to indicator RNA in a sample, it is clear that this also encompasses indicator cDNA obtainable from said mRNA. Preferably Real time RT-PCR using primers which amplify the indicator transcripts (or a subset thereof) is used as described in the Examples.

In one embodiment, the mRNA (or cDNA) sequences, which are detected in a sample, and which are indicative of the *Botrytis* susceptibility / resistance of the tissue are SEQ ID NO: 77-109 and/or SEQ ID NO: 172-174, or variants thereof, or fragments of any of these (the main set of indicator genes). Thus, any method may be used to detect the relative or absolute amounts of SEQ ID NO: 77-109 and/or SEQ ID NO: 172-174, variants of SEQ ID NO: 77-109 and/or SEQ ID NO: 172-174, or fragments of these in the sample(s). For example, PCR primer pairs which amplify fragments of each of SEQ ID NO: 77-109 and/or SEQ ID NO: 172-174 may be used in quantitative RT-PCR reactions. Alternatively, the nucleic acid sample may be labeled and hybridized to a nucleic acid carrier comprising oligonucleotides of each of SEQ ID NO: 77-109 and/or SEQ ID NO: 172-174 (and/or variants thereof), whereby the level of these transcripts in the sample is determined. Expression levels may be normalized against housekeeping gene expression levels, such as those of SEQ ID NO: 110-112.

In another embodiment a subset of indicator genes is detected in the sample, and the transcript level is compared to the transcript level of the same subset of indicator genes in a suitable control. A subset may comprise any subset of SEQ ID NO: 77-109 and/or SEQ ID NO: 172-174 (or variants thereof), such as the detection of 20, 15, 10 or less of the sequences.

The expression profile of SEQ ID NO: 77-109 and/or SEQ ID NO: 172-174, and/or variants thereof, predicts the severity of *Botrytis* symptoms which develop later on, after about one week at room temperature (about 21°C). Thus, when the expression levels of the indicator sequences is analyzed and the expression of the indicator genes is such that it fits the expression levels of the batches of the training set labeled as 'good' (measured using exactly the same method, using the same protocol and software programs, such as e.g. Predicted Analysis of Microarray or PAM), it is very likely that the new tested plant material also will have relative low occurrence (less then 10%) of *Botrytis* diseased flowers during post-harvest vase-life, as was found for the batches in quality class 'good' of the train set.
When the expression levels of the indicator sequences is analyzed and the expression of the indicator genes is such that it fits the expression levels of the batches of the training set labeled as 'moderate' (measured using exactly the same method, using the same protocol and software programs, e.g. Predicted Analysis of Microarray), it is very likely that the new tested plant material also will have relative moderate occurrence (between 10-30%) of *Botrytis* diseased flowers during post-harvest vase-life, as was found for the batches in quality class 'moderate' of the train set.
When the expression levels of the indicator sequences is analyzed and the expression of the indicator genes is such that it fits the expression levels of the batches of the train set labeled as 'bad' (measured using exactly the same method, using the same protocol and software programs, such as e.g. Predicted Analysis of Microarray), it is very likely that the new tested plant material also will have relative high occurrence (more then 30%) of Botrytis diseased flowers during post-harvest vase-life, as was found for the batches in quality class 'bad' of the train set.

In a preferred embodiment the "minimal set" of indicator mRNAs comprises at least 2, 3, 4, 5, 6, 7, 8, 9, 10, 15, 20 or more mRNAs selected from SEQ ID NO: 77-109 and/or SEQ ID NO: 172-174 (or variants or fragments thereof).

As already mentioned, it is understood that also "variants" of SEQ ID NO: 77-109 and/or SEQ ID NO: 172-174 may be detected in a sample, such as nucleic acid sequences essentially similar to any of SEQ ID NO: 77-109 and/or SEQ ID NO: 172-174, i.e. comprising at least 70, 75, 80, 85, 90, 95, 98, 99% or more nucleic acid sequence identity to any of SEQ ID NO: 77-109 and/or SEQ ID NO: 172-174. Such variants may for example be present in different species or different varieties.

The actual method used for determining the level of the set of indicator mRNA transcripts is not important. Any gene expression profiling method may be used, such as RT-PCR, microarrays or chips, Northern blot analysis, cDNA-AFLP, etc. See elsewhere herein. For example, PCR primer pairs specific for each of SEQ ID NO: 77-109 and/or SEQ ID NO: 172-174 (or variants thereof) may be designed using known methods. Alternatively, nucleic acid probes, which hybridize to SEQ ID NO: 77-109 and/or SEQ ID NO: 172-174 (or variants thereof) may be made for use in the detection. Any fragment of at least about 10, 12, 14, 15, 20, 22, 30, 50, 100, 200, 300, 500 or more consecutive nucleotides of SEQ ID NO: 77-109 and/or SEQ ID NO: 172-174, or the complement strand, or of a variant of SEQ ID NO: 77-109 and/or SEQ ID NO: 172-174, may be suitable for detection of the full length transcript in a sample. Equally, any fragment of a "variant" of any one of SEQ ID NO: 77-109 and/or SEQ ID NO: 172-174 (as defined above) may be used.

In one embodiment a carrier is provided comprising nucleic acid molecules SEQ ID NO: 77-109 and/or SEQ ID NO: 172-174, variants of SEQ ID NO: 77-109 and/or SEQ ID NO: 172-174 and/or most preferably fragments (oligonucleotides) of any of these or of a subset of any of these. The carrier may, for example, be contacted under hybridizing conditions with the (labeled) nucleic acid sample of the sample of step (a), allowing detection of the level of each of the indicator transcripts present in the sample. The carrier may also comprise housekeeping nucleic acids, such as for example single stranded or double stranded oligonucleotides of SEQ ID NO: 110 to 112, or variants of these.

In a further embodiment, kits, oligonucleotides (e.g. PCR primers, nucleic acid probes) and antibodies are provided, for determining the *Botrytis* sensitivity/resistance of harvested plant tissue. Such kits comprise instructions for use and one or more reagents for use in the method. Optionally, tissue samples or nucleic acid samples suitable as controls may be included. Thus, such a kit may comprise a carrier to receive therein one or more containers, such as tubes or vials. The kit may further comprise unlabeled or labelled oligonucleotide sequences of the invention (SEQ ID NO: 77-109 and/or SEQ ID NO: 172-174, or variants thereof, or parts thereof, such as degenerate primers or probes and optionally also oligonucleotides of housekeeping genes, e.g. those of SEQ ID NO: 110-112 or others), e.g. to be used as primers, probes, which may be contained in one or more of the containers, or present on a carrier. The oligonucleotides may be present in lyophilized form, or in an appropriate buffer. One or more enzymes or reagents for use in isolation of nucleic acids, purification, restriction, ligation and/or amplification reactions may be contained in one or more of the containers. The enzymes or reagents may be present alone or in admixture, and in lyophilised form or in appropriate buffers. The kit may also contain any other component necessary for carrying out the present invention, such as manuals, buffers, enzymes (such as preferably reverse transcriptase and a thermostable polymerase), pipettes, plates, nucleic acids (preferably labelled probes), nucleoside triphosphates, filter paper, gel materials, transfer materials, electrophoresis materials and visualization materials (preferably dyes, labelled antibodies or -enzymes) autoradiography supplies.

### Assays and kits for the determination of cold tolerance in tree seedlings of the family Fagaceae, especially beech seedlings

In one embodiment of the invention a method for determining cold tolerance (or frost tolerance) of *Fagaceae* seedlings, especially in beech seedlings is provided.

Tree seedlings grown in nurseries have to be lifted and transferred to cold storage in autumn. However, lifting at a suboptimal moment, when the seedling is not yet fully cold-hardened, causes reduced vitality of the plants after storage. The only available test method for seedling hardiness is to date electrolyte leakage measurements. However, these measurements are not very accurate and are highly time consuming, taking at least 4 days. This is difficult to fit into nursery logistic schedules. In addition seedlings often have to be transferred to a test lab. During transport the physiology of the plant can be influenced.

The method provided herein uses a set of 29 indicator genes whose expression profile can be used as measurement for the cold tolerance level of *Fagaceae* seedlings, preferably beech seedlings. Based on the relative or absolute expression level of the described indicator genes conclusions can be drawn about the level of cold tolerance that is reached in tree seedlings. It was found that, as soon as the expression of the 29 cold tolerance related genes stabilizes, cold tolerance has reached the maximal level (see Examples).

The method for determining cold tolerance of *Fagaceae* seedlings comprises the following steps:
(a) providing a nucleic acid sample (comprising mRNA or cDNA) of a batch of *Fagaceae* seedlings (e.g. a representative sample of buds),
(b) analysing the sample by determining the level of a set of indicator mRNA transcripts in the sample, which are indicative of the cold tolerance stage of the *Fagaceae* seedlings, and optionally
(c) identifying and selecting the *Fagaceae* seedlings which comprises a certain level of the indicator mRNA transcripts, relative to suitable controls, for further use, e.g. for transfer to cold storage. Thus, seedlings which comprise an "indicator mRNA profile" which is indicative of cold-tolerance are identified.

Optionally, between step (b) and (c), or replacing step (c), the following step may be present:
(b') feeding the resulting data obtained in (b) into a ripening model that relates expression of the indicator genes to ripening stage. This can be done using computer programs. An analogous step (b') can be applied in any of the other embodiments of the invention.

The method can be applied to any tree seedlings of the family *"Fagaceae",* including for example varieties of *Fagus sylvatica* L., other species from the genus *Fagus,* such as *Fagus crenata* (Japanese Beech), *Fagus engleriana* (Chinese Beech), *Fagus grandifolia* (American Beech), *Fagus hayatae* (Taiwan Beech), *Fagus japonica* (Japanese Blue Beech), *Fagus longipetiolata* (South Chinese Beech), *Fagus lucida* (Shining Beech), *Fagus mexicana* (Mexican Beech or Haya), *Fagus orientalis* (Oriental Beech), and other genera of the family *Fagaceae,* such as *Castanea* (chestnuts) and *Quercus* (oaks) species. In a preferred embodiment seedlings of the genus *Fagus,* more preferably of the species *Fagus sylvatica* are used. The seedlings may be of various ages, e.g. one or two years old. They may have been grown in the field or in a controlled environment. Preferably, nucleic acids of a batch of seedlings refers to nucleic acids obtained from a batch of seedlings grown at the same location and under the same growth conditions.

The method can be used to identify and select those tree seedlings which are ready to be transferred to cold storage, without reducing the viability of the seedlings during or after cold storage. Cold storage refers to storage of seedlings for several weeks or months in controlled environments at temperatures of -2 to +4 °C. Thus, the optimal developmental stage of the plants for transfer into cold storage can be assessed. Preferably, the RNA profile of the indicator genes, or of a subset thereof, is analysed more then once, i.e. at one or more time intervals. This allows the expression level of the indicator genes to be compared relative to the earlier level(s). For example, once a month, once every 3, 2, or 1 week, the mRNA profiling method may be repeated until the mRNA profile is found which indicates that the plants are now cold-hardened and ready to be transferred to cold storage. Alternatively, expression levels of a test batch are compared to the expression levels of one or more training batches (for example a batch of cold-sensitive seedlings).

Any tissue of the plant may be used in the method, for example leaf, flower, stem, root, twigs, fruit, seeds, embryos, pollen, whole seedlings, etc., although preferably, the buds of the tree seedlings are used to prepare the nucleic acid sample. Most preferably apical buds are used. Thus, first suitable tissue is sampled for nucleic acid extraction. In the present method, it is preferred that in step (a) a nucleic acid sample is prepared by harvesting bud-tissue of a representative number of plants and extracting the total RNA or total mRNA from the pooled sample. The sample can be prepared using known nucleic acid extraction methods, e.g. total RNA or mRNA purification methods and kits provided in the art (e.g. RNAeasy kits of Qiagen, kits of BIORAD, Clontech, Dynal etc.). The mRNA may be reverse transcribed into cDNA, using known methods.

In step (b), the nucleic acid sample is analysed for the presence and the level (abundance or relative level) of indicator RNA transcripts (mRNA) in the sample. When referring to indicator RNA in a sample, it is clear that this also encompasses indicator cDNA obtainable from said mRNA.

In one embodiment, the mRNA (or cDNA) sequences indicative of cold tolerance which are detected in the sample are SEQ ID NO: 1-29, or variants thereof, or fragments of any of these (the main set of indicator genes). Thus, any method may be used to detect the relative or absolute amounts of SEQ ID NO: 1-29, variants of SEQ ID NO: 1-29, or fragments of these in the sample(s). For example, PCR primer pairs which amplify fragments of each of SEQ ID NO: 1-29 may be used in quantitative RT-PCR reactions. Alternatively, the nucleic acid sample may be labeled and hybridized to a nucleic acid carrier comprising oligonucleotides of each of SEQ ID NO: 1-29, whereby the level of these transcripts in the sample is determined.

In another embodiment a subset of indicator genes is detected in the sample, and the transcript level is compared to the transcript level of the same subset in a suitable control.

SEQ ID NO: 1-15, and variants thereof, are upregulated in cold-tolerant seedlings compared to cold sensitive seedlings (referred to as "upregulated transcripts" indicative of cold tolerance). SEQ ID NO: 16-27, and variants thereof, are downregulated in cold tolerant seedlings compared to cold sensitive seedlings (referred to as "downregulated transcripts" indicative of cold tolerance). Further, SEQ ID NO: 28 and 29, and variants thereof, are about equal in their expression level in cold tolerant compared to cold sensitive seedling. Most preferably, the mRNA or cDNA level of a set of at least 2, 3, 4, 5, 6, 7, 8, 9, 10, 15, 20, 25 or more of any one of SEQ ID NO: 1-29, or variants or fragments thereof, is determined in the sample in step (b). The expression level of the indicator transcripts is preferably compared to the level of transcript of a suitable control, e.g. either the same plant analysed at an earlier stage, or another suitable control sample, such as the sample of a cold-sensitive beech seedling.

In a preferred embodiment the expression level of at least one "upregulated transcript" and at least one "downregulated transcript" are detected. Optionally, also the expression level of a "constant" transcript, e.g. SEQ ID NO: 28 and/or 29, may be detected. Thus, the "minimal set" of indicator mRNAs comprises at least two mRNAs, one selected from SEQ ID NO: 1-15 (or variants or fragments thereof) and one selected from SEQ ID NO: 16-27 (or variants or fragments thereof).

As already mentioned, it is understood that also "variants" of SEQ ID NO: 1-29 may be detected in a sample, such as nucleic acid sequences essentially similar to any of SEQ ID NO: 1-29, i.e. comprising at least 70, 75, 80, 85, 90, 95, 98, 99% or more nucleic acid sequence identity to any of SEQ ID NO: 1-29. Such variants may for example be present in different tree species or different varieties.

The actual method used for determining the level of the set of indicator mRNA transcripts is not important. Any gene expression profiling method may be used, such as RT-PCR, microarrays or chips, Northern blot analysis, cDNA-AFLP, etc. See elsewhere herein. For example, PCR primer pairs for each of SEQ ID NO: 1-29 may be designed using known methods. Suitable primer pairs are, for example, the PCR primer pairs provided in the Examples and depicted in SEQ ID NO: 30 - 41. In one embodiment of the invention two or more of these primer pairs are used in the method. Alternatively, nucleic acid probes, which hybridize to SEQ ID NO: 1-29 may be made for use in the detection. Any fragment of 15, 20, 22, 30, 50, 100, 200, 300, 500 or more consecutive nucleotides of SEQ ID NO: 1-29, or the complement strand, or of a variant of SEQ ID NO: 1-29, may be suitable for detection of the full length transcript in a sample. Equally, any fragment of a "variant" of any one of SEQ ID NO: 1-29 (as defined above) may be used.

In one embodiment a carrier is provided comprising nucleic acid molecules SEQ ID NO: 1-29, variants of SEQ ID NO: 1-29 and/or most preferably fragments (oligonucleotides) of any of these or of a subset of any of these. The carrier may, for example, be contacted under hybridizing conditions with the (labeled) nucleic acid sample of the sample of step (a), allowing detection of the level of each of the indicator transcripts present in the sample.

If the expression profile of the indicator mRNAs of the seedling corresponds to the profile of cold-tolerant tree seedlings, the plant can be identified and selected for further use. Preferably, the seedlings can be transferred to cold storage, as this is now safe to do (without risking reduced viability). Therefore, the method provides a way of determining whether or not seedlings can be transferred to cold storage without loss of viability during storage.

In a further embodiment, kits, oligonucleotides (e.g. PCR primers, nucleic acid probes) and antibodies are provided, for determining the cold-tolerance of tree seedlings. Such kits comprise instructions for use and one or more reagents for use in the method. Optionally, tissue samples or nucleic acid samples suitable as controls may be included. Thus, such a kit may comprise a carrier to receive therein one or more containers, such as tubes or vials. The kit may further comprise unlabeled or labeled oligonucleotide sequences of the invention (SEQ ID NO: 1-29, or variants thereof, or parts thereof, such as degenerate primers or probes), e.g. to be used as primers, probes, which may be contained in one or more of the containers, or present on a carrier. The oligonucleotides may be present in lyophilized form, or in an appropriate buffer. One or more enzymes or reagents for use in isolation of nucleic acids, purification, restriction, ligation and/or amplification reactions may be contained in one or more of the containers. The enzymes or reagents may be present alone or in admixture, and in lyophilised form or in appropriate buffers. The kit may also contain any other component necessary for carrying out the present invention, such as manuals, buffers, enzymes (such as preferably reverse transcriptase and a thermostable polymerase), pipettes, plates, nucleic acids (preferably labelled probes), nucleoside triphosphates, filter paper, gel materials, transfer materials, electrophoresis materials and visualization materials (preferably dyes, labelled antibodies or -enzymes) autoradiography supplies.

### Assays and kits for the determination of the ripening stage of fruit of the family Maloideae, especially pears

Harvested fruit, such as pears, are often stored for several months in cold storage before they are transferred to retail. Storage disorders occur regularly and are usually related to developmental stage at the time of harvest. A proper monitoring of the ripening process would allow selecting batches that are likely to maintain high quality during storage and would prevent the economic losses associated with storage disorders.

At present no reliable measurement for discriminating between various stages of ripening of fruit, such as pears, is available. Firmness measurements are sometimes used, but they have proven to lack the accuracy, that is needed for a good indicator of developmental stage.

In one embodiment a method for determining the ripening stage of fruit of the family *Maloideae,* especially of the genus *Pyrus* or *Malus,* is provided.

The method provided herein uses a set of at least 2, 3, 4, 5 or more indicator genes whose expression profile can be used to discriminate between different (relative) ripening stages of fruit of the family *Maloideae,* preferably pear. Based on the relative or absolute expression level of the described indicator genes conclusions can be drawn about the ripening stage of the fruit that is reached when the fruit are still attached to the plant or post-harvest.

As shown in the Examples, comparison of expression levels of a set of genes in various batches of pears provided information about relative ripening stages. The present method is much more informative than firmness measurements (see Examples). Thus, discrimination between batches is possible in cases where firmness measurements fail.

The method for determining the ripening stage of fruit of the family *Maloideae* comprises the following steps:
(a) providing a nucleic acid sample (comprising mRNA or cDNA) from fruit or fruit tissue (or a plurality of fruit or fruit tissues; batch),
(b) analysing the sample by determining the level of a set of indicator mRNA transcripts in the sample, which are indicative of the ripening stage of the fruit, and optionally
(c) identifying and selecting the fruit which comprises a certain level of the indicator mRNA transcripts, relative to suitable controls, for further use, e.g. for harvest and/or for (cold)storage, processing or sale.

Thus, fruit which comprise an "indicator mRNA profile" which is indicative that the fruit is at a ripening stage which allows harvest and/or cold-storage of the fruit, without quality loss during cold storage, are identified. Also the ripening stage during storage can be followed using the method, allowing the discrimination between batches, which are at different ripening stages. Similarly, storage conditions can be optimized, by testing the effect of various parameters (temperature, humidity, etc.) on the ripening process of fruit.

The method is especially suitable for relative discrimination between batches from the same season. With this method it is possible to discriminate between batches, in situations where known methods (such as firmness measurement) fail. As absolute expression levels will vary from season to season, training batches are preferably developed each season for different ripening stages. In these training batches the ripening stage is roughly correlated to the expression level of the indicator genes. The indicator mRNA level in a "test" batch is then compared relative to that of the training batches and can thereby be assigned a ripening stage. Thereby, relatively more ripe and/or relatively less ripe batches can be differentiated and optionally selected for further use.

The expression level of several indicator genes (SEQ ID NO: 43-46 and/or SEQ ID NO: 158-161 and/or SEQ ID NO: 163-165, and/or SEQ ID NO: 167-171 and variants thereof) increases progressively with ripening, while three genes (SEQ ID NO: 42, SEQ ID NO: 162 and SEQ ID NO: 166 and variants thereof) remain constant. Thus, a relative higher expression level of any of SEQ ID NO: 43-46 and/or SEQ ID NO: 158-161 and/or SEQ ID NO: 163-165, and/or SEQ ID NO: 167-171 (and/or variants thereof) in a batch (e.g. at least about 5x, 10x, 20x, 50x, 100x higher mRNA levels) indicates a more advanced ripening stage of the batch.

The method can be applied to determine the ripening stage of fruit of the family *Maloideae.* In a preferred embodiment fruit of the genus *Pyrus* or *Malus,* preferably of the species *Pyrus communis* L. (e.g. cv. Conference), but may also be applied on any other cultivar of the species, or in other genera from the subfamily of *Maloideae.*

The method can be used to identify and select those fruit which are ready to be harvested and transferred to cold storage, without reducing the quality during or after cold storage. Cold storage refers to storage of seedlings for several weeks or months in controlled environments at temperatures of -2 to +4 °C. Thus, the optimal developmental stage of the plants for harvest and/or transfer into cold storage can be assessed. Alternatively, the ripening stages of different batches, e.g. already in storage, can be compared and unripe or ripe batches can be selected for further use.

Preferably, the RNA profile of the indicator genes, or of a subset thereof, is analysed more then once, i.e. at one or more time intervals. This allows the expression level of the indicator genes to be compared relative to the earlier level(s). The ripening progress of a batch can thereby be followed over time, either prior to harvest and/or after harvest. For example, once a month, once every 3, 2, or 1 week, or once every few days (e.g. at 2 day, 3 day, 4 day or 5 day intervals) the mRNA profiling method may be repeated until the mRNA profile is found which indicates that the fruit are now ready to be harvested and/or ready to be transferred to cold storage.

Any tissue of the plant may be used in the method, for example leaf, flower, stem, root, twigs, fruit, seeds, embryos, pollen, whole seedlings, etc., although preferably, the mesocarp tissue of fruit is used to prepare the nucleic acid sample. Most preferably the control tissue is taken from unripe fruit well before harvest and the expression level of the indicator genes may be compared relative to the level in this unripe batch. For example, the unripe fruit or batch may have an average firmness of at least 6 Newton (measured by penetrometer analysis). Thus, first suitable tissue is sampled for nucleic acid extraction. In the present method, it is preferred that in step (a) nucleic acid samples are prepared by harvesting fruit samples of a plant and extracting the total RNA or total mRNA from the sample. The sample can be prepared using known nucleic acid extraction methods, e.g. total RNA or mRNA purification methods and kits provided in the art (e.g. RNAeasy kits of Qiagen, kits of BIORAD, Clonetech, Dynal etc.). The mRNA may be reverse transcribed into cDNA, using known methods.

In step (b), the nucleic acid sample is analysed for the presence and the level (abundance or relative level) of indicator RNA transcripts (mRNA) in the sample. When referring to indicator RNA in a sample, it is clear that this also encompasses indicator cDNA obtainable from said mRNA.

In one embodiment, the mRNA (or cDNA) sequences indicative of the fruit ripening stage detected in the sample are SEQ ID NO: 42-46 and/or SEQ ID NO: 158-171, or variants thereof, or fragments of any of these. Thus, any method may be used to detect the relative or absolute amounts of one or more of SEQ ID NO: 42-46 and/or SEQ ID NO: 158-171, variants of SEQ ID NO: 42-46 and/or SEQ ID NO: 158-171, or fragments of these in the sample(s). For example, PCR primer pairs which amplify fragments of each of SEQ ID NO: 42-46 and/or SEQ ID NO: 158-171 may be used in quantitative RT-PCR reactions. Alternatively, the nucleic acid sample may be labeled and hybridized to a nucleic acid carrier comprising oligonucleotides of each of SEQ ID NO: 42-46 and/or SEQ ID NO: 158-171, whereby the level of these transcripts in the sample is determined.

SEQ ID NO: 43-46, SEQ ID NO: 158-161, SEQ ID NO: 163-165 and SEQ ID NO: 167-171, and variants thereof, are upregulated during fruit ripening compared to unripe fruit (referred to as "upregulated transcripts" indicative of fruit ripening). Further, SEQ ID NO: 42, SEQ ID NO: 162 and SEQ ID NO: 166, and variants thereof, are about equal in their expression level in ripe fruit tissue compared to unripe tissue (referred to as "constant transcript" indicative of fruit ripening). Most preferably, the mRNA or cDNA level of a set of at least 2, 3, 4 or 5 of any one of SEQ ID NO: 42-46 and/or SEQ ID NO: 158-171, or variants or fragments thereof, is determined in the sample in step (b). The expression level of the indicator transcripts is preferably compared to the level of transcript of a suitable control, e.g. either the same fruit batch analysed at an earlier stage, or another suitable control sample, such as the sample of an unripe fruit, and/or training batches.

As already mentioned, it is understood that also "variants" of SEQ ID NO: 42-46 and/or of SEQ ID NO: 158-171 may be detected in a sample, such as nucleic acid sequences essentially similar to any of SEQ ID NO: 42-46 and/or of SEQ ID NO: 158-171, i.e. comprising at least 70, 75, 80, 85, 90, 95, 98, 99% or more nucleic acid sequence identity to any of SEQ ID NO: 42-46 and/or of SEQ ID NO: 158-171. Such variants may for example be present in different species or different varieties.

The actual method used for determining the level of the set of indicator mRNA transcripts is not important. Any gene expression profiling method may be used, such as RT-PCR, microarrays or chips, Northern blot analysis, cDNA-AFLP, etc. See elsewhere herein. For example, PCR primer pairs for each of SEQ ID NO: 42-46 and/or of SEQ ID NO: 158-171 may be designed using known methods. Suitable primer pairs are, for example, the PCR primer pairs provided in the Examples and depicted in SEQ ID NO: 47-56 and/or of SEQ ID NO: 158-171. In one embodiment of the invention two or more of these primer pairs are used in the method. Alternatively, nucleic acid probes, which hybridize to SEQ ID NO: 42-46 and/or of SEQ ID NO: 158-171 may be made for use in the detection. Any fragment of at least about 15, 20, 22, 30, 50, 100, 200, 300, 500 or more consecutive nucleotides of SEQ ID NO: 42-46 and/or of SEQ ID NO: 158-171, or the complement strand, or of a variant of SEQ ID NO: 42-46 and/or of SEQ ID NO: 158-171, may be suitable for detection of the full length transcript in a sample. Equally, any fragment of a "variant" of any one of SEQ ID NO: 42-46 and/or of SEQ ID NO: 158-171 (as defined above) may be used.

In one embodiment a carrier is provided comprising nucleic acid molecules SEQ ID NO: 42-46 and/or of SEQ ID NO: 158-171, variants of SEQ ID NO: 42-46 and/or of SEQ ID NO: 158-171 and/or most preferably fragments (oligonucleotides) of any of these or of a subset of any of these. The carrier may, for example, be contacted under hybridizing conditions with the (labeled) nucleic acid sample of the sample of step (a), allowing detection of the level of each of the indicator transcripts present in the sample.

In practice the expression profile of the indicator mRNAs of the fruit can be used determine the optimal moment for harvest, depending on choices for downstream chains, e.g. ready- to-eat delivery to local retail, export or long-term storage without risking storage disorders developing.

In a further embodiment, kits, oligonucleotides (e.g. PCR primers, nucleic acid probes) and antibodies are provided, for determining the ripening stage of fruit. Such kits comprise instructions for use and one or more reagents for use in the method. Optionally, tissue samples or nucleic acid samples suitable as controls may be included. Thus, such a kit may comprise a carrier to receive therein one or more containers, such as tubes or vials. The kit may further comprise unlabeled or labelled oligonucleotide sequences of the invention (SEQ ID NO: 42-46 and/or of SEQ ID NO: 158-171, or variants thereof, or parts thereof, such as degenerate primers or probes), e.g. to be used as primers, probes, which may be contained in one or more of the containers, or present on a carrier. The oligonucleotides may be present in lyophilized form, or in an appropriate buffer. One or more enzymes or reagents for use in isolation of nucleic acids, purification, restriction, ligation and/or amplification reactions may be contained in one or more of the containers. The enzymes or reagents may be present alone or in admixture, and in lyophilised form or in appropriate buffers. The kit may also contain any other component necessary for carrying out the present invention, such as manuals, buffers, enzymes (such as preferably reverse transcriptase and a thermostable polymerase), pipettes, plates, nucleic acids (preferably labelled probes), nucleoside triphosphates, filter paper, gel materials, transfer materials, electrophoresis materials and visualization materials (preferably dyes, labelled antibodies or -enzymes) autoradiography supplies.

### Assays and kits for the determination of sensory decay of fruit of the family Maloideae, preferably of the genus Malus or Pyrus, especially apples

Fruit, such as apples are stored for up to 9 months before they are transferred to retail and consumers. During storage quality decay may occur, of which the severity is related to the physiological status of the apples at the start of the storage period. A commonly occurring storage disorder is "mealiness". This characteristic is cultivar (genotype) dependent but there are also large batch differences (phenotype). Soft apples are B quality and often have to be discarded.

Mealiness of apples is to date measured using a penetrometer, which registers firmness. In practice fruit samples are taken during storage and tested for sensory aspects by human taste, or using firmness measurements. All these measurements detect secondary effects and can not be used for early warning.

In one aspect of the invention a method is provided for detecting early changes in relative expression levels of indicator genes, which serve as an early warning for sensory decay in fruit, especially apples after harvest. Using method which rely on firmness (softening) or sensory analysis by humans (assessing mealiness, flavor, odor, juiciness, etc.) one can only detect deterioration of fruit quality once it is already quite advanced (e.g. three weeks after placement into suboptimal storage conditions). In the present method much earlier signs of quality loss, especially sensory quality loss, can be determined (already one week after placement into suboptimal storage conditions).

In one embodiment a method for detecting sensory decay in fruit of the family *Maloideae,* especially of the genus *Pyrus* or *Malus,* is provided.

The method provided herein uses a set of 20 indicator genes whose expression profile can be used as measurement for the (relative) sensory decay of fruit of the family *Maloideae,* preferably apple. Based on the relative or absolute expression level of the described indicator genes conclusions can be drawn about the stage of sensory decay of the fruit that is reached post-harvest.

As shown in the Examples, comparison of expression levels of a set of 20 genes (or variants thereof, or subsets thereof) in various batches of apples provided an early warning of sensory decay. Thus, discrimination between batches which are starting to develop sensory decay and between good quality batches is possible.

The method for detecting signs of sensory decay of fruit of the family *Maloideae* comprises the following steps:
(a) providing a nucleic acid sample (comprising mRNA or cDNA) of a fruit or fruit tissue (or a plurality of fruit or fruit tissues; batch),
(b) analysing the sample by determining the level of a set of indicator mRNA transcripts in the sample, which are indicative of the sensory decay stage of the fruit, and optionally
(c) identifying and selecting the fruit which comprises a certain level of the indicator mRNA transcripts, relative to suitable controls (e.g. training batches, or batches of known sensory decay stages such as a sample taken at harvest time when no decay has taken place yet), for further use, e.g. for removal from storage and immediate processing or sale.

Thus, fruit which comprise an "indicator mRNA profile" which is indicative that the fruit (or batch) has already initiated sensory decay allows decaying fruit or batches comprising decaying fruit to be differentiated and removed from storage. Also the sensory decay during storage can be followed using the method, allowing the discrimination between batches, which are at different sensory decay stages. Similarly, storage conditions can be optimized, by testing the effect of various parameters (temperature, humidity, etc.) on the sensory decay process of fruit.

The method can be applied to determine the sensory decay stage of fruit of the family *Maloideae.* In a preferred embodiment fruit of the genus *Pyrus* or *Malus,* preferably of the species *Malus domestica* (e.g. cv. Cox orange).

Preferably, the RNA profile of the indicator genes, or of a subset thereof, is analysed more then once, i.e. at one or more time intervals. This allows the expression level of the indicator genes to be compared relative to the earlier level(s). For example, once a month, once every 3, 2, or 1 week, or several times a week, the mRNA profiling method may be repeated until the mRNA profile is found which indicates that the fruit or batch shows early signs of sensory decay.

Any tissue of the plant may be used in the method, for example leaf, flower, stem, root, twigs, fruit, seeds, embryos, pollen, whole seedlings, etc., although preferably, the mesocarp tissue of fruit is used to prepare the nucleic acid sample. Most preferably the control tissue is taken from fruit at harvest time, when no sensory decay has occurred. SEQ ID NO: 57-66 (and variants thereof) are upregulated , while SEQ ID NO: 67-76 (and variants thereof) are downregulated relative to a sample taken at harvest time, indicating sensory decay of the batch. Thus, first suitable tissue is sampled for nucleic acid extraction. In the present method, it is preferred that in step (a) nucleic acid samples are prepared by harvesting fruit samples of a plant and extracting the total RNA or total mRNA from a pooled sample. The sample can be prepared using known nucleic acid extraction methods, e.g. total RNA or mRNA purification methods and kits provided in the art (e.g. RNAeasy kits of Qiagen, kits of SIGMA, Clonetech, etc.). The mRNA may be reverse transcribed into cDNA, using known methods.

In step (b), the nucleic acid sample is analysed for the presence and the level (abundance or relative level) of indicator RNA transcripts (mRNA) in the sample. When referring to indicator RNA in a sample, it is clear that this also encompasses indicator cDNA obtainable from said mRNA.

In one embodiment, the mRNA (or cDNA) sequences, which are detected in a sample, and which are indicative of the sensory decay are SEQ ID NO: 57-76, or variants thereof, or fragments of any of these (the main set of indicator genes). Thus, any method may be used to detect the relative or absolute amounts of SEQ ID NO: 57-76, variants of SEQ ID NO: 57-76, or fragments of these in the sample(s). For example, PCR primer pairs which amplify fragments of each of SEQ ID NO: 57-76 may be used in quantitative RT-PCR reactions. Alternatively, the nucleic acid sample may be labeled and hybridized to a nucleic acid carrier comprising oligonucleotides of each of SEQ ID NO: 57-76 (and/or variants thereof), whereby the level of these transcripts in the sample is determined.

In another embodiment a subset of indicator genes is detected in the sample, and the transcript level is compared to the transcript level of the same subset in a suitable control.

SEQ ID NO: 57-66, and variants thereof, are upregulated when sensory decay is initiated, compared to non-decaying fruit (referred to as "upregulated transcripts" indicative of sensory decay). Further, SEQ ID NO: 67-76, and variants thereof, are downregulated when sensory decay is initiated, compared to non-decaying fruit (referred to as "downregulated transcripts" indicative of sensory decay). Most preferably, the mRNA or cDNA level of a set of at least 2, 3, 4, 5, 6, 7, 8, 9, 10, 15, 18 or more (e.g. 20) of any one of SEQ ID NO: 57-76, and/or variants or fragments thereof, is determined in the sample in step (b). The expression level of the indicator transcripts is preferably compared to the level of transcript of a suitable control, e.g. either the same fruit analysed at an earlier stage (non decaying), or another suitable control sample, such as the sample of an non-decaying fruit and/or training batches of known decay stages. The method is, thus, especially suitable for discriminating between various fruit batches after harvest, such as non-decaying, slightly decaying, very decaying batches, etc.

In a preferred embodiment the expression level of at least one "upregulated transcript" and one "downregulated transcript" are detected. Thus, the "minimal set" of indicator mRNAs comprises at least two mRNAs, one selected from SEQ ID NO: 57-66 (or variants or fragments thereof) and one selected from SEQ ID NO: 67-76 (or variants or fragments thereof).

As already mentioned, it is understood that also "variants" of SEQ ID NO: 57-76 may be detected in a sample, such as nucleic acid sequences essentially similar to any of SEQ ID NO: 57-76, i.e. comprising at least 70, 75, 80, 85, 90, 95, 98, 99% or more nucleic acid sequence identity to any of SEQ ID NO: 57-76. Such variants may for example be present in different species or different varieties.

The actual method used for determining the level of the set of indicator mRNA transcripts is not important. Any gene expression profiling method may be used, such as RT-PCR, microarrays or chips, Northern blot analysis, cDNA-AFLP, etc. See elsewhere herein. For example, PCR primer pairs for each of SEQ ID NO: 57-76 may be designed using known methods. Suitable primer pairs are, for example, the PCR primer pairs provided in the Examples and depicted in SEQ ID NO: 57-76. In one embodiment of the invention two or more of these primer pairs are used in the method. Alternatively, nucleic acid probes, which hybridize to SEQ ID NO: 57-76 may be made for use in the detection. Any fragment of at least about 10, 12, 15, 20, 22, 30, 50, 100, 200, 300, 500 or more consecutive nucleotides of SEQ ID NO: 57-76, or the complement strand, or of a variant of SEQ ID NO: 57-76, may be suitable for detection of the full length transcript in a sample. Equally, any fragment of a "variant" of any one of SEQ ID NO: 57-76 (as defined above) may be used.

In one embodiment a carrier is provided comprising nucleic acid molecules SEQ ID NO: 57-76, variants of SEQ ID NO: 57-76 and/or most preferably fragments (oligonucleotides) of any of these or of a subset of any of these. The carrier may, for example, be contacted under hybridizing conditions with the (labeled) nucleic acid sample of the sample of step (a), allowing detection of the level of each of the indicator transcripts present in the sample.

If the expression profile of the indicator mRNAs of the fruit corresponds to the profile of fruit which have initiated sensory decay, or which are non-decaying, or at an advanced stage of decay, the plant, fruit or batch can be identified and selected for further use (or for being discarded). Preferably, the fruit or batch of fruit can be selected and removed from non-decaying fruit or batches.

In a further embodiment, kits, oligonucleotides (e.g. PCR primers, nucleic acid probes) and antibodies are provided, for determining the stage of sensory decay of fruit. Such kits comprise instructions for use and one or more reagents for use in the method. Optionally, tissue samples or nucleic acid samples suitable as controls may be included. Thus, such a kit may comprise a carrier to receive therein one or more containers, such as tubes or vials. The kit may further comprise unlabeled or labeled oligonucleotide sequences of the invention (SEQ ID NO: 57-76, or variants thereof, or parts thereof, such as primers or probes), e.g. to be used as primers, probes, which may be contained in one or more of the containers, or present on a carrier. The oligonucleotides may be present in lyophilized form, or in an appropriate buffer. One or more enzymes or reagents for use in isolation of nucleic acids, purification, restriction, ligation and/or amplification reactions may be contained in one or more of the containers. The enzymes or reagents may be present alone or in admixture, and in lyophilised form or in appropriate buffers. The kit may also contain any other component necessary for carrying out the present invention, such as manuals, buffers, enzymes (such as preferably reverse transcriptase and a thermostable polymerase), pipettes, plates, nucleic acids (preferably labelled probes), nucleoside triphosphates, filter paper, gel materials, transfer materials, electrophoresis materials and visualization materials (preferably dyes, labelled antibodies or -enzymes) autoradiography supplies.

### Assays and kits for the prediction of brown discoloration in edible mushrooms

Edible mushrooms, such as *Agaricus bisporus,* are consumed worldwide, both as fresh product or processed in pots, canned, frozen etc. For many mushrooms, and especially white mushrooms, product quality is generally judged visually, based on colour. Fresh *Agaricus bisporus* has a white cap and stalk, but the colour of the cap or the gills can unexpectedly change to a light or darker brown colour, lowering the product's quality. Thus, a test to predict the quality, days before severe browning will occur, would be of great potential value for the mushroom industry.

So far, no such tests are available. A visually screen by growers or inspectors does not give a conclusive prediction. Also computer image analysis has been tried but also these do not give solid predictions about how fast the mushroom product will decay.

The present inventors were able to develop an assay to indicate the freshness stage of mushrooms and the product quality stage prior to visual sign, such as browning of the cap. Twenty-three indicator genes depicted in SEQ ID NO: 113-135 (of which 7 sequences, depicted in SEQ ID NO: 113-119, where previously published by other investigators), were selected, whose expression correlates with browning of the tissue. Thus, a specific expression profile of these indicator genes in a sample indicates, relative to other batches, what the time span is in which the batch is predicted to start browning.

Thus, in one aspect of the invention a method is provided for detecting early changes in relative expression levels of indicator genes, which serve as an early warning for browning in edible mushrooms. Using methods which rely on visual symptoms of browning one can only detect deterioration of mushroom quality once it is already visible. In the present method much earlier signs of quality loss can be determined.

In one embodiment a method for detecting the quality stage (browning stage) of edible mushrooms, especially of edible homobasidiomycetes, such as edible species of the families *Agaricaceae, Tricholomataceae, Lepista, Pleurotaceae, Cantharellaceae,* and *Boletaceae,* is provided. Most preferably, the method is used in Agaricus species, especially *Agaricus bisporus,* and in shiitake (*Lentinus edodes*), *Pleurotus ostreatus* (Oyster mushroom), *Lepista nuda* (synonyms *Clitocybe nuda, Tricholoma nudum* en *Rhodopaxillus nudus*) which are close relatives of *Agaricus bisporus,* as well as *Cantharellus cibarius* and *Boletus edulis.*

The method provided herein a set of 23 indicator genes whose expression profile can be used as measurement for the (relative) browning stage of fresh mushrooms and fresh mushroom based products. Based on the relative or absolute expression level of the described indicator genes conclusions can be drawn about the stage of browning and freshness of the mushroom that is reached post-harvest.

As shown in the Examples, comparison of expression levels of a set of 23 genes correlates with browning stages, prior to visible browning being seen. Thus, discrimination between batches which are starting to develop browning (although not yet visible) and between good quality batches is possible.

The method for detecting signs of quality loss (initiation of browning) of edible mushrooms, especially homobasidiomycetes, comprises the following steps:
(a) providing a nucleic acid sample (comprising mRNA or cDNA) of a mushroom or mushroom tissue (or a plurality of mushrooms or mushroom tissues; batch or batches),
(b) analysing the sample by determining the level of a set of indicator mRNA transcripts in the sample, which are indicative of the quality stage of the mushrooms, and optionally
(c) identifying and selecting the mushrooms which comprises a certain level of the indicator mRNA transcripts, relative to suitable controls, for further use, e.g. for immediate processing or sale.

Thus, mushrooms which comprise an "indicator mRNA profile" which is indicative that the mushrooms (or batch) has already initiated browning allows mushrooms or batches comprising a more advanced browning stage to be differentiated and removed from mushrooms which show no signs of quality loss. Also the browning stage during storage can be followed using the method, allowing the discrimination between batches, which are at different browning stages. Similarly, mushroom production and storage conditions can be optimized, by testing the effect of various parameters (temperature, humidity, compost etc.) on the browning process of mushrooms.

The method can be applied to determine the browning stage of mushrooms of the above families. Most preferably, it is applied to white mushrooms, especially *Agaricus bisporus.*

Any tissue of the mushroom may be used in the method, for example the cap, stem, or any other part of the fruiting body. Preferably cap tissue is used to prepare the nucleic acid sample. To have a good coverage of the potency of the whole batch, preferably at least about 10-20 fruiting bodies are sampled randomly from the batch. Definition of a batch is a product, sampled on the same day from the same climate room and that have been treated the same from harvest until sampling. Thus, first suitable tissue is sampled for nucleic acid extraction. In the present method, it is preferred that in step (a) nucleic acid samples are prepared by harvesting mushroom samples, grind and mix sample material and extracting the total RNA or total mRNA from the sample. The sample can be prepared using known nucleic acid extraction methods, e.g. total RNA or mRNA purification methods and kits provided in the art (e.g. RNAeasy kits of Qiagen, kits of SIGMA, Clonetech, etc.). The mRNA may be reverse transcribed into cDNA, using known methods. Preferably expression levels of the indicator genes are analyzed relative to the expression level of the indicator genes in a training set of batches having a known browning stage (see herein below). Having a training set of at least about 30, preferably at least about 45 samples, e.g. at least about 10 or at least about 15 from three 'browning stage' classes, the genes expression of new 'test' batches is determined relative to the gene expression of the indicator genes in the training set batches, to predict in which quality class the new 'test' batches fit best.

In step (b), the nucleic acid sample is analysed for the presence and the level (abundance or relative level) of indicator RNA transcripts (mRNA) in the sample. When referring to indicator RNA in a sample, it is clear that this also encompasses indicator cDNA obtainable from said mRNA.

In one embodiment, the mRNA (or cDNA) sequences, which are detected in a sample, and which are indicative of the browning (or predicted browning) are SEQ ID NO: 113-135, or variants thereof, or fragments of any of these (the main set of indicator genes). Thus, any method may be used to detect the relative or absolute amounts of SEQ ID NO: 113-135, variants of SEQ ID NO: 113-135, or fragments of these in the sample(s). For example, PCR primer pairs which amplify fragments of each of SEQ ID NO: 113-135 may be used in quantitative RT-PCR reactions. Alternatively, the nucleic acid sample may be labeled and hybridized to a nucleic acid carrier comprising oligonucleotides of each of SEQ ID NO: 113-135, whereby the level of these transcripts in the sample is determined.

In another embodiment a subset of indicator genes is detected in the sample, and the transcript level is compared to the transcript level of the same subset in a suitable control. Most preferably, the mRNA or cDNA level of a set of at least 2, 3, 4, 5, 8, 10, 15, 18 or more (e.g. 20) of any one of SEQ ID NO: 113-135, and/or variants or fragments thereof, is determined in the sample in step (b). The expression level of the indicator transcripts is preferably compared to the level of transcript of a suitable control, e.g. either the same mushroom analysed at an earlier stage, or another suitable control sample, such as the sample of a fresh, white mushroom.

As already mentioned, it is understood that also "variants" of SEQ ID NO: 113-135 may be detected in a sample, such as nucleic acid sequences essentially similar to any of SEQ ID NO: 113-135, i.e. comprising at least 70, 75, 80, 85, 90, 95, 98, 99% or more nucleic acid sequence identity to any of SEQ ID NO: 113-135. Preferably, the putative linker sequence present at the 5' end (as shown in the Sequence Listing) is removed prior to sequence alignment. Such variants may for example be present in different species or different varieties of edible mushrooms.

The actual method used for determining the level of the set of indicator mRNA transcripts is not important. Any gene expression profiling method may be used, such as RT-PCR, microarrays or chips, Northern blot analysis, cDNA-AFLP, etc. See elsewhere herein. For example, PCR primer pairs for each of SEQ ID NO: 113-135 may be designed using known methods. Alternatively, nucleic acid probes, which hybridize to SEQ ID NO: 113-135 may be made for use in the detection. Any fragment of at least about 10, 12, 14, 15, 20, 22, 30, 50, 100, 200, 300, 500 or more consecutive nucleotides of SEQ ID NO: 113-135, or the complement strand, or of a variant of SEQ ID NO: 113-135, may be suitable for detection of the full length transcript in a sample. Equally, any fragment of a "variant" of any one of SEQ ID NO: 113-135 (as defined above) may be used.

In one embodiment a carrier is provided comprising nucleic acid molecules SEQ ID NO: 113-135, variants of SEQ ID NO: 113-135 and/or most preferably fragments (oligonucleotides) of any of these or of a subset of any of these. The carrier may, for example, be contacted under hybridizing conditions with the (labeled) nucleic acid sample of the sample of step (a), allowing detection of the level of each of the indicator transcripts present in the sample.

If the expression profile of the indicator mRNAs of the mushrooms corresponds to the profile of mushrooms in a training set which is known to have good postharvest storability potency (days until visual browning appears), the new 'test' mushrooms are likely also to have good storability capacity (e.g. at least about 5, 6, 7 or more days at 2°C) without visible signs of browning developing.
When the expression levels of the indicator sequences is analyzed and the expression of the indicator genes is such that it fits the expression levels of the batches of a training set labeled as 'moderate storability' (measured using exactly the same method, using the same protocol and software programs, such as e.g. Predicted Analysis of Microarray or PAM), it is very likely that the new 'test' mushroom material also will have relative moderate post-harvest storability potency (e.g. it can be stored at least about 2-5 days at 2°C without developing visible browning).
When the expression levels of the indicator sequences is analyzed and the expression of the indicator genes is such that it fits the expression levels of the batches of a training set labeled as 'bad' (measured using exactly the same method, using the same protocol and software programs, such as e.g. Predicted Analysis of Microarray), it is very likely that the new 'test' mushroom material also will have relative low storability potency and can only be stored only for a very short time without browning (e.g. it can be stored for two days or for less than 2 days at 2°C without developing visible browning).

These batch quality indications can be used to select batches for specific markets, like far away countries with long logistic track (good quality), markets that require high quality mushrooms, local markets or discount markets, value packages (moderate quality) or processing industry (low quality).

In a further embodiment, kits, oligonucleotides (e.g. PCR primers, nucleic acid probes) and antibodies are provided, for determining the stage of browning. Such kits comprise instructions for use and one or more reagents for use in the method. Optionally, tissue samples or nucleic acid samples suitable as controls may be included. Thus, such a kit may comprise a carrier to receive therein one or more containers, such as tubes or vials. The kit may further comprise unlabeled or labelled oligonucleotide sequences of the invention (SEQ ID NO: 113-135, or variants thereof, or parts thereof, such as degenerate primers or probes), e.g. to be used as primers, probes, which may be contained in one or more of the containers, or present on a carrier. The oligonucleotides may be present in lyophilized form, or in an appropriate buffer. One or more enzymes or reagents for use in isolation of nucleic acids, purification, restriction, ligation and/or amplification reactions may be contained in one or more of the containers. The enzymes or reagents may be present alone or in admixture, and in lyophilised form or in appropriate buffers. The kit may also contain any other component necessary for carrying out the present invention, such as manuals, buffers, enzymes (such as preferably reverse transcriptase and a thermostable polymerase), pipettes, plates, nucleic acids (preferably labelled probes), nucleoside triphosphates, filter paper, gel materials, transfer materials, electrophoresis materials and visualization materials (preferably dyes, labelled antibodies or -enzymes) autoradiography supplies.

### Assays and kits for the determination / prediction of post-harvest loss of firmness in Solanaceous fruit, such as tomatoes

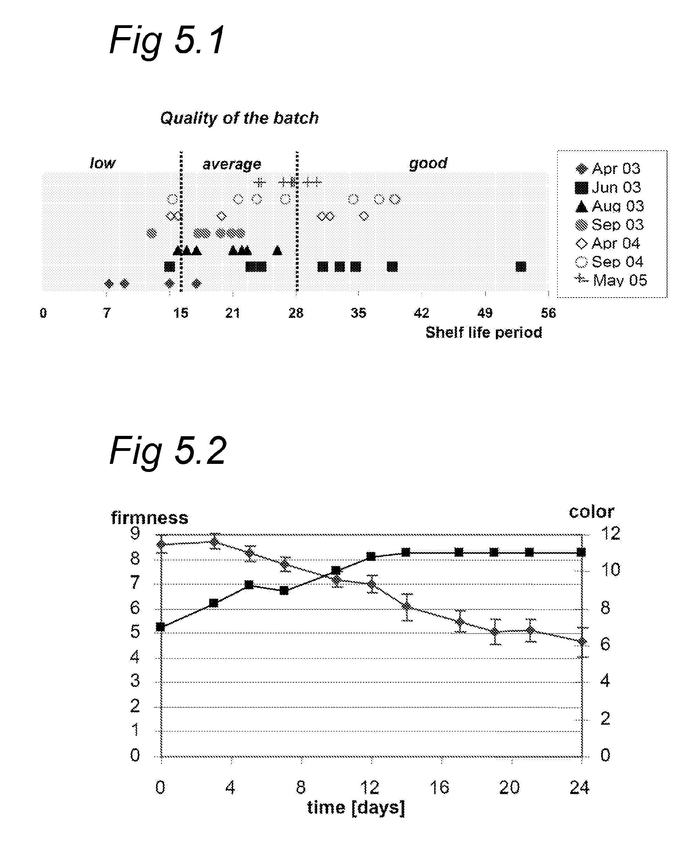

Post-harvest quality loss in fleshy fruits, such as tomatoes, can be separated into various components. One of the most prominent component is loss of firmness. The biological variation with respect to this characteristic between cultivars and between batches of the same cultivar is large. Genetic variation has been explored to a large extent by breeders and has resulted in tomato cultivars that produce fruit with long tenability. However, even in the best-performing tomato cultivars the intra-cultivar variation can still result in batches of fast-softening tomatoes. Figure 5.1 shows the biological variation with respect to firmness in tomatoes with the same genetic background (cultivar Aromata) but cultured by different growers and in different seasons. In addition, extended firmness is often associated with a decrease in flavor and aroma components, resulting in the recent trend towards softer, less tenable fruits, such as tomatoes. Both the use of cultivars with shorter shelf-lives and the non-genetic, environmentally induced biological variation enhance the need for reliable quality monitoring tools for use in trade and distribution of fresh fruits, such as tomatoes.

Tests available at present can be used for monitoring actual firmness, but do not allow predicting future firmness. The future firmness is the most important factor in deciding on distribution chains for harvested fleshy fruit batches, e.g. tomato batches.

Herein a method is provided which uses a set of 19 indicator genes and optionally 3 control genes to predict the post-harvest firmness development of fruits from Solanaceous species, especially tomatoes. Based on the expression level of the indicator genes conclusions can be drawn about the predicted quality class of a batch of fruit, e.g. a batch of tomatoes. For tomatoes, firmness development is generally assessed every 2 or 3 days during a 4 week period. During this period the batches are stored in climate-controlled rooms at 18°C and 75% relative humidity. These conditions were chosen in the Examples to induce a decrease in quality at a moderate speed that would allow for accurate measurements of loss of firmness and gene expression levels over time.

"Firmness" of harvested fruit can be assessed using physical means and can be determined for example on a scale of 2 to 8 (Sensoric values, with 2 being very soft, 5 being firm and 8 being extra hard; see Examples) and/or Instron values on a scale of 0 to -1.3mm (whereby 0 mm is extra hard, -0.6 mm is firm and -1.3 mm is very soft; see Examples). The critical sensoric firmness value of about 5 (corresponding to the Instron value of about -0.6 mm) has been found to be the lowest firmness level that is still acceptable to consumers. The sensoric value of 5 is therefore referred to as the "critical firmness value" herein, at least for tomatoes. The critical firmness value may be different for other fruits, but can be established by the skilled person.

The quality class labeled 'good' refers to batches of tomatoes that have a shelf life of about 28 days or more before they drop below the critical firmness value of 5. Quality class labeled 'average' is used for batches having between about 15 and 28 days shelf life. Batches labeled as 'bad' refer to batches that drop below the critical firmness value of 5 within about 15 days after harvest.

In one aspect of the invention a method is provided for determining and predicting the post-harvest firmness development (or 'loss of firmness'; development of 'softness') of Solanaceous fruits, preferably tomatoes (*Solanum lycopersicum*), but also of other Solanaceous fruit, such as peppers (*Capsicum annuum; Capsicum frutescens,* etc.) and aubergines (*Solanum melongena*). The mRNA levels of a set of indicator genes, thus, serve as an indicator of the quality of the fruit with respect to firmness loss and one can determine early on whether a batch of fruit has a long or short shelf life and a slow or rapid loss of firmness respectively.

Thus, in one embodiment a method for determining the firmness development of fruits of the family *Solanaceae,* especially of the genera *Capsicum* and *Solanum,* is provided.

The method provided herein uses a set of 19 indicator genes whose expression profile can be used as measurement of the likelihood that the fruits will loose firmness faster than average. Based on the relative or absolute expression level of the described indicator genes conclusions can be drawn about the quality of plants or plant parts regarding their predicted firmness decrease during post-harvest storage.

As shown in the Examples, comparison of expression levels of a set of 19 genes in various batches of tomatoes provided an indication of the future firmness decrease of a fruit or batch, under conditions similar to storage conditions in practice. Thus, early discrimination between batches which are of "poor" quality (likely to show rapid decrease in firmness) and "good" quality (likely to show slow decrease in firmness) is possible.

The method for determining (predicting) the future firmness loss of fruits (especially tomato fruits) of the family *Solanaceae* comprises the following steps:
(a) providing a nucleic acid sample (comprising mRNA or cDNA) of a plant tissue (or a plurality of plant tissues; batch),
(b) analysing the sample by determining the level of a set of indicator mRNA transcripts in the sample, which are indicative of the firmness development of the fruit or batch, and optionally
(c) identifying and selecting the plant or plant parts or batch which comprises a certain level of the indicator mRNA transcripts, relative to suitable controls, for further use, e.g. good quality batches can be transported or sold or stored for longer (as they soften slowly and have a longer shelf life), while bad quality batches can be destroyed or sold immediately (as they soften faster and have a shorter shelf life).

Thus, plants or plant parts which comprise an "indicator mRNA profile" which is indicative of the post-harvest firmness development can be differentiated and handled differently.

Preferably, the method is carried out once (or several times, e.g. at regular time intervals, such as once every two days, once a week, etc.) after harvest, in order to sort plants or batches into different groups based on prediction of firmness development.

Any tissue of the fruit may be used in the method, for example pericarp, mesocarp, stem etc., although preferably, the mesocarp is used to prepare the nucleic acid sample. To have a good coverage of the potency of the whole batch, preferably at least about 15, more preferably at least about 20 individual fruits are sampled randomly from the batch. Definition of a batch is a product, sampled at the same day from the same greenhouse that have been treated the same from harvest until sampling. Thus, first suitable tissue is sampled for nucleic acid extraction. In the present method, it is preferred that in step (a) nucleic acid samples are prepared by harvesting mesocarp samples of a fruit, grind and mix sample material and extracting the total RNA or total mRNA from the sample. The sample can be prepared using known nucleic acid extraction methods, e.g. total RNA or mRNA purification methods and kits provided in the art (e.g. RNAeasy kits of Qiagen, kits of SIGMA, Clonetech, etc.). The mRNA may be reverse transcribed into cDNA, using known methods. Expression levels of the indicator genes are preferably analyzed relative to a training set of batches (preferably same plant materials and same cultivar) with known development of firmness over time in a shelf-life test. Having a training set of at least about 25 or 30, preferably at least about 40 or 45 samples, i.e. at least about 10, 12, or more, e.g.15 samples from each quality class (for example 15 samples from a 'good' class and 15 samples from a 'bad' class, as described above), the gene expression of new 'test' batch(es) is then analyzed relative to the indicator gene expression of the training set batches to predict in which quality class the new 'test' batch(es) fit(s) best.

In step (b), the nucleic acid sample is analysed for the presence and the level (abundance or relative level) of indicator RNA transcripts (mRNA) in the sample. When referring to indicator RNA in a sample, it is clear that this also encompasses indicator cDNA obtainable from said mRNA. Preferably Real time RT-PCR using primers which amplify the indicator transcripts (or a subset thereof) is used as described in the Examples.

In one embodiment, the mRNA (or cDNA) sequences, which are detected in a sample, and which are indicative of the firmness development of the tissue are selected from or consist of SEQ ID NO: 136-154, optionally including one or more of SEQ ID NO: 155-157, or variants or fragments of any of these. SEQ ID NO: 136-154 (or variants or fragments thereof) is herein referred to as the "main set" of indicator genes. Thus, any method may be used to detect the relative or absolute amounts of SEQ ID NO: 136-154 or variants or fragments of these in the sample(s). For example PCT primer pairs which amplify fragments of each of SEQ ID NO: 136-154 may be used in qPCR reactions. Alternatively, the nucleic acid sample may be labeled and hybridized to a nucleic acid carrier comprising oligonucleotides of each of SEQ ID NO: 136-154 (and optionally 155-157; and/or variants of any of these) whereby the level of these transcripts in the sample is determined. Expression levels may be normalized against the expression levels of genes having a "constant" expression during fruit storage, such as e.g. those of SEQ ID NO: 155-157.

In another embodiment a subset of indicator genes is detected in the sample, and the transcript level is compared to the transcript level of the same subset of indicator genes in a suitable control. A subset may comprise any subset of SEQ ID NO: 136-154 (or variants thereof), such as the detection of 20, 15, 10, 6, 5, 4, 3 or less (e.g. 3) of the sequences.

SEQ ID NO: 136-144, and variants thereof, are down-regulated in poor quality batches (referred to as "down-regulated transcripts indicative of rapid loss of firmness and shorter shelf-life), i.e. batches which are predicted to develop a rapid loss of firmness during storage. Further, SEQ ID NO: 145-154 are up-regulated in poor quality batches (referred to as "up-regulated transcripts indicative of rapid loss of firmness and shorter shelf-life).

In a preferred embodiment the expression level of at least one, preferably at least 1, 2, 3, 4 or 5 of the "down-regulated" transcripts and at least one, preferably at least 1, 2, 3, 4 or 5 of the "up-regulated" transcripts is determined. Therefore, a "minimal set" of indicator mRNA transcripts preferably comprises at least 2 transcripts, one from the up-regulated set and one from the down-regulated set.

The expression profile of SEQ ID NO: 136-154, and/or variants thereof, predicts the speed with which firmness decreases in the 4 to 6 weeks after harvest. Thus, when the expression levels of the indicator sequences is analyzed and the expression of the indicator genes is such that it fits the expression levels of the batches of the training set labeled as 'good' (measured using exactly the same method, using the same protocol and using software programs like Predicted Analysis of Microarray), it is very likely that the new tested plant material also will have slow decrease of firmness (drops below the value of 5 after 28 days or more) as was found for the batches in quality class 'good' of the train set.
When the expression levels of the indicator sequences is analyzed and the expression of the indicator genes is such that it fits the expression levels of the batches of the train set labeled as 'moderate' (measured using exactly the same method, using the same protocol and using software programs like Predicted Analysis of Microarray), it is very likely that the new tested plant material also will have relative moderate development of firmness loss (drops below critical value of 5 between 15 and 28 days) during post-harvest shelf-life as was found for the batches in quality class 'moderate' of the train set.
When the expression levels of the indicator sequences is analyzed and the expression of the indicator genes is such that it fits the expression levels of the batches of the train set labeled as 'bad' (measured using exactly the same method, using the same protocol and using software programs like Predicted Analysis of Microarray), it is very likely that the new tested plant material also will have relative fast development of firmness loss (drops below critical value of 5 within 15 days) during post-harvest shelf-life as was found for the batches in quality class 'bad' of the train set.

In a preferred embodiment the "minimal set" of indicator mRNAs comprises at least 2, 3, 4, 5, 6, 7, 8, 9, 10, 15, 20 or more mRNAs selected from SEQ ID NO: 136-154 (or variants or fragments thereof). Preferably, a "minimal set" comprises at least one "upregulated" and at least one "down-regulated" transcript, as described above.

As already mentioned, it is understood that also "variants" of SEQ ID NO: 136-154 may be detected in a sample, such as nucleic acid sequences essentially similar to any of SEQ ID NO: 136-154, i.e. comprising at least 70, 75, 80, 85, 90, 95, 98, 99% or more nucleic acid sequence identity to any of SEQ ID NO: 136-154. Such variants may for example be present in different species or different varieties.

The actual method used for determining the level of the set of indicator mRNA transcripts is not important. Any gene expression profiling method may be used, such as RT-PCR, microarrays or chips, Northern blot analysis, cDNA-AFLP, etc. See elsewhere herein. For example, PCR primer pairs for each of SEQ ID NO: 136-154 or variants thereof, optionally also to one or more of SEQ ID NO: 155-157 or variants thereof, may be designed using known methods. In one embodiment of the invention two or more of these primer pairs are used in the method. Alternatively, nucleic acid probes, which hybridize to SEQ ID NO: 136-154, or variants thereof, may be made for use in the detection. Any fragment of at least about 10, 12, 14, 15, 20, 22, 30, 50, 100, 200, 300, 500 or more consecutive nucleotides of SEQ ID NO: 136-154, or the complement strand, or of a variant of SEQ ID NO: 136-154, may be suitable for detection of the full length transcript in a sample. Equally, any fragment of a "variant" of any one of SEQ ID NO: 136-154 (as defined above) may be used.

In one embodiment a carrier is provided comprising nucleic acid molecules selected from SEQ ID NO: 136-154, variants of SEQ ID NO: 136-154 and/or most preferably fragments (oligonucleotides) of any of these or of a subset of any of these. The carrier may, for example, be contacted under hybridizing conditions with the (labeled) nucleic acid sample of the sample of step (a), allowing detection of the level of each of the indicator transcripts present in the sample.

If the expression profile of the indicator mRNAs of the fruit corresponds to the profile of fruit which are prone to rapid firmness loss, the fruit or batch can be identified and selected for further use. Preferably, the fruit or batch of fruit can be selected and removed from non-decaying fruit or batches or treated differently or be destined for shorter post-harvest distribution chains.

In a further embodiment, kits, oligonucleotides (e.g. PCR primers, nucleic acid probes) and antibodies are provided, for determining the firmness loss of harvested fruits. Such kits comprise instructions for use and one or more reagents for use in the method. Optionally, tissue samples or nucleic acid samples suitable as controls may be included. Thus, such a kit may comprise a carrier to receive therein one or more containers, such as tubes or vials. The kit may further comprise unlabeled or labelled oligonucleotide sequences of the invention (SEQ ID NO: 136-154, or variants thereof, or parts thereof, such as degenerate primers or probes), e.g. to be used as primers, probes, which may be contained in one or more of the containers, or present on a carrier. The oligonucleotides may be present in lyophilized form, or in an appropriate buffer. One or more enzymes or reagents for use in isolation of nucleic acids, purification, restriction, ligation and/or amplification reactions may be contained in one or more of the containers. The enzymes or reagents may be present alone or in a mixture, and in lyophilised form or in appropriate buffers. The kit may also contain any other component necessary for carrying out the present invention, such as manuals, buffers, enzymes (such as preferably reverse transcriptase and a thermostable polymerase), pipettes, plates, nucleic acids (preferably labelled probes), nucleoside triphosphates, filter paper, gel materials, transfer materials, electrophoresis materials and visualization materials (preferably dyes, labelled antibodies or -enzymes) autoradiography supplies.

### Preferred embodiments of the present invention

1. A diagnostic method for determining a quality trait stage of a plant, or of a plurality of plants, or of a plant part, or of edible mushrooms, comprises the steps:
   (a) providing a nucleic acid sample of a plant or mushroom, or of a plant or mushroom part, or of a plurality of plants or mushrooms,
   (b) analyzing the nucleic acid sample by determining the level of a set of indicator mRNA transcripts in the sample, which are indicative of a quality trait stage, and optionally
   (c) identifying and selecting the plants or mushrooms, or plant parts or mushroom parts, which comprise a certain expression profile of the indicator mRNA transcripts, and
   (d) separating the plant, plurality of plants, plant parts or mushrooms having different expression profiles of the indicator mRNA transcripts from one another in the further distribution and use thereof.
2. The diagnostic method according to claim 1, wherein the plant is of a family selected from the group consisting of *Fagaceae, Maloideae, Rosoideae, Solanaceae* and wherein the edible mushroom is a basidiomycete.
3. The diagnostic method according to claim 1 or 2, wherein the quality trait is one of the group consisting of cold tolerance of tree seedlings, ripening stage of fruit, sensory decay of fruit, *Botrytis* incidence of roses, discoloration development in mushrooms, firmness development in tomato.
4. The diagnostic method according to any one of claims 1-3, wherein batches of plants or plant parts are separated in step (d) and wherein for each batch a nucleic acid sample comprising nucleic acids obtained from at least 5 different plants of the batch is analysed in steps (a) and (b).
5. The diagnostic method according to any one of claims 1-4, wherein the nucleic acid sample of step (a) is obtained from tissue homogenate present on FTA® cards.
6. The diagnostic method according to any one of claims 1-3, wherein said set of indicator mRNA transcripts comprises one of the following sets:
   (a) SEQ ID NO: 77-109 and SEQ ID NO: 172-174, or nucleic acid sequences comprising at least 70% sequence identity over the entire lengths to the sequences of SEQ ID NO: 77-109 or SEQ ID NO: 172-174;
   (b) SEQ ID NO: 42-46 and SEQ ID NO: 158-171, or nucleic acid sequences comprising at least 70% sequence identity over the entire lengths to the sequences of SEQ ID NO: 42-46 or SEQ ID NO: 158-171;
   (c) SEQ ID NO: 113-135, or nucleic acid sequences comprising at least 70% sequence identity over the entire lengths to the sequences of SEQ ID NO: 113-135;
   (d) SEQ ID NO: 136-154, or a nucleic acid sequence comprising at least 70% sequence identity over the entire length to the sequences of SEQ ID NO: 136-154;
   (e) SEQ ID NO: 1-29, or nucleic acid sequences comprising at least 70% sequence identity over the entire lengths to the sequences of SEQ ID NO: 1-29;
   (f) SEQ ID NO: 57-76, or nucleic acid sequences comprising at least 70% sequence identity over the entire lengths to the sequences of SEQ ID NO: 57-76;
      and whereby the level of at least 3 different indicator mRNA transcripts of any one of the sets (a)-(f) in said nucleic acid sample are determined.
7. The method according to claim 6, whereby the level of at least 4, preferably at least 5, more preferably of each of the different indicator mRNAs of a set is determined.
8. Use of at least 3 sequences, or fragments thereof, of any one of the following sets (a)-(f):
   (a) SEQ ID NO: 77-109 and SEQ ID NO: 172-174, or nucleic acid sequences comprising at least 70% sequence identity over the entire lengths to the sequences of SEQ ID NO: 77-109 or SEQ ID NO: 172-174;
   (b) SEQ ID NO: 42-46 and SEQ ID NO: 158-171, or nucleic acid sequences comprising at least 70% sequence identity over the entire lengths to the sequences of SEQ ID NO: 42-46 or SEQ ID NO: 158-171;
   (c) SEQ ID NO: 113-135, or nucleic acid sequences comprising at least 70% sequence identity over the entire lengths to the sequences of SEQ ID NO: 113-135;
   (d) SEQ ID NO: 136-154, or a nucleic acid sequence comprising at least 70% sequence identity over the entire length to the sequences of SEQ ID NO: 136-154;
   (e) SEQ ID NO: 1-29, or nucleic acid sequences comprising at least 70% sequence identity over the entire lengths to the sequences of SEQ ID NO: 1-29;
   (f) SEQ ID NO: 57-76, or nucleic acid sequences comprising at least 70% sequence identity over the entire lengths to the sequences of SEQ ID NO: 57-76;
      for the determination of a quality trait stage of a plant tissue sample, said quality trait being one of the group consisting of (a) ripening stage of fruit, (b) *Botrytis* incidence of roses, (c) discoloration development in mushrooms or (d) firmness development in tomato, (e) cold tolerance of tree seedlings, (f) sensory decay of fruit, respectively.
9. A solid carrier comprising at least 3, preferably at least 4 or 5, nucleic acid molecules attached to said carrier, said molecules being capable of hybridizing to at least 3, preferably at least 4 or 5, nucleic acid molecules of any one of the following sets (a)-(f):
   (a) SEQ ID NO: 77-109 and SEQ ID NO: 172-174, or nucleic acid sequences comprising at least 70% sequence identity over the entire lengths to the sequences of SEQ ID NO: 77-109 or SEQ ID NO: 172-174;
   (b) SEQ ID NO: 42-46 and SEQ ID NO: 158-171, or nucleic acid sequences comprising at least 70% sequence identity over the entire lengths to the sequences of SEQ ID NO: 42-46 or SEQ ID NO: 158-171;
   (c) SEQ ID NO: 113-135, or nucleic acid sequences comprising at least 70% sequence identity over the entire lengths to the sequences of SEQ ID NO: 113-135;
   (d) SEQ ID NO: 136-154, or a nucleic acid sequence comprising at least 70% sequence identity over the entire length to the sequences of SEQ ID NO: 136-154;
   (e) SEQ ID NO: 1-29, or nucleic acid sequences comprising at least 70% sequence identity over the entire lengths to the sequences of SEQ ID NO: 1-29;
   (f) SEQ ID NO: 57-76, or nucleic acid sequences comprising at least 70% sequence identity over the entire lengths to the sequences of SEQ ID NO: 57-76.
10. The carrier according to claim 9, wherein the carrier is made of glass, plastic, nitrocellulose, nylon or silicon.
11. A kit for determining the quality trait stage of a plant or mushroom tissue sample, said kit comprising nucleic acid probes or primers capable of detecting the presence and/or quantity of at least 3, preferably of at least 4 or 5, nucleic acid molecules within a set of nucleic acid molecules, said set being selected from the group consisting of:
   (a) SEQ ID NO: 77-109 and SEQ ID NO: 172-174, or nucleic acid sequences comprising at least 70% sequence identity over the entire lengths to the sequences of SEQ ID NO: 77-109 or SEQ ID NO: 172-174;
   (b) SEQ ID NO: 42-46 and SEQ ID NO: 158-171, or nucleic acid sequences comprising at least 70% sequence identity over the entire lengths to the sequences of SEQ ID NO: 42-46 or SEQ ID NO: 158-171;
   (c) SEQ ID NO: 113-135, or nucleic acid sequences comprising at least 70% sequence identity over the entire lengths to the sequences of SEQ ID NO: 113-135;
   (d) SEQ ID NO: 136-154, or a nucleic acid sequence comprising at least 70% sequence identity over the entire length to the sequences of SEQ ID NO: 136-154;
   (e) SEQ ID NO: 1-29, or nucleic acid sequences comprising at least 70% sequence identity over the entire lengths to the sequences of SEQ ID NO: 1-29;
   (f) SEQ ID NO: 57-76, or nucleic acid sequences comprising at least 70% sequence identity over the entire lengths to the sequences of SEQ ID NO: 57-76.
12. The kit according to claim 11, further comprising one or more of the following: instructions for use, control samples, control data, labeling reagents, detection reagents, hybridization or amplification reagents, primers or probes for detecting housekeeping-gene transcripts, containers or carriers, preferably a carrier according to claim 9 or 10.
13. The kit according to claim 10 or 11, further comprising material for sampling plant or mushroom tissue, such as FTA® cards.

### Figure Legends

Figure 1.1: Frost tolerance determined by electrolyte leakage (see Example 1)
Figure 1.2 A and B: indicator gene expression (see Example 1)
Figure 2.1: Firmness of pears from two orchards harvested at two days intervals in September 2003.
Figure 2.2: Relative expression levels (normalized against the actin gene) of selected genes measured during a part of the period described in Figure 2.1 and in one orchard.
Figure 2.3: Expression level of three indicator genes in pears, namely ACC oxidase (SEQ ID NO: 43), Galactosidase (SEQ ID NO: 163) and SAM synthase (SEQ ID NO: 42), as well as firmness levels (kg/cm²) and starch levels (using a color chart with a scale of 1-10).
Figure 3.1: PCA plot of sensory characteristics of batches of apples stored at two different temperatures for various periods. Data from two trials are displayed (FI and FII).
Figure 3.2: Firmness data from trial FII. Significant differences between apples stored at 4 and 18°C can only be measured after two weeks of storage
Figure 3.3: PCA plot of the various batches of apples based on results from expression analysis of 900 genes.
Figure 4.1: Clearness, whiteness, %browning and diameter of the white button mushrooms selected for microarray analysis.
Figure 4.2: Hierarchical clustering of the selected genes and physiological post-harvest data (whiteness, lightness and browning day 7).
Figure 4.3: Pedigree of Homobasidiomycete, to which *Agaricus bisporus* belongs.
Figure 5.1: Biological variation in shelf life in batches tomato fruit from cultivar Aromata harvested in April, June, August and September 2003, April and September 2004 and May 2005. At each harvest date tomatoes were obtained from various growers. Shelf life period is defined as the number of days needed before the average firmness value for the batch drops below the arbitrary critical value of 5. This is the value below which batches can no longer be sold to retailers.
Figure 5.2: Typical post-harvest development of firmness (diamonds) and colour squares) over time. Firmness and colour are indicated with arbitrary units that refer to standards used in Dutch practice.
Figure 5.3:
   Predictive analysis of tomato samples. For each sample the firmness development was determined and they were classified as having low (triangle), average (square) or good (diamond) accordingly. Subsequently gene expression profiles of the same samples taken at the start of the shelf life period, when all samples had equivalent firmness,
   were used to perform a prediction of quality. The figure shows that most samples are placed in the right class. When a yellow triangle is placed at 0.9 height in the right section of the graph, this should be interpreted as a likelihood of 95% that the sample has low quality.

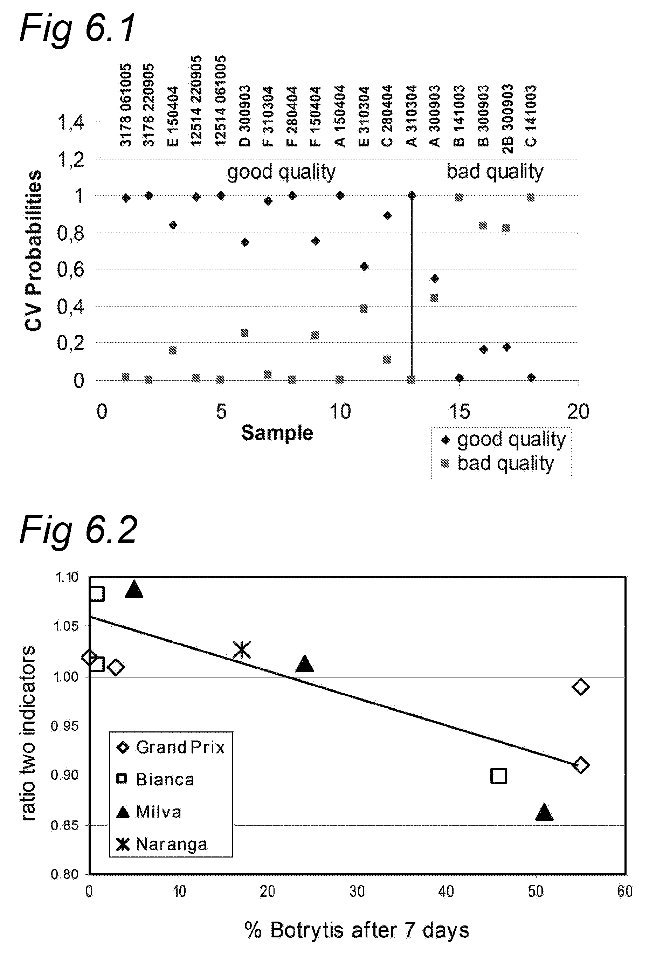

Figure 6.1: PAM analysis of 18 different batches of Rose, classified good (percentage of flowers with visable *Botrytis* infection after 7 days vaselife lower than 20%) and bad. Prediction based on expression data of 22 genes (indicated in Table 8) shows that in most cases cross validation gives a reliable result (probability higher than 0.8).
Figure 6.2: Expression ratio of ORoseR0626/ORoseR0277 (SEQ ID NO: 106 to SEQ ID NO: 102 mRNA ratio) in batches of Rose from four different cultivars that develop various degrees of visable Botrytis infection after 7 days vaselife.

### SEQUENCES

SEQ ID NO 1-29: upregulated, downregulated and constant beech seedling sequences;
SEQ ID NO 30-41: PCR primer pairs for amplification of:
   - transcript SEQ ID NO: 9 (primers of SEQ ID NO: 30 and 31);
   - transcript SEQ ID NO: 11 (primers of SEQ ID NO: 32 and 33);
   - transcript SEQ ID NO: 1 (primers of SEQ ID NO: 34 and 35);
   - transcript SEQ ID NO: 20 (primers of SEQ ID NO: 36 and 37);
   - transcript SEQ ID NO: 24 (primers of SEQ ID NO: 38 and 39);
   - transcript SEQ ID NO: 28 (primers of SEQ IDNO: 40 and 41);
SEQ ID NO 42-46: indicator-gene transcripts of pears;
SEQ ID NO 47-56: PCR primer pairs for amplification of pear indicator transcripts;
SEQ ID NO 57-76: upregulated and downregulated apple mRNA sequences;
SEQ ID NO 77-109: Rose indicator mRNA sequences;
SEQ ID NO 110-112: Rose housekeeping mRNA sequences;
SEQ ID NO 113-135: Agaricus indicator mRNA sequences. SEQ ID NO: 120-135 contain putative SSH linker sequences at the 5' end (indicated), which are preferably removed prior to sequence alignments or for detection purposes.
SEQ ID NO 136-154: Solanaceae indicator mRNA sequences from tomato. SEQ ID NO: 136-144 are downregulated in low quality batches, while SEQ ID NO: 145-154 are upregulated in low quality batches.
SEQ ID NO 155-157: additional Solanaceae indicator mRNA sequences, comprising a constant mRNA expression level during post-harvest storage.
SEQ ID NO: 158-171: additional pear indicator mRNA sequences.
SEQ ID NO: 172-174 additional rose indicator mRNA sequences

### EXAMPLES

### Example 1 - quality assay for determining cold tolerance in Fagaceae, exemplified by Fagus sylvatica L. seedlings (beech)

### 1.1 Indicator genes

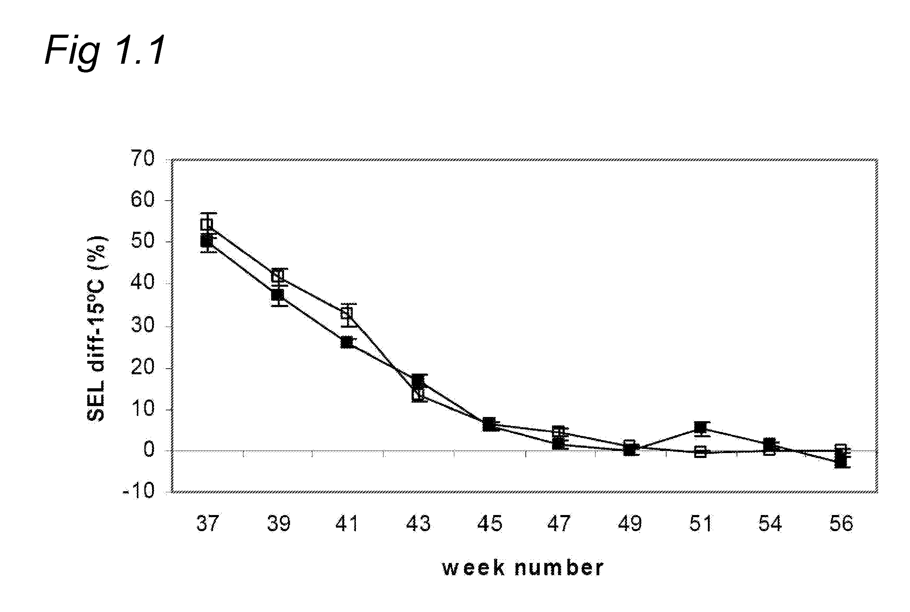

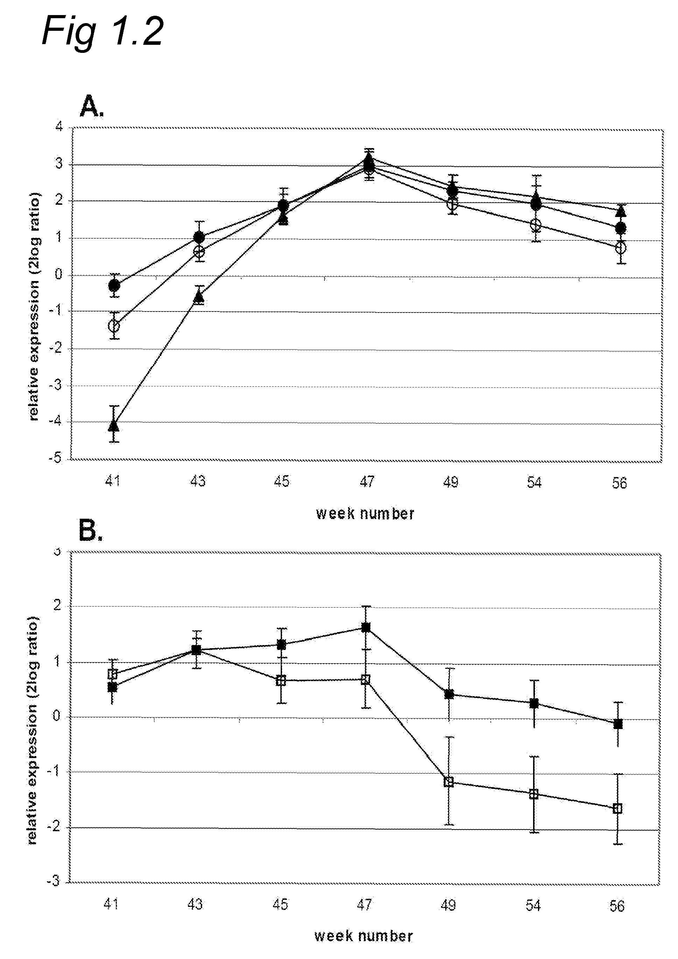

A set of 29 indicator genes (SEQ ID NO: 1-29) have been selected whose expression profile can be used as measurement for cold tolerance level of beech seedlings. Based on the expression level of the described genes conclusions can be drawn about the level of frost tolerance that is reached in beech seedlings. As soon as the expression of the frost tolerance related genes stabilises at high levels, frost tolerance has reached the maximal level (Figure 1.1 and 1.2).

Figure 1.1. shows typical frost tolerance pattern of two batches of one-year-old beech seedlings, planted at two different locations (Scotland, black squares and Denmark, open squares), season 2001/2002. Tolerance is defined as percentage of electrolyte leakage (SEL) as a result of freezing until -15°C. When SEL diff-values fall below 10%, seedlings are considered to be completely frost tolerant. In this case seedlings are frost tolerant from week 45 on.

Figure 1.2. shows the expression patterns of groups of indicator genes selected after hybrisations using a microarray. Groups consist of genes that showed the same expression patterns in both batches described in Figure 1.1. Selected indicator sequences are derived from both groups; upregulated genes (A.) and downregulated genes (B.).

Using indicator genes and a proper test setup, results can be generated within one day.

Table 1 and Table 2 show the indicator gene expression data. Data with the code 704 is 1 year old beech seedling plant material from a field in Scotland (2001/2002). Data with the code 406 is 1 year old plant material from a field in Denmark (2001/2002). WK indicates the week.

**Table 1**

| Gene | 704wk37 | 740wk41 | 704wk43 | **704wk45** | **704wk47** | **704wk51** | 704wk04 |
|---|---|---|---|---|---|---|---|
| Frost tolerance upregulated genes (SEQ ID NO: 1-15) | | | | | | | |
| | | | | | | | |
| b1nr013 | -0,742 | -1,0665 | 0,62 | **1,6715** | **1,287** | **1,1155** | -0,0135 |
| b1nr031 | -0,3855 | -1,4625 | -0,6785 | **0,5275** | **0,9095** | **0,601** | -0,421 |
| b1nr039 | -1,667 | -1,004 | 1,0465 | **1,7605** | **1,8405** | **0,5895** | 0,332 |
| b4nr049 | -1,595 | -0,909 | 0,9385 | **1,7255** | **1,597** | **0,5985** | 0,261 |
| b4nr081 | 0,685 | 0,119 | 1,196 | **2,598** | **1,953** | **1,817** | 1,065 |
| b4nr096 | 0,614 | 0,0415 | 1,274 | **2,6205** | **1,8345** | **1,9235** | 1,3495 |
| b5nr012 | 0,124 | -0,4045 | 0,964 | **1,8355** | **1,2145** | **1,0425** | 1,0295 |
| b5nr018 | -1,6505 | -1,276 | -0,119 | **1,3115** | **1,562** | **1,487** | 0,2135 |
| b5nr019 | -3,446 | -3,0855 | -0,184 | **1,608** | **1,98** | **2,0435** | 0,817 |
| b5nr052 | -0,5365 | -0,57 | 0,5625 | **1,423** | **1,233** | **1,363** | 0,6995 |
| b5nr078 | 0,15375 | 0,98125 | 1,862 | **1,9135** | **1,662** | **1,65** | 0,71425 |
| b6nr008 | -1,675 | -1,1715 | 0,7695 | **1,548** | **1,654** | **0,4545** | 0,123 |
| b6nr046 | -0,5985 | -0,022 | 0,7945 | **1,9615** | **1,8125** | **2,0745** | 0,7005 |
| b6nr057 | -0,411 | -0,046 | 0,308 | **2,114** | **1,749** | **2,441** | 1,6495 |
| b6nr061 | -0,1815 | 0,1395 | 0,486 | **1,8925** | **1,803** | **2,1965** | 1,255 |
| Frost tolerance downregulated genes (SEQ ID NO: 16-27) | | | | | | | |
| | | | | | | | |
| b1nr005 | 0,9655 | 2,5035 | 1,0975 | **0,184** | **-1,7615** | **-1,613** | -1,671 |
| b1nr019 | 0,5855 | 1,247 | 0,476 | **0,08** | **-0,2005** | **-0,384** | -1,0045 |
| b1nr025 | 1,072 | 1,837 | 0,63 | **0,2925** | **-1,241** | **-1,138** | 0,4295 |
| b1nr082 | 0,7775 | 0,7205 | 0,132 | **-0,308** | **-0,2205** | **-0,9535** | -0,742 |
| b2nr070 | 0,922 | 1,3905 | 0,9605 | **0,8755** | **0,2645** | **0,173** | -0,2945 |
| b2nr074 | 1,114 | 1,1255 | 0,9965 | **0,7465** | **0,0885** | **-0,3255** | -0,293 |
| b3nr031 | 0,621 | 0,6195 | 0,563 | **-0,7715** | **-1,695** | **-2,3195** | -1,1815 |
| b3nr056 | 0,7615 | 0,567 | 0,424 | **0,218** | **-0,1285** | **-0,8065** | -0,6275 |
| b3nr058 | 0,8255 | 1,15275 | 0,87925 | **0,365** | **-0,5085** | **-0,79375** | -0,6185 |
| b3nr083 | 1,319 | 1,0715 | 0,8255 | **0,948** | **0,427** | **0,4205** | 0,425 |
| b3nr095 | 0,99175 | 0,71275 | 0,67825 | **0,645** | **0,28625** | **0,097** | 0,2415 |
| b6nr003 | 0,8605 | 0,683 | 0,686 | **1,13** | **0,4305** | **0,4605** | -0,2845 |
| Genes with stable expression (SEQ ID NO: 28 and 29) | | | | | | | |
| | | | | | | | |
| b3nr038 | 0,698 | 0,078 | -0,268 | **-0,3045** | **-0,202** | **-0,296** | -0,644 |
| b4nr068 | 1,7015 | 1,719 | 1,5975 | **1,861** | **1,268** | **0,9425** | 1,2175 |

**Table 2**

| Clone | 406wk41 | 406wk43 | **406wk45** | **406wk47** | **406wk49** | 406wk02 | 406wk04 |
|---|---|---|---|---|---|---|---|
| Frost tolerance upregulated genes (SEQ ID NO: 1-15) | | | | | | | |
| b1nr013 | -1,635 | 0,6145 | **1,7145** | **2,871** | **1,836** | 1,235 | 0,4545 |
| b1nr031 | -2,133 | 0,575 | **1,506** | **2,6795** | **2,3185** | 1,289 | 0,671 |
| b1nr039 | -1,64 | 0,8285 | **2,264** | **3,0345** | **2,181** | 1,2005 | 0,655 |
| b4nr049 | -1,369 | 0,762 | **2,074** | **2,7605** | **1,7175** | 0,829 | 0,429 |
| b4nr081 | -0,475 | 1,292 | **2,3985** | **3,267** | **2,533** | 1,9915 | 1,1565 |
| b4nr096 | -0,5885 | 1,198 | **2,4515** | **3,3535** | **2,61** | 2,089 | 1,1585 |
| b5nr012 | -1,3065 | 0,71 | **2,2365** | **3,1315** | **1,9625** | 2,17 | 0,9345 |
| b5nr018 | -2,0685 | -0,162 | **1,585** | **2,969** | **2,204** | 1,9435 | 1,5025 |
| b5nr019 | -3,786 | -0,381 | **1,753** | **3,338** | **2,658** | 2,4595 | 1,9485 |
| b5nr052 | -0,764 | 0,739 | **1,7565** | **2,709** | **1,9** | 1,7235 | 1,156 |
| b5nr078 | 0,2 | 1,44975 | **2,1715** | **3,18825** | **1,87475** | 0,59525 | 1,096 |
| b6nr008 | -1,4095 | 0,7345 | **2,234** | **2,8925** | **1,906** | 1 | 0,633 |
| b6nr046 | -0,752 | 0,636 | **1,568** | **2,896** | **2,0885** | 2,2565 | 1,3965 |
| b6nr057 | -0,5665 | 0,513 | **1,313** | **2,6415** | **2,703** | 2,8945 | 1,9005 |
| b6nr061 | -0,4095 | 0,656 | **1,5** | **2,628** | **2,486** | 2,697 | 1,7525 |
| Frost tolerance downregulated genes (SEQ ID NO: 16-27) | | | | | | | |
| b1nr005 | 0,971 | 0,9845 | **0,236** | **-0,389** | **-3,047** | -2,796 | -2,786 |
| b1nr019 | 0,567 | 1,1315 | **0,6415** | **0,93** | **-0,491** | -0,938 | -1,123 |
| b1nr025 | 1,3225 | 1,5505 | **0,749** | **0,856** | **-0,837** | -1,0685 | -0,9395 |
| b1nr082 | 0,7995 | 1,2045 | **0,525** | **0,56** | **-0,684** | -1,393 | -1,452 |
| b2nr070 | 0,587 | 1,0985 | **1,4655** | **1,4875** | **0,083** | 0,031 | -0,5615 |
| b2nr074 | 0,591 | 1,0015 | **1,278** | **1,4895** | **-0,052** | -0,041 | -0,512 |
| b3nr031 | 0,6235 | 1,2575 | **-0,008** | **0,104** | **-2,7185** | -2,8945 | -3,132 |
| b3nr056 | 1,0265 | 1,042 | **0,899** | **0,8475** | **-0,729** | -1,237 | -1,4325 |
| b3nr058 | 0,63725 | 1,2795 | **0,96925** | **1,0065** | **-0,88975** | -1,13425 | -1,3605 |
| b3nr083 | 0,474 | 1,2695 | **1,3035** | **1,457** | **0,2995** | 0,391 | -0,259 |
| b3nr095 | 0,99433 3333 | 1,356333 333 | **1,1205** | **1,2215** | **-0,0895** | -0,024 | -0,463 |
| b6nr003 | -0,154 | 1,043 | **1,42** | **2,113** | **1,1115** | 0,5065 | 0,065 |
| Genes with stable expression (SEQ ID NO: 28 and 29) | | | | | | | |
| b3nr038 | 0,6465 | 1,2055 | **0,8485** | **1,3285** | **0,449** | 0,463 | -0,022 |
| b4nr068 | 0,6065 | 1,4755 | **1,5845** | **2,119** | **0,7945** | 0,8845 | 0,2935 |

Some of the selected indicator genes have sequence homology to known sequences, as indicated in Table 3.

| Frost tolerance upregulated gene sequences | | | | |
|---|---|---|---|---|
| SEQ ID 1 | b1nr013 | Dehydrin | Prunus persica | 8,00E-11 |
| SEQ ID 2 | b1nr031 | Embryonic abundant protein AtEm1 | Arabidopsis | 5,00E-07 |
| SEQ ID 3 | b1nr039 | Unknown | | |
| SEQ ID 4 | b4nr049 | Unknown | | 3,2 |
| SEQ ID 5 | b4nr081 | Unknown | | 4,6 |
| SEQ ID 6 | b4nr096 | Unknown | | 0 |
| SEQ ID 7 | b5nr012 | embryonic abundant protein, 59K - soybean | unknown origin | 7,00E-12 |
| SEQ ID 8 | b5nr018 | protein kinase family | [Arabidopsis thaliana] | 7,00E-27 |
| SEQ ID 9 | b5nr019 | ABA-inducible protein | [Fagus sylvatica] | 6,00E-32 |
| SEQ ID 10 | b5nr052 | PRL1 associated protein -related | [Arabidopsis thaliana] | 3,00E-39 |
| SEQ ID 11 | b5nr078 | LTCOR11 | [Lavatera thuringiaca] | 1,00E-24 |
| SEQ ID 12 | b6nr008 | Unknown | | 0,55 |
| SEQ ID 13 | b6nr046 | Unknown | | 0,024 |
| SEQ ID 14 | b6nr057 | early light-induced protein | [Arabidopsis thaliana] | 4,00E-32 |
| SEQ ID 15 | b6nr061 | probable light induced protein -Arabidopsis thaliana | [Arabidopsis thaliana] | 2,00E-09 |
| Frost tolerance downregulated gene sequences | | | | |
| SEQ ID 16 | b1nr005 | GDSL-motif lipase/hydrolase protein | Arabidopsis | 2,00E-47 |
| SEQ ID 17 | b1nr019 | arabinogalactan protein | Gossypium hirsutum | 2,00E-37 |
| SEQ ID 18 | b1nr025 | Unknown | Arabidopsis | 4,00E-23 |
| SEQ ID 19 | b1nr082 | allergenic isoflavone reductase-like protein | Betula pendula | 3,00E-83 |
| SEQ ID 20 | b2nr070 | Unknown | | 0,029 |
| SEQ ID 21 | b2nr074 | Unknown | | 0,042 |
| SEQ ID 22 | b3nr031 | Unknown | | 0 |
| SEQ ID 23 | b3nr056 | expansin- related | [Arabidopsis thaliana] | 4,00E-55 |
| SEQ ID 24 | b3nr058 | alpha-tubulin | [Gossypium hirsutum] | 1,00E-62 |
| SEQ ID 25 | b3nr083 | Unknown | | 0,047 |
| SEQ ID 26 | b3nr095 | beta tubulin | [Arabidopsis thaliana] | 6,00E-67 |
| SEQ ID 27 | b6nr003 | Unknown | | 0,32 |
| Constant | | | | |
| SEQ ID 28 | b3nr038 | protein kinase, putative | [Arabidopsis thaliana] | 4,00E-44 |
| SEQ ID 29 | b4nr068 | Unknown | | 0 |

### 1.2 Material and Methods

Expression levels can be determined in buds of tree seedlings using RT-PCR, or microarrays (described below) or any other gene expression profiling format. Results are most reliable when samples are related to a cold-sensitive sample taken in early autumn.

### 1.2.1 On site, robust sampling

Use about 10-20 mg of plant tissue for the homogenate. Add 5 parts double distilled water to the tissue. Grind until it is apparent that some plant tissue is homogenized. The homogenate does not have to have a smooth consistency. Apply 25 microliter of plant homogenate to each circle on an FTA card (Whatman). Allow plant homogenate on FTA to dry for at least one hour at room temperature. Do not heat assist the drying period. Archive the sample in a dessicated environment.

### 1.2.2 RNA isolation from plant homogenate on FTA Cards

Take a sample disc from the dried spot using and place it in an Eppendorf vial. Add 400 microliter RNA processing buffer (10 mM Tris-HCl, pH 8.0, 0.1 mM EDTA, 1 microliter RNAse inhibitor, 200 microgram/ml glycogen and 2 mM DTT, freshly prepared).

Mix and incubate on ice for 15 minutes (mix every five minutes). Remove the disc. Precipitate the RNA with 1/10th volume of 3M sodium acetate pH 5.2 and two volumes of ice cold 100% isopropanol. Incubate for 1 hour ad -20 C. Spin down the RNA at top speed in an eppendorf centrifuge. Wash the pellet with 75% ethanol. Air dry the pellet. Resuspend the pellet in a suitable volume of double distilled water. Use DNA free (AMBION) for removal of traces of DNA following the protocol of the manufacturer. After that, the RNA preparation can be directly used for cDNA synthesis and subsequent PCR.

### 1.2.3 Microarray hybridisation

Total RNA, up till 20 microgram, purified with RNeasy (Qiagen, The Netherlands) and complemented with 1 nanogram luciferase polyA mRNA was used for each individual labeling. Reference RNA was labeled with Cy3 and sample RNA with Cy5 using the CyScribe First-Strand cDNA Labeling Kit (Amersham Biosciences). After checking the integrity of the labeled cDNA using agarose electrophoresis, sample and reference cDNA were mixed and used for hybidisation of the microarray following the protocol supplied by the manufacturer of the slides. Cover slides and hybridisation chambers from Agilent Technologies (Palo Alto) were used. Hybridisation was allowed to continue overnight in an incubator where the slides were continuously rotating (Sheldon Manufacturing). Post hybridisation washes were according to the Nexterion protocol.

### 1.2.4 RT-PCR

Total RNA was isolated according to the protocol described above. Preparations were DNAseI (AP Biotech) treated and purified using RNeasy (Qiagen, The Netherlands). Half a microgram of pure total RNA was used for cDNA synthesis using Anchored Oligo(dT)23 (SIGMA, The Netherlands) and M-MLV Reverse Transcriptase (Invitrogen, Life Technologies). Dilutions of this cDNA were used for Realtime PCR using the qPCR Mastermix Plus for SYBR GreenI (Eurogentec, Belgium). Product formation was measured using the iCycler system (BIORAD Laboratories, The Netherlands). Primer sets are described in SEQ ID NO: 30-41. The signal obtained from the same batch of cDNA using primers homologous to Arabidopsis thaliana 18S rRNA was taken as a reference for normalisation. Relative changes in expression were calculated using the Gene Expression Macro (Version 1.1) supplied by BIORAD.

### Example 2 - A method for determining the ripening stage of pears, exemplified by Pyrus communis L. cv Conference

### 2.1 Indicator genes

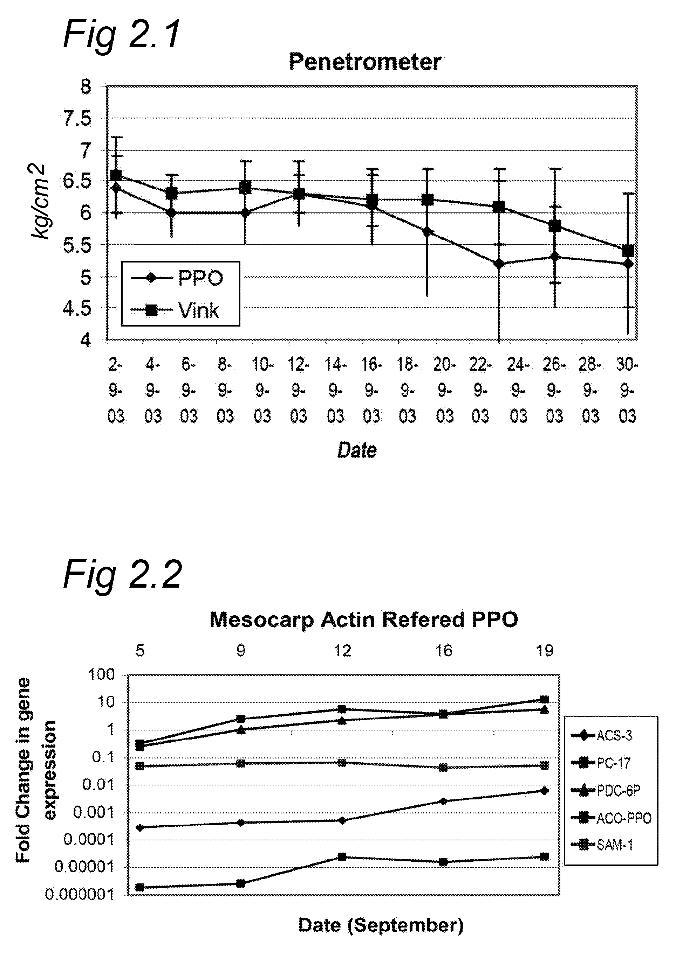

Comparison of expression levels of a set of 5 genes, SEQ ID NO: 42-46, in various batches of pears gives information about relative ripening stages. This method is much more informative than firmness measurements (Figure 2.1 and 2.2). Discrimination between batches is possible in cases where firmness measurements fail. The data in Fig 2.1 and 2.2 show that during the test period the firmness hardly changes but expression of all genes, except SAM-1, increased 10 to 100 fold. The test can also be used to check the effect of storage conditions on the produce.

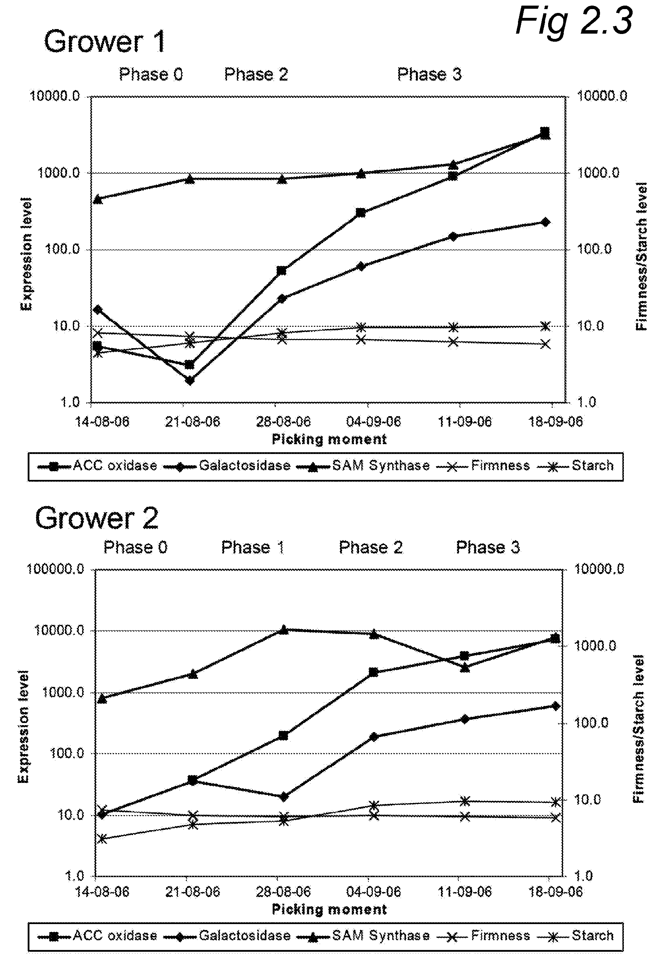

Figure 2.3. shows a result of the validation of the test in practice. Based on the expressiondata of the indicated genes, ripening phases can be defined. This typical example shows two orchards, from two different growers, which exhibit clear differences in ripening up until 11^{th} of September. These differences in ripening behaviour directly influences optimal picking date but may also have an effect on storage behaviour.

Expression data of the indicator genes is shown in Table 4, below, and in Figure 2.2.

**Table 4**

| Date | ACS-3 | PC-17 | PDC-6P | ACO-PPO | SAM-1 |
|---|---|---|---|---|---|
| Sept. | (SEQ ID 44) | (SEQ ID 46) | (SEQ ID 45) | (SEQ ID 43) | (SEQ ID 42) |
| 5 | 0,000281151 | 1,86E-06 | 0,247823794 | 0,321849928 | 0,047935697 |
| 9 | 0,000436517 | 2,58E-06 | 1,038177613 | 2,531683248 | 0,0596776 |
| 12 | 0,000523976 | 2,38E-05 | 2,220848325 | 5,763191619 | 0,064704058 |
| 16 | 0,002586697 | 1,58E-05 | 3,745937856 | 3,938307235 | 0,042266044 |
| 19 | 0,006349136 | 2,46E-05 | 5,587947537 | 12,75888406 | 0,051376208 |

Table 5 shows putative homology of indicator genes to known genes

| Sequence ID | Homology to |
|---|---|
| SEQ ID 42 | SAM synthase 1 |
| SEQ ID 43 | ACC oxidase (ACO-2) |
| SEQ ID 44 | ACC synthase (ACS3-4) |
| SEQ ID 45 | Pyruvate decarboxylase PDC-6P |
| SEQ ID 46 | (PC17) No significant homology |
| SEQ ID NO 158 | ACS1-6 |
| SEQ ID NO 159 | ACS2 (old name ACS3-6) |
| SEQ ID NO 160 | ACS6 (old name ACS4-8M) |
| SEQ ID NO 161 | ACS5-4 |
| SEQ ID NO 162 | GAPDH-7 |
| SEQ ID NO 163 | beta-galactosidase (AJ811694) |
| SEQ ID NO 164 | Polygalacturonase 1 (AJ504855.2) |
| SEQ ID NO 165 | Polygalacturonase 2 (AJ811693.1) |
| SEQ ID NO 166 | Actin (AF386514.1) |
| SEQ ID NO 167 | Beta xylosidase (AJ811690) |
| SEQ ID NO 168 | Expansin 2 (AB093029) |
| SEQ ID NO 169 | Expansin 3 (AB093030) |
| SEQ ID NO 170 | Expansin 5 (AB093032) |
| SEQ ID NO 171 | Expansin 6 (AB093033) |

### 2.2 Material and Methods

Expression levels can be determined in mesocarp of pear fruit using RT-PCR, or microarrays (described below) or any other gene expression profiling format. Results are most reliable when samples are related to an unripe sample taken well before harvest time.

### 2.2.1 On site, robust sampling

Use about 10-20 mg of plant tissue for the homogenate. Add 5 parts double distilled water to the tissue. Grind until it is apparent that some plant tissue is homogenized. The homogenate does not have to have a smooth consistency. Apply 25 microliter of plant homogenate to each circle on an FTA card (Whatman). Allow plant homogenate on FTA to dry for at least one hour at room temperature. Do not heat assist the drying period. Archive the sample in a dessicated environment.

### 2.2.2 RNA isolation from plant homogenate on FTA Cards

Take a sample disc from the dried spot using and place it in an Eppendorf vial Add 400 microliter RNA processing buffer (10 mM Tris-HCl, pH 8.0, 0.1 mM EDTA, 1 microliter RNAse inhibitor, 200 microgram/ml glycogen and 2 mM DTT, freshly prepared).

Mix and incubate on ice for 15 minutes (mix every five minutes). Remove the disc.

Precipitate the RNA with 1/10th volume of 3M sodium acetate pH 5.2 and two volumes of ice cold 100% isopropanol. Incubate for 1 hour ad -20 C. Spin down the RNA at top speed in an eppendorf centrifuge. Wash the pellet with 75% ethanol. Air dry the pellet. Resuspend the pellet in a suitable volume of double distilled water. Use DNA free (AMBION) for removal of traces of DNA following the protocol of the manufacturer. After that, the RNA preparation can be directly used for cDNA synthesis and subsequent PCR.

### 2.2.3 Microarray hybridisation

Total RNA, up to 20 microgram, purified with RNeasy (Qiagen, The Netherlands) and complemented with 1 nanogram luciferase polyA mRNA was used for each individual labeling. Reference RNA was labeled with Cy3 and sample RNA with Cy5 using the CyScribe First-Strand cDNA Labeling Kit (Amersham Biosciences). After checking the integrity of the labeled cDNA using agarose electrophoresis, sample and reference cDNA were mixed and used for hybidisation of the microarray following the protocol supplied by the manufacturer of the slides. Cover slides and hybridisation chambers from Agilent Technologies (Palo Alto) were used. Hybridisation was allowed to continue overnight in an incubator where the slides were continuously rotating (Sheldon Manufacturing). Post hybridisation washes were according to the Nexterion protocol.

### 2.2.4 RT-PCR

Total RNA was isolated according to the protocol described above. Preparations were DNAseI (AP Biotech) treated and purified using RNeasy (Qiagen, The Netherlands). Half a microgram of pure total RNA was used for cDNA synthesis using Anchored Oligo(dT)23 (SIGMA, The Netherlands) and M-MLV Reverse Transcriptase (Invitrogen, Life Technologies). Dilutions of this cDNA were used for Realtime PCR using the qPCR Mastermix Plus for SYBR Greenl (Eurogentec, Belgium). Product formation was measured using the iCycler system (BIORAD Laboratories, The Netherlands). Primer sets are described in SEQ ID NO: 47-56. The signal obtained from the same batch of cDNA using primers homologous to Arabidopsis thaliana 18S rRNA was taken as a reference for normalisation. Relative changes in expression were calculated using the Gene Expression Macro (Version 1.1) supplied by BIORAD.

### Example 3 - A sensitive method for measuring sensory decay of fruit, exemplified by apples

### 3.1 Indicator genes

In an experimental approach in which transcriptional profiling (using microarrays) was combined with sensory analysis and physiological measurements a set of 20 genes was selected (SEQ ID NO: 57-76) that can be used for early warning of quality decay. In the experiments quality loss was induced by storage at a temperature of 18°C, whereas normal storage temperature is 4°C.

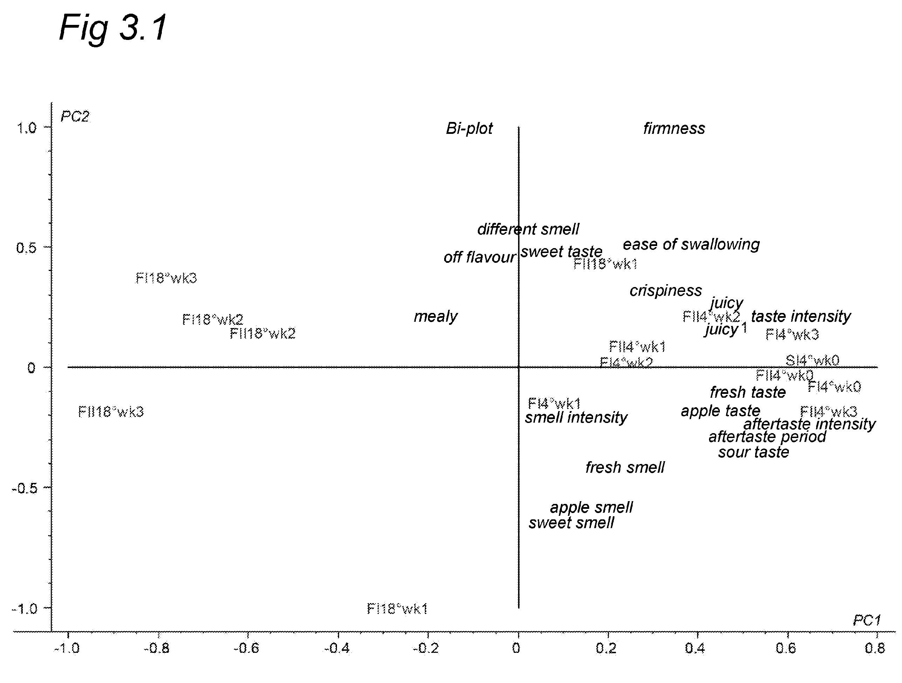

Relative expression levels of the selected genes can be used to determine whether a batch of apples is approaching a status of quality loss. Analyzing the genes provides better insight in quality loss than sensory analysis or firmness measurements. Two batches of apples were tested with the three different methods mentioned. Figure 3.1 shows the result of the sensory analysis. From this plot it becomes clear that after one week storage at suboptimal temperature (18°C) differences with apples stored at optimal temperature (4°C) are hard to establish. Only after two week storage differences between the two storage conditions can be sensed.

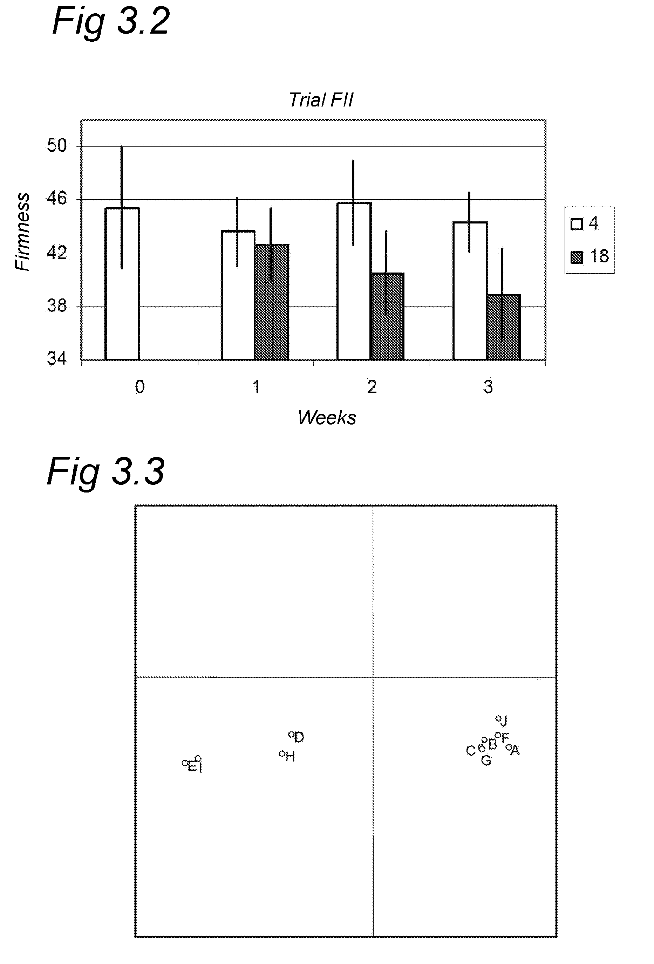

Figure 3.2 shows the results of the firmness measurement of one of the trials (FII). Only after two weeks storage statistical significant differences can be measured between the two storage conditions.

Figure 3.3 shows the PCA plot of the data obtained with a microarray that contains around 900 genes. From this it is clear that already after 1 week suboptimal storage, reproducible differences can be observed. These differences were found to be explained by a very limited number of genes. A selection of genes has been made from this group.

**Table 6 below shows the microarray expression data of indicator genes**

| ID | A | B | C | **D** | **E** | F | G | **H** | **I** | J |
|---|---|---|---|---|---|---|---|---|---|---|
| upregulated genes as a result of storage at too high temperature, 18°C (SEQ ID NO: 57-66) | | | | | | | | | | |
| Apple13nr042 | -1,25 | -0,87 | -1,15 | **0,77** | **0,94** | -1,07 | -0,58 | **0,42** | **0,53** | -0,45 |
| Apple13nr062 | -5,12 | -4,17 | -4,62 | **0,42** | **1,89** | -4,44 | -3,74 | **0,24** | **1,63** | -3,55 |
| Apple15nr090 | -1,08 | -0,74 | -1,27 | **0,31** | **0,40** | -0,72 | -0,24 | **0,35** | **0,28** | -0,21 |
| Apple5nr032 | -0,96 | -0,69 | -1,45 | **0,37** | **0,52** | -0,63 | -0,39 | **0,35** | **0,64** | -0,02 |
| Apple5nr041 | -0,84 | -0,36 | -1,04 | **0,37** | **0,79** | -0,19 | -0,29 | **0,26** | **0,62** | -0,35 |
| Apple5nr069 | -3,77 | -2,89 | -4,21 | **0,29** | **2,13** | -3,07 | -3,19 | **0,13** | **1,76** | -2,83 |
| Apple6nr013 | -1,20 | -0,59 | -1,32 | **0,84** | **1,28** | -0,73 | -1,06 | **0,64** | **0,94** | -0,69 |
| Apple6nr096 | -1,03 | -0,54 | -1,04 | **0,69** | **0,35** | -0,41 | -0,27 | **0,48** | **0,23** | -0,13 |
| Apple7nr006 | -1,27 | -1,23 | -1,96 | **1,00** | **0,29** | -1,71 | -0,94 | **0,91** | **1,35** | -0,59 |
| Apple8nr019 | -1,08 | -0,10 | -1,56 | **0,43** | **0,37** | -0,66 | -0,59 | **0,49** | **0,47** | -0,28 |
| downregulated genes as a result of storage at too high temperature, 18°C (SEQ ID NO: 67-76) | | | | | | | | | | |
| Apple11nr011 | 0,27 | 0,59 | -0,12 | **-2,52** | **-2,79** | 0,75 | 0,11 | **-2,96** | **-2,22** | 1,11 |
| Apple12nr048 | 0,36 | 0,35 | -0,19 | **-1,15** | **-2,25** | 0,17 | 0,74 | **-1,57** | **-2,48** | 0,69 |
| Apple12nr056 | 0,43 | 0,40 | -0,31 | **-1,08** | **-2,44** | 0,12 | 0,64 | **-1,50** | **-2,28** | 0,62 |
| Apple12nr062 | -0,07 | 0,00 | -0,88 | **-0,95** | **-2,01** | 0,85 | -0,22 | **-1,24** | **-2,26** | 1,67 |
| Apple12nr094 | 0,37 | 0,29 | -0,10 | **-1,76** | **-5,11** | 0,76 | 0,29 | **-2,77** | **-5,54** | 1,35 |
| Apple1nr054 | 0,67 | 0,51 | -0,26 | **-1,77** | **-4,21** | 0,97 | 0,79 | **-2,17** | **-3,78** | 1,12 |
| Apple1nr089 | 0,67 | 0,18 | -0,72 | **-1,25** | **-1,58** | 0,99 | 0,44 | **-2,03** | **-1,88** | 1,42 |
| Apple2nr039 | -0,11 | 0,27 | -1,01 | **-0,83** | **-1,99** | 1,02 | -0,11 | **-0,91** | **-2,29** | 1,55 |
| Apple2nr080 | 0,29 | 0,69 | -0,27 | **-1,81** | **-1,77** | 0,27 | 0,37 | **-1,69** | **-1,37** | 1,04 |
| Apple3nr034 | -0,11 | 0,46 | -0,82 | **-0,12** | **-1,89** | 0,77 | 0,99 | **-0,93** | **-1,85** | 0,95 |

| | | | | | | | | | | |
|---|---|---|---|---|---|---|---|---|---|---|
| A= wk0, 4°C (experiment II) B= wk1, 4°C (experiment II) C= wk2, 4°C (experiment II) D= wk1, 18°C (experiment II) E = wk2, 18°C (experiment II) F = wk0, 4°C (experiment I) G = wk2, 4°C (experiment I) H = wk1, 18°C (experiment I) I = wk2, 18°C (experiment I) J = wk0, 4°C (experiment I) | | | | | | | | | | |

**Table 7 shows homology of indicator genes to known genes**

| | | |
|---|---|---|
| Upregulated when stored at 18°C | | |
| SEQ ID 57 | Apple13nr042 | SNF8 like protein (Arabidopsis thaliana) |
| SEQ ID 58 | Apple13nr062 | None |
| SEQ ID 59 | Apple15nr090 | None |
| SEQ ID 60 | Apple5nr032 | VPE-CITSI vacuolar processing enzyme precursor |
| SEQ ID 61 | Apple5nr041 | ubiquitin conjugating protein |
| SEQ ID 62 | Apple05nr069 | ripening related gene |
| SEQ ID 63 | Apple06nr013 | Glutathione S-transferase |
| SEQ ID 64 | Apple6nr096 | sarcosine oxidase |
| SEQ ID 65 | Apple07nr006 | dicyanin (Lycopersicon esculentum) |
| SEQ ID 66 | Apple8nr019 | Lipoxygenase |
| Downregulated when stored at 18°C | | |
| SEQ ID 67 | Apple11nr011 | None |
| SEQ ID 68 | Apple12nr048 | vacuole associated annexin VCaB42 (Nicotiana tabacum) |
| SEQ ID 69 | Apple12nr056 | C-methyltransferase from soybean |
| SEQ ID 70 | Apple12nr062 | endo-xyloglucan transferase from cotton |
| SEQ ID 71 | Apple12nr094 | NADP-dependent D-sorbitol-6-phosphate |
| SEQ ID 72 | Apple01nr054 | None |
| SEQ ID 73 | Apple1nr089 | None |
| SEQ ID 74 | Apple2nr039 | xyloglucan endo-transglycolase-like protein |
| SEQ ID 75 | Apple2nr080 | heat shock protein 70 (Arabidopsis thaliana) |
| SEQ ID 76 | Apple3nr034 | None |

### 3.2 Material and Methods

Expression levels were determined in mesocarp of apple fruit using microarrays (described below) or any other gene expression profiling format, such as RT-PCR. Results are most reliable when samples are related to an sample taken at harvest time.

### 3.2.1 On site, robust sampling

Use about 10-20 mg of plant tissue for the homogenate. Add 5 parts double distilled water to the tissue. Grind until it is apparent that some plant tissue is homogenized. The homogenate does not have to have a smooth consistency. Apply 25 microliter of plant homogenate to each circle on an FTA card (Whatman).

Allow plant homogenate on FTA to dry for at least one hour at room temperature. Do not heat assist the drying period. Archive the sample in a dessicated environment.

### 3.2.2 RNA isolation from plant homogenate on FTA Cards

Take a sample disc from the dried spot using and place it in an Eppendorf vial Add 400 microliter RNA processing buffer (10 mM Tris-HCl, pH 8.0, 0.1 mM EDTA, 1 microliter RNAse inhibitor, 200 microgram/ml glycogen and 2 mM DTT, freshly prepared). Mix and incubate on ice for 15 minutes (mix every five minutes). Remove the disc. Precipitate the RNA with 1/10th volume of 3M sodium acetate pH 5.2 and two volumes of ice cold 100% isopropanol. Incubate for 1 hour ad -20 C. Spin down the RNA at top speed in an eppendorf centrifuge. Wash the pellet with 75% ethanol. Air dry the pellet. Resuspend the pellet in a suitable volume of double distilled water. Use DNA free (AMBION) for removal of traces of DNA following the protocol of the manufacturer. After that, the RNA preparation can be directly used for cDNA synthesis and subsequent PCR.

### 3.2.3 Microarray hybridisation

Total RNA, up till 20 microgram, purified with RNeasy (Qiagen, The Netherlands) and complemented with 1 nanogram luciferase polyA mRNA was used for each individual labeling. Reference RNA was labeled with Cy3 and sample RNA with Cy5 using the CyScribe First-Strand cDNA Labeling Kit (Amersham Biosciences). After checking the integrity of the labeled cDNA using agarose electrophoresis, sample and reference cDNA were mixed and used for hybidisation of the microarray following the protocol supplied by the manufacturer of the slides. Cover slides and hybridisation chambers from Agilent Technologies (Palo Alto) were used. Hybridisation was allowed to continue overnight in an incubator where the slides were continuously rotating (Sheldon Manufacturing). Post hybridisation washes were according to the Nexterion protocol.

### Example 4 - Prediction greymold (Botrytis cinerea) in cut flowers, exemplified in Rose (Rosa hybrida L. cv. Bianca)

### 4.1 Indicator genes

A set of 36 indicator genes (SEQ ID NO: 77-109 and SEQ ID NO: 172-174) have been selected whose expression profile can be used as measurement to predict the susceptibility of roses to *Botrytis.*

Based on the expression level of the described genes conclusions can be drawn about the predicted quality class of the batch of roses (good-almost no *Botrytis,* moderate-some *Botrytis* disease will develop or bad-severe *Botrytis* disease can be expected). The genes have been selected based on 12 batches of Bianca roses with different levels of *Botrytis* decay after 1 week of vase-life. Evaluation of gene expression and prediction using the indicator genes has been performed using Real Time PCR analysis of the same 12 batches, normalized using the housekeeping genes listed in SEQ ID NO: 110-112.

Figure 6.1 shows a typical result where expression data of 22 genes (marked in Table 8 below) were used to classify two quality groups. High probability (1) indicates a reliable prediction. From the Figure it is clear that in most cases quality prediction is reliable (probability above 0.8).

Figure 6.2 shows a graph where the expression ratio of only two genes was plotted against the percentage of decay as a result of Botrytis invasion.. It indicates that the expression levels of small number of genes, whether or not after mathematical conversion, also gives a good prediction.

**Table 8 shows homologies of indicator genes from rose to known genes**

| SEQ ID NO: | Name | Putative function based on homology | Used in fig 6.1 |
|---|---|---|---|
| SEQ ID 96 | OProseR0008 | Unknown | |
| SEQ ID 97 | OProseR0053 | putative xyloglucan endotransglycosylase | X |
| SEQ ID 98 | OProseR0060 | endo-xyloglucan transferase | X |
| SEQ ID 99 | OProseR0106 | phosphate transport protein (propably Botrytis cinerea) | |
| SEQ ID 100 | OProseR0238 | putative lipid transfer protein | X |
| SEQ ID 101 | OProseR0260 | Unknown | |
| SEQ ID 102 | OProseR0277 | Unknown | X |
| SEQ ID 103 | OProseR0286 | Protein disulfide isomerases | X |
| SEQ ID 104 | OProseR0371 | Unknown | X |
| SEQ ID 105 | OProseR0556 | Unknown | X |
| SEQ ID 106 | OProseR0626 | Unknown | X |
| SEQ ID 107 | OProseR0763 | polygalacturonase inhibitor protein | X |
| SEQ ID 108 | OProseR0774 | aquaporin protein | X |
| SEQ ID 109 | OProseR0812 | S-adenosyl-L-methionine decarboxylase | X |
| SEQ ID 77 | OProseR1069 | glutathione S-conjugate transporting ATPase | |
| SEQ ID 78 | OProseR1072 | Unknown | X |
| SEQ ID 79 | OProseR1093 | Unknown | X |
| SEQ ID 80 | OProseR1094 | Vacuolar ATP synthase 16 kDa proteolipid subunit | |
| SEQ ID 81 | OProseR1100 | chalcone synthase | X |
| SEQ ID 82 | OProseR1117 | amygdalin hydrolase isoform AH I precursor | X |
| SEQ ID 83 | OProseR1198 | delta 9 acyl-lipid desaturase | |
| SEQ ID 84 | OProseR1208 | Unknown | |
| SEQ ID 85 | OProseR1246 | Unknown | |
| SEQ ID 86 | OProseR1322 | mitochondrial formate dehydrogenase precursor | X |
| SEQ ID 87 | OProseR1391 | Unknown | |
| SEQ ID 88 | OProseR1459 | translation initiation factor IF 1 | |
| SEQ ID 89 | OProseR1481 | Unknown | X |
| SEQ ID 90 | OProseR1674 | Probable NADH-ubiquinone oxidoreductase | X |
| SEQ ID 91 | OProseR1700 | Unknown | |
| SEQ ID 92 | OProseR1727 | 3-hydroxy-3-methylglutaryl coenzyme A reductase | |
| SEQ ID 93 | OProseR1783 | GAST-like gene product | X |
| SEQ ID 94 | OProseR1792 | Unknown | |
| SEQ ID 95 | OProseR1807 | putative proteasome alpha subunit | |
| SEQ ID 172 | OProseR1663 | HHG4 nucleoid DNA-binding protein | X |
| SEQ ID 173 | OProseR0948 | Unknown | X |
| SEQ ID 174 | OProseR0049 | Actin | X |

### 4.2 Material and Methods

### 4.2.1 Sampling

First three outer petals of 25 roses have been used for development of the test. The remaining 75 roses of the same batch were used to determine the % of flowers showing Botrytis disease after a 7 days incubation period at 21°C, 60% RH using standard light regime of 10h light, 14 hours darkness. The 25 x 3 outer petals were frozen directly in liquid nitrogen, powered using mortal and pestle and stored at -80 °C.

### 4.2.2 RNA isolation

As described above using RNA easy. Concentration can be determined spectrophotometrically or using nanodrop apparatus.

### 4.2.3. Microarray hybridisation

Hybridisations have been performed using a indirect labeling protocol.

### 4.2.4. Statistical analysis

Genes have been selected using T-tests and the software Significant Analysis of Microarray- (http://www-stat.stanford.edu/∼tibs/SAM), profile analysis using the programme Spotfire

(http://www.spotfire.com/products/decisionsite_microarray_analysis.cfm) in which Botrytis disease % were correlated to genes expression profiles, and using the software Predicted Analysis Microarray (http://www-stat.stanfbrd.edu/∼tibs/PAM).

### 4.2.5. Primer development

Primers for the selected genes were designed using Primer 3 software (http://frodo.wi.mit.edu/cgi-bin/primer3/primer3_www.cgi) in combination with DNA mfold software (http://www.bioinfo.rpi.edu/applications/mfold/old/dna/).

### 4.2.6 Real Time RT PCR

Reverse Transciptase reaction using oligodT and Real Time analysis using the diluted cDNA can be used in a standard RealTime PCR protocol.

### Example 5 - Discolouration of mushrooms, exemplified by white button mushroom (Agaricus bisporus) fruiting body

### 5.1 Indicator genes

Based on two batches white button mushrooms of the same development stage but with different degrees of browning a suppressive subtraction hybridisation library has been constructed. Out of the cloned and sequenced clones 878 clones were selected for printing on the microarray, together with 19 clones from literature (Eastwood,D.C., Kingsnorth,C.S., Jones,H. and Burton,K.S. 2001, Genes with increased transcript levels following harvest of the sporophores of Agaricus bisporus have multiple physiological roles. Mycol. Res. 105 (10), 1223-1230). Also a partial sequence of the published polyphenol oxidase sequence PPO1 (Wichers,H.J., Recourt,K., Hendriks,M., Ebbelaar,C.E., Biancone,G.,Hoeberichts,F.A., Mooibroek,H. and Soler-Rivas,C. 2003. Cloning, expression and characterisation of two tyrosinase cDNAs from Agaricus bisporus. Appl. Microbiol. Biotechnol. 61 (4), 336-341) was printed on the same array. Samples with different storage quality were selected form a large range of samples that we randomly collected from growers in the Netherlands. The representative sample of 250g mushrooms were taken from a batch of 2 kg. The 250 g was frozen in liquid nitrogen, the remaining mushrooms were analysed for colour using computer image analysis at day of sampling and after 7 days storage at 2°C.

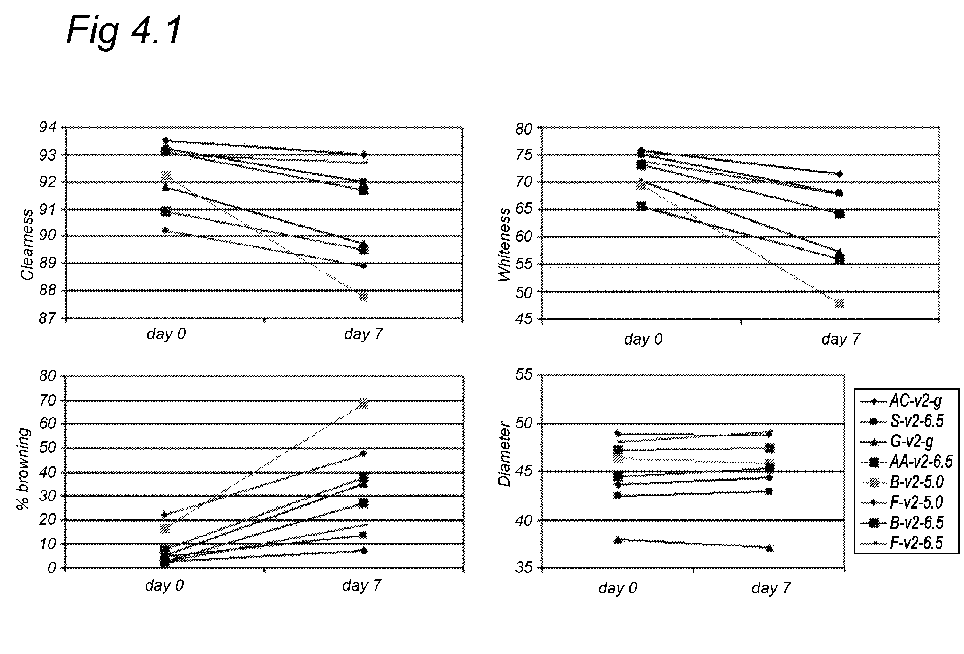

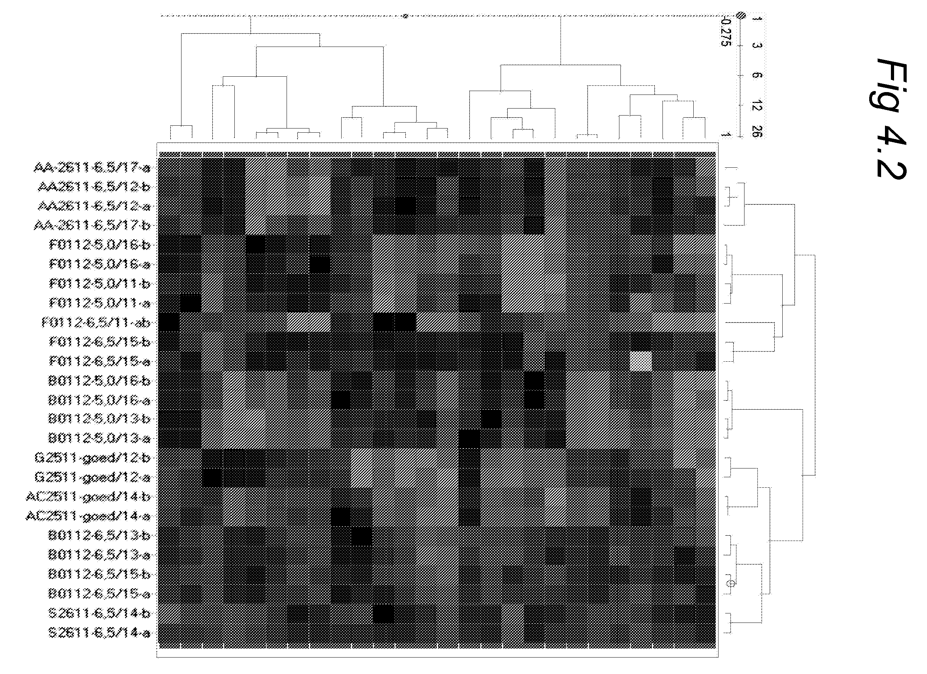

The microarrays were hybridized with 8 samples (see figure 4.1) of which two (AC2511 en G2511) were harvested stored overnight by 4°C and then sampled. The other six were harvested at the same day as they were sampled with a night cold storage. The letters indicate the grower, so two batches with different storage quality of the same grower delivered the same day were obtained from grower B and grower F. Using the same selection tools t-tests, SAM, profile correlation with browning after 7 days, hierarchical clustering, PAM a selection of putative indicator genes was generated (see SEQ ID NO: 113-135 and fig 4.2). For some of the genes expression profiles were validated by real Time PCR analysis, normalized by 18S analysis, which fitted in almost al cases with microarray based gene expression.

**Table 9 shows homology of indicator genes to known genes, indicating putative function**

| SE ID NO: | Name | Putative function based on homology |
|---|---|---|
| SEQ ID 113 | AJ271698 | Unknown |
| SEQ ID 114 | AJ271702 | Unknown |
| SEQ ID 115 | AJ271701 | Unknown |
| SEQ ID 116 | AJ271693 | B-(1-6) glucan synthase |
| SEQ ID 117 | AJ271707 | cytochrome P450 |
| SEQ ID 118 | AJ271696 | Involved in DNA binding and repair |
| SEQ ID 119 | X85113 | Polyphenoloxidase |
| SEQ ID 120 | SSH03nr023 | unknown |
| SEQ ID 121 | SSH05nr012 | unknown |
| SEQ ID 122 | SSH05nr019 | unknown |
| SEQ ID 123 | SSH07nr010 | Unknown |
| SEQ ID 124 | SSH07nr032 | Unknown |
| SEQ ID 125 | SSH07nr041 | Unknown |
| SEQ ID 126 | SSH07nr083 | endochitinase |
| SEQ ID 127 | SSH09nr088 | Urease |
| SEQ ID 128 | SSH10nr021 | Unknown |
| SEQ ID 129 | SSH10nr028 | Unknown |
| SEQ ID 130 | SSH10nr080 | unknown |
| SEQ ID 131 | SSH11nr017 | Putative sugar transporter |
| SEQ ID 132 | SSH12nr023 | Unknown |
| SEQ ID 133 | SSH12nr053 | myosin heavy chain A |
| SEQ ID 134 | SSH13nr021 | Unknown |
| SEQ ID 135 | SSH13nr086 | Unknown |

### 5.2 Material and Methods

### 5.2.1 Sampling

Stipes of mushrooms were cut off at the based of the cap. The cap was sliced and directly frozen in liquid nitrogen and stored at -80C. Frozen, sliced caps were powdered in a blender while continuously chilled using liquid nitrogen.

### 5.2.2 RNA isolation

RNA was isolated using RNAeasy, including a shredder homogenizing and clarification step. Concentration can be determined photospectrometrically or using nanodrop apparatus.

### 5.2.3. Microarray hybridisation

Hybridisations have been performed using a indirect labeling protocol.

### 5.2.4. Statistical analysis

Genes have been selected using T-tests and the software Significant Analysis of Microarray- (http://www-stat.stanford.edu/∼tibs/SAM), profile analysis using the programme Spotfire

(http://www.spotfire.com/products/decisionsite_microarray_analysis.cfm) in which clearness, witness and % browning were correlated to gene expression profiles, and using the software Predicted Analysis Microarray (http://www-stat.stanford.edu/∼tibs/PAM).

### 5.2.5 Primer generation

Primers for the selected genes were designed using Primer 3 software (http://frodo.wi.mit.edu/cgi-bin/primer3/primer3_www.cgi) in combination with DNA mfold software (http://www.bioinfo.rpi.edu/applications/mfold/old/dna/).

### 5.2.6 Real Time RT PCR

Reverse Transcriptase reaction using oligodT and Real Time analysis using the diluted cDNA can be used in a standard RealTime PCR protocol.

### 5.3 Species

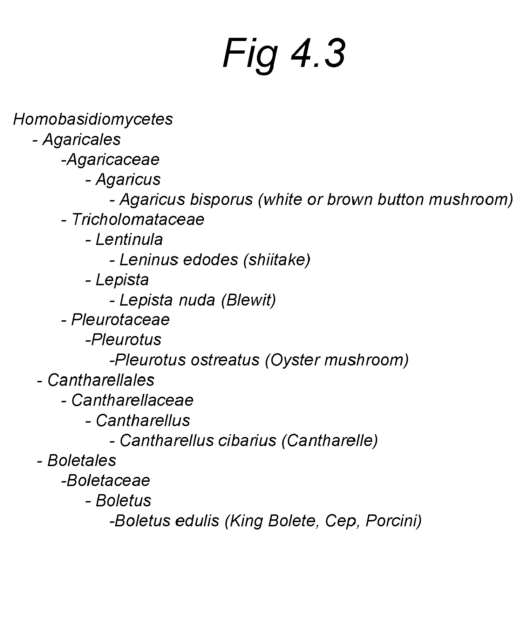

The technique has been developed on *Agricus bisporus* strain U1 and A15 and is (because genetic variation is very limited between cultivated strains of *Agaricus bisporus*) also applicable for other strain (brown, portobello) of the same species. Also other species of Agaricus can be diagnosed using this method. Homologous genes of the genes listed in the table above may also be applicable for quality diagnostic of other edible mushrooms like shiitake *(Lentinus edodes), Pleurotus ostreatus,* the Oyster mushroom and *Lepista nuda* (synonyms Clitocybe nuda, Tricholoma nudum en Rhodopaxillus nudus) which are close relatives of *Agaricus bipsorus* or even more distantly related edible mushrooms like *Cantharellus cibarius* and *Boletus edulis* (see figure 4.3).

### Example 6 - Prediction of postharvest firmness development in Solanaceous fruits, exemplified in Tomato (Solanum lycopersicum L. cv. Aromata)

### 6.1 Indicator genes

A set of 19 indicator genes (SEQ ID NO:136-154) have been selected whose expression profile can be used as measurement to predict the rate at which harvested Solanaceous fruits loose their firmness.

Based on the expression level of the described genes conclusions can be drawn about the predicted quality class of the batch of fleshy fruit such as tomatoes (good-firmness retained for more than 28 days, moderate- firmness retained for more than 15 days or bad- firmness drops below acceptable levels within 15 days). The genes have been selected based on 44 batches of Aromata tomatoes with different levels of firmness development during 6 weeks of post-harvest storage. Evaluation of gene expression and prediction using the indicator genes has been performed using microarray analysis of a subset of 16 batches, normalized using the housekeeping genes listed in SEQ ID NO: 155-157.

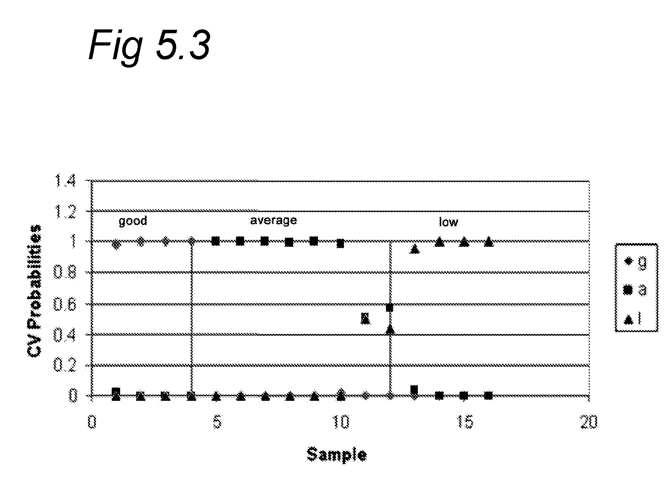

As shown in figure 5.3 the 19 indicator genes are able to discriminate between the 3 quality classes indicated before.

**Table 10 shows homologies of indicator genes from tomato to known genes**

| SEQ ID NO: | Name | Putative function based on homology | E value |
|---|---|---|---|
| SEQ ID 136 | Tomaat1Fnr092 | fruit-specific protein - tomato | 4,00 E-46 |
| SEQ ID 137 | Tomaat1Fnr176 | Unknown | 7,00 E-04 |
| SEQ ID 145 | Tomaat1Fnr224 | Unknown | 3,00 E-11 |
| SEQ ID 146 | Tomaat3Fnr100 | protein kinase | 2,00 E-05 |
| SEQ ID 138 | Tomaat3Fnr355 | Unknown | 0,00 E+00 |
| SEQ ID 147 | Tomaat7Rnr298 | pyruvate decarboxylase | 3,00 E-15 |
| SEQ ID 148 | Tomaat7Rnr310 | Unknown | 6,00 E-84 |
| SEQ ID 149 | Tomaat7Rnr355 | malate dehydrogenase | 3,00 E-69 |
| SEQ ID 150 | Tomaat7Rnr478 | Alcohol dehydrogenase | 3,00 E-12 |
| SEQ ID 139 | Tomaat7Rnr558 | ATPase beta subunit | 1,00 E-23 |
| SEQ ID 151 | Tomaat7Rnr563 | 1 cytochrome P450-dependent fatty acid hydroxylase | 3,00 E-66 |
| SEQ ID 152 | Tomaat7Rnr567 | Unknown | 2,00 E-03 |
| SEQ ID 153 | Tomaat9Fnr019 | DNA binding protein | 3,00 E+00 |
| SEQ ID 140 | Tomaat9Fnr046 | Unknown | 0,00 E+00 |
| SEQ ID 141 | Tomaat9Fnr216 | Ribulose bisphosphate carboxylase small subunit 2A | 1,00 E-32 |
| SEQ ID 142 | Tomaat9Fnr244 | Pathogenesis-related protein STH-2 | 2,00 E-83 |
| SEQ ID 154 | Tomaat9Fnr248 | sucrose transport protein | 1,00 E-45 |
| SEQ ID 143 | Tomaat9Fnr323 | chitinase | 2,00 E-17 |
| SEQ ID 144 | Tomaat9Fnr349 | two-component sensor histidine kinase | 2,00 E-19 |

**Table 11 shows additional genes which have a constant expression and are suitable for normalization**

| SEQ ID NO | Name | Putative function | E value |
|---|---|---|---|
| SEQ ID 155 | Tomaat3Fnr244 | Unknown protein Arabidopsis Genbank NP_566171 | 1,00 E-63 |
| SEQ ID 156 | Tomaat3Fnr268 | GTP-binding nuclear protein Ran1 GenBank P38546 | 8,00 E-35 |
| SEQ ID 157 | Genbank L08255 | Tomato abscisic stress ripening protein 1 | |

### 6.2 Material and Methods

### 6.2.1 Sampling

Tomatoes (variety Aromata) were obtained from different growers and moved to the laboratory 1 day after picking. Pericarp slices (2 from each fruit, 12 or more fruits per batch) spanning approximately 1 cm² of surface and the entire pericarp thickness were taken from the equatorial region of each fruit. Slices from the same batch were pooled and stored at -80°C until further use.

### 6.2.2 RNA isolation

For mRNA isolation 4 g of frozen material was ground in liquid nitrogen and transferred to an RNAse free centrifuge tube. To this 25 ml of lysis buffer (100 mM TrisHCl pH7.5, 500 mM LiCl, 10 mM EDTA, 1% lithium dodecysulphate, 5 mM dithiothreitol) was added and homogenized by vortexing. After incubation for 5 min at 65°C the tuber was centrifuged and supernatant transferred to a clean tube. 200 µl of washed oligo -dT-conjugatedDynabeads were added and incubated for 60 min on roller bench. Beads were isolated using a magnet and RNA was washed and eluteted according to the manufacturer's instructions. Eluted mRNA underwent an extra purification round by binding to Dynabeads and subsequent elution. Concentration was determined spectrophotometrically using nanodrop apparatus.

### 6.2.3. Microarray hybridisation

mRNA was purified as described above. 2.5 µg of poly (A⁺) RNA was spiked with 1.0 ng of *in vitro* synthesized luciferase mRNA (Promega) and reverse transcribed in the presence of 5-(3-aminoallyl)-2'-dUTP (Sigma A0410) using 2 µg oligo (dT)₂₁ as a primer. A 25 µL reaction containing, in addition to the oligo (dT)-annealed RNA template, 1× first strand buffer (Life Technologies), 10 mM DTT, 15 U ribonuclease inhibitor (Life Technologies), 0.5 mM dATP, 0.5 mM dCTP, 0.5 mM dGTP, 0.3 mM dTTP, 0.2 mM aminoallyl-dUTP and 150 U SuperScript II RNase H-reverse transcriptase (Life Technologies) was incubated at 37°C for 2 hr. Nucleic acids were then ethanol precipitated at room temperature and dissolved in 10 µL 1× TE (pH 8.0). Next, cDNA/mRNA hybrids were denatured (3 min at 98°C) and chilled on ice. RNA was degraded by adding 2.5 µL 1 M NaOH and incubating 10 min at 37°C. After neutralizing the mixture by adding 2.5 µL 1M HEPES (pH 6.8) and 2.0 µL 1 M HCl, the cDNA was recovered by ethanol precipitation and resuspended in 10.0 µL 0.1 M sodium carbonate buffer (pH 9.3).

In a second step the modified cDNA was coupled to a fluorescent dye, either Cyanine 3 (Cy3) or Cyanine 5 (Cy5), using reactive Cy3- or Cy5-NHS-esters (Amersham Pharmacia). To this end 10.0 µL of a 10 mM dye solution in DMSO was added to 10.0 µL of the cDNA sample and incubated at room temperature for 30 min. Finally, the labeled cDNA was ethanol precipitated twice and dissolved in 5 µL MQ.

Following prehybridization at 42°C for 2 hr in a few ml of hybridization buffer (50% formamide, 5× Denhardt's reagent, 5×SSC, 0.2% SDS, 0.1 mg/ml denatured fish DNA), slides were rinsed in MQ and in isopropanol and then dried by centrifugation (1 min, 470×g). For a dual hybridization, 35 µL of hybridization mixture, containing both (Cy3- and Cy5-labelled) samples at a concentration corresponding to 8 ng of the initial mRNA per µL mixture, was used. Prior to use, the hybridization mixture was heated at 95°C (1 min), cooled on ice and spun down to remove any debris. Hybridizations were done over night at 42°C using a Gene Frame (10×10 mm, 25 µL volume; ABgene) in a hybridization chamber. After hybridization, slides were washed at room temperature in 1× SSC, 0.1% SDS (5 min) followed by 0.1× SSC, 0.1% SDS (5 min) and rinsed briefly in 0.1× SSC before drying by centrifugation (1 min, 470×g).
Microarray slides were scanned with a ScanArray 3000 (Packard BioScience) with 75% laser power and 75% attenuation at a resolution of 10 µm. The resulting Cy3 and Cy5 images were stored as TIFF-files. Total pixel intensities within a fixed area (circle, ø 12 pixels) were obtained for each spot using Array Vision image analysis software (Imaging Research). Fluorescence data were imported in a spreadsheet for further work. Background spot fluorescence was determined as the mean of the fluorescence of designated spots and subtracted for each channel.

### 6.2.4. Statistical analysis

Genes have been selected using GeneMaths 2.1 software ((Applied Maths, Sint-Martens-Latem, Belgium) in which rate of firmness loss was correlated to genes expression profiles, and using the software Predicted Analysis Microarray (http://www-stat.stanford.edu/∼tibs/PAM).

### 6.2.5. Primer development

Primers for the selected genes were designed using Primer Express 1.0 software (Applied Biosystems).

### 6.2.6 Real Time RT PCR

Reverse Transciptase reaction using oligodT and Real Time analysis using the diluted cDNA was performed with standard RealTime PCR protocol utilizing one step SYBR green mastermix for qPCR (Eurogentec) on a ABI Prism 7700 sequence detection system (Applied Biotechnologies).

### 6.2.7 Determination of firmness

Tomato firmness was measured on a representative selection of 15 tomatoes per batch per sample moment. Firmness was determined using sensoric measurements or an Instron firmness tester. The Instron firmness test measures the impression of the fruits upon pressure of a plunger that is applied with a force of 3 N (non-dectructive). Both measurements correlate high in our lab (r > 0.91), this correlation was previously established by Kader et al. (1978, J.Amer.Soc.Hort.Sci 103(1); 70-73) and Polderdijk et al. (1993, Postharvest Biol. Technol. 2; 179-185).

In the test sensoric measure values are used. These correlate with Instron values as depicted in the Table 12 below:

**Table 12**

| Sensoric value | Instron value (mm) | Consumer validation |
|---|---|---|
| 2 | -1.3 | Very Soft |
| 3 | -1.1 | Soft |
| 4 | -0.75 | Fairly firm |
| 5 | -0.6 | Firm |
| 6 | -0.4 | Very Firm |
| 7 | -0.2 | Hard |
| 8 | 0 | Extra Hard |

For the experiments the value of 5 was determined as the lowest firmness level that is still acceptable for consumers.

## Claims

1. A diagnostic method for determining a future quality trait stage of a plant, or of a plurality of plants, or of a plant part, or of edible mushrooms, comprises the steps:
(a) providing a nucleic acid sample of a plant or mushroom, or of a plant or mushroom part, or of a plurality of plants or mushrooms,
(b) analyzing the nucleic acid sample by determining the expression level of a set of at least two indicator mRNA transcripts in the sample, which are indicative of a quality trait stage, and optionally
(c) identifying and selecting the plants or mushrooms, or plant parts or mushroom parts, which comprise a certain expression profile of the indicator mRNA transcripts, and
(d) identifying and selecting the plant, plurality of plants, plant parts or mushrooms having different expression profiles of the indicator mRNA transcripts from one another in the further distribution and use thereof.

2. The method according to claim 1, wherein the plant is of a family selected from the group consisting of *Fagaceae, Maloideae, Rosoideae, Solanaceae* and wherein the edible mushroom is a basidiomycete.

3. The diagnostic method according to claim 1 or 2, wherein the quality trait is a physiological trait or a morpholocical characteristic.

4. The diagnostic method according to any one of claims 1-3, wherein batches of plants or plant parts are separated in step (d) and wherein for each batch a nucleic acid sample comprising nucleic acids obtained from at least 5 different plants of the batch is analysed in steps (a) and (b).

5. The diagnostic method according to any one of claims 1-4, wherein the nucleic acid sample of step (a) is obtained from tissue homogenate and the mRNA is stabilized on a solid carrier, such as FTA® cards.

6. The diagnostic method according to any one of claims 1-3, wherein the expression level of at least 3, preferably at least 4 and more preferably at least 5 different indicator mRNA transcripts is determined.

7. Use of at least 3 indicator genes, or fragments thereof for the determination of a quality trait stage of a plant tissue sample.

8. A solid carrier comprising at least 3, preferably at least 4 or 5, nucleic acid molecules attached to said carrier, said molecules being capable of hybridizing to at least 3, preferably at least 4 or 5 indicator mRNA transcripts indicative of a quality trait stage.

9. The carrier according to claim 8, wherein the carrier is made of glass, plastic, nitrocellulose, nylon or silicon.

10. A kit for determining the quality trait stage of a plant or mushroom tissue sample, said kit comprising nucleic acid probes or primers capable of detecting the presence and/or quantity of at least 3, preferably of at least 4 or 5, nucleic acid molecules within a set of indicator mRNA transcripts indicative of a quality trait stage.

11. The kit according to claim 10, further comprising one or more of the following: instructions for use, control samples, control data, labeling reagents, detection reagents, hybridization or amplification reagents, primers or probes for detecting housekeeping-gene transcripts, containers or carriers, preferably a carrier according to claim 8 or 9.

12. The kit according to claim 10 or 11, further comprising a material for sampling plant or mushroom tissue, such as FTA® cards.
